# EUROPEAN PATENT APPLICATION

(11) **EP 1 369 126 A1**
(43) Date of publication of application: **10.12.2003**
(21) Application number: 02008774.8
(22) Date of filing: 18.04.2002
(51) Int. Cl.: A61K 39/00, A61K 48/00, A61P 35/00

(54) **Use of coding microsatellite region frameshift mutation-derived peptides for treating cancer**

(71) Applicant: MTM Laboratories AG, 69120 Heidelberg (DE)
(72) Inventor: Von Knebel Doeberitz, Magnus,, 69120 Heidelbeg (DE); Gebert, Johannes, 69221 Heidelberg (DE); Linnebacher, Michael, 66578 Stennweiler (DE); Wörner, Stefan, 69115 Heidelberg (DE); Ridder, Rüdiger, 69198 Schriessheim (DE); Bork, Peer, 69117 Heidelberg (DE); Yuan, Y.R, 69118 Heidelberg (DE)
(74) Representative: Schüssler, Andrea, Dr.

(57) **Abstract**

The present invention relates to a method of treatment of cancer, esp. of DNA-mismatch repair deficient (MMR) sporadic tumours and HNPCC associated tumours, which uses immunotherapy with combinatorial mixtures of tumour specific frameshift mutation-derived peptides or their corresponding nucleic acid sequences from different coding microsatellite regions (e.g. TCF-4, IGFIIR, caspase5, TAF1B, PTHL3) to elicit a cytotoxic T-cell response specifically directed against tumour cells. The method may be used for prevention of cancers and for the curative treatment of cancers and precancers.

## Description

The present invention relates to a method for the treatment of cancer, especially of DNA mismatch repair deficient (MMR) sporadic tumours and HNPCC associated tumours, which uses immuno therapy with combinatorial mixtures of tumour specific frameshift peptides to elicit a cytotoxic T-cell response specifically directed against tumour cells. The method may be used for prevention of cancers and for the curative treatment of cancers and precancers.

Tumour cells accumulate mutations in components of cellular pathways, that are essential for the maintenance of normal growth and differentiation. In human epithelial tumours, 2 types of genetic instability have been identified: chromosomal instability (CIN), which marks structural and numerical chromosomal aberration in aneuploid neoplastic cells, and microsatellite instability (MSI), which reflects length variations at repetitive DNA sequences in tumour cells. The type and spectrum of mutated genes markedly differs among CIN and MSI tumours, suggesting distinct but not mutually exclusive pathways of carcinogenesis. MSI occurs in about 90% of hereditary nonpolyposis colorectal cancers (HNPCC) as well as in about 15% of sporadic tumours of the colon and other organs, and is caused by mutational inactivation of different DNA mismatch repair genes.

The accumulation of genetic alterations and resulting mutant proteins represent a major obstacle for tumour cells to escape immune surveillance. The mutant proteins or peptides encoded by expressed mutant genes may elicit a specific cellular immune response and thus may be recognized by CTL. This is especially true concerning mutations resulting from chromosomal instabilities, but also pertains to more subtle genetic alterations, such as small deletions and insertions in microsatellites.

This situation can be used for therapies in cancers. The cancer cells are characterized by the expression of neo-peptides, which arose from the genetic alterations. These proteins are not present in normal, non cancerous tissues. Thus the neo-peptides may be used to distinguish on a molecular level between tumours and normal tissues. As tools suitable for the molecular discrimination antigen specific molecules or cells may be used. Thus it is possible to elicit an immune response against frameshift peptides, as might arise from MSI mutations, specific for tumours. Using cytotoxic T lymphocytes or FSP-specific antibodies it is thus possible to specifically attack and eliminate tumour cells in organisms and tissues.

A large number of genes containing coding microsatellites have been identified. Various of these genes show mutations within the microsatellites with certain frequencies in sporadic tumours. For few of these genes it could be shown, that they are involved in the majority of cases of particular tumours. For example the (A)₁₀ tract within the TGFβRII gene and the (G)₈ tract within the BAX gene are commonly mutated in gastrointestinal cancers such as colon cancer or gastric cancer.

The T-cell mediated immune response on the other hand provides a strong and specific selection pressure for the rapidly growing tumour cells (Tomlinson I, et al., Nat. Med. 1999; **5**: 11-2). Thus tumour evolution is forced to circumvent the immune surveillance by the cellular immune response. As a consequence of this selection pressure, mutations in genes of the antigen processing or presenting machinery arise in MMR-deficient tumour cells. In fact evasion of the immune surveillance by acquiring β2-microglobulin mutations has been observed at high frequency in MSI⁺ tumour cells (Bicknell DC, et al., Curr. Biol 1996; **6**: 1695-7). Other targets of specific mutation or down-regulation of expression are TAP1/TAP2 or HLA alleles. Direct down-regulation of expression of immunogenic epitopes is also a possible mechanism of immune escape as has been shown for melanoma-associated antigens in vivo (Jager E, et al., Int. J. Cancer 1997; **71**: 142-7). Due to this fact the relevant epitopes may not be detectable by the immune system.

As a consequence the promising therapies based on immune response directed against frameshift peptides in tumour tissues are prone to fail in a not yet determined number of cases of tumours. So the approaches for the use of the vaccination therapy of cancer mediated by frameshift peptides might be limited by the fact, that the potential immunogenic frameshift peptides are not always detectable by the immune system due to mutations in the antigen presenting and processing properties of the cancer cells. Even supposed, that during specific stages of tumour development cells express the aberrant proteins and thus are vulnerable to the immune surveillance, there potentially remains a population of tumour cells, that is not eliminated by the therapy. This state is quite undesirable for any therapy of cancers, for the remaining cells may continue growing and thus the tumour is not eliminated from the organism.

The methods known in the prior art for treatment of cancers associated with frameshift mutations, that lead to the expression of neo-peptides, are unsatisfying in that they are prone to leave active tumour cells within the organism, that may further grow. So there is a need to enhance the therapeutic methods to overcome this problem.

The present invention provides methods for enhanced therapeutic methods based on immune response directed against frameshift peptides in tumour cells.

The present invention is based on the inventors findings, that a mixture of frameshift peptides chosen according to combinatorial parameters minimizes the escape of particular populations of tumour cells from immunogenic elimination and may be used as a vaccine against a wide variety of DNA mismatch repair deficient tumours. The invention is based on the fact, that the immune escape of tumour cells is directed towards single immunogenic epitopes (frameshift peptides) in a potentially restricted subpopulation of the tumour cells. In the vast majority of MSI+ tumours various mutations may be found within the genome of the affected cells. So a combinatorial vaccination approach including several antigenic peptides could overcome this obstacle.

Generally using an immense or unlimited number of occurring frameshift peptides for vaccination it would be possible to address any potential number of disorder, that is associated with frameshift mutations. Due to practical and immunological concerns the number of peptides, that are included in a vaccine must be limited. To ensure a broad range of impact for the vaccine comprising a set of frameshift polypeptides, the selection of particular peptides has to be based on rationale considerations.

It is one aspect of the present invention to provide sets of frameshift peptides, that occur in a wide range of different MSI⁺ tumours and that diminish the probability of escape of tumours to be attacked by the immuno-therapy.

Another aspect of the present invention is a method for vaccination against MSI⁺ tumours using said set of frameshift peptides.

A third aspect of the present invention is a method for treatment of MSI⁺ tumours using a set of frameshift peptides, that elicit an immune response directed against a wide range of tumours.

Frameshift polypeptides as used herein shall comprise any polypeptides or fragments thereof, that arise by a frameshift mutation within a coding microsatellite sequence of a gene. A gene may harbour one or more coding microsatellite regions, that may give rise to frameshift peptides. The coding microsatellites according to the present invention comprise mononucleotide repeats, dinucleotide repeats, trinucleotide repeats, tetranucleotide repeats and pentanucleotide repeats of any length. According to present invention coding microsatellites contain preferably at least 3 and more preferably at least 5 repeats of the respective nucleotide pattern (1-5 nucleotides, which are repeated). The frameshift polypeptides as used according to the present invention comprise at least 1, more preferred at least 2 and even more preferred at least 3 amino acids of the mutated part of the polypeptide. Additionally, the frameshift polypeptides may comprise fragments of the originally non-mutated proteins and/or may be fused to any other polypeptide sequences suitable for the purposes of the present invention. Examples of such polypeptide sequences are linker sequences, or structural peptide sequences, such as beta barrels, loop sequences etc. that facilitate the immunogenicity of the frameshift sequences according to the present invention within the fusion polypeptide.

Frameshift polypeptides suitable for the method disclosed herein have to be immunogenic polypeptides. This requires, that the polypeptides may stimulate immune responses in host organisms either in the form the polypeptides adopt in their natural environment and/or especially in the form the polypeptides adopt after processing by the cellular antigen processing and presenting machinery.

According to the present invention the frameshift polypeptides may also be represented by nucleic acids coding for said polypeptides. These nucleic acids may for example be used for the in situ expression of the respective polypeptides. For the purpose of expression of nucleic acids, the particular nucleic acids may be joined with suitable other nucleic acid sequences, that enable for the cloning and expression of said nucleic acids encoding the frameshift polypeptides.

Mutation as used in the context of the present invention may comprise insertions or deletions of one or several nucleotides (or nucleotide repeats) within the specified microsatellite sequences. In a preferred embodiment of the present invention the number of nucleotides to be inserted or deleted is not 3 or must not be divisible by 3, such that the mutation leads to a frameshift with respect to the translational reading frame of the downstream nucleic acid sequence. Thus the nucleic acid sequence downstream of the mutation point will render a polypeptide-sequence different from the native sequence encoded by the respective gene. The mutation in these cases leads to a novel peptide sequence (a neo-peptide). Commonly the new peptide sequence is short due to the fact, that novel stop codons arise from the shift in the reading frame.

The method according to the present invention may be used for the treatment of disorders associated with frameshift mutations within coding microsatellite regions of genes. These disorders comprise for example degenerative diseases, such as neurodegenerative diseases, vascular disorders, disorders caused by stress, such as oxidative stress, chemically induced stress, irradiation induced stress, etc. and cancers including all sporadic cancers as well as HNPCC associated cancers. Cancers as used herein may comprise e.g. colorectal cancer, small cell lung cancer, liver cancer (primary and secondary), renal cancer, melanoma, cancer of the brain, head and neck cancer, gastrointestinal cancers, leukemias, lymphomas. prostate cancer, breast cancer, ovary cancer, lung cancer, bladder cancer etc..

The vaccines according to the present invention comprise one or more sets of frameshift polypeptides.

A set of frameshift polypeptides used according to the method disclosed herein comprises at least 5, more preferably at least 7 and most preferred at least 10 frameshift polypeptides. Due to immunological as well as practical concerns the set of frameshift peptides used as a vaccine may not include an unlimited number of frameshift peptides. Preferably the set of frameshift peptides comprises at maximum 15, in a more preferred embodiment at maximum 20 and in the most preferred embodiment of the invention at maximum 30 frameshift polypeptides. In order to ensure a maximum range of disorders to be addressed by the selected set of frameshift polypeptides the members of the set have to be selected by reasonable considerations. The frameshift polypeptides to be used as a set according to the present invention are selected with respect to a number of parameters characterizing said polypeptides.

One crucial aspect influencing the selection of the frameshift peptides is the mutation frequency of the relevant microsatellite region and thus the frequency of a particular expressed frameshift polypeptide. Mutation frequency as used in the context of the present invention pertains to the percentage of samples within a defined range of total samples, which show a particular mutation. Any method suitable for the determination of the percentage of individuals in a range of samples displaying the existence of a particular genotype or phenotype (with respect to the expression of polypeptides) may be employed for the determination of the frequency according to the present invention. The frequencies may be determined e.g. as described below in example 1.

The frequency may be the frequency of frameshift mutation within a coding microsatellite region or a frequency of expression of a frameshift polypeptide. Furthermore the frequency may be e.g. a frequency over a total of different tumour entities, the frequency with respect to a particular tumour entity, a frequency over several tumours entities restricted to particular stages of tumourigenesis or a frequency with respect to a particular tumour entity restricted to particular stages of tumourigenesis.

The frequency according to the present invention has to be determined taking into account a range of samples sufficient to render significant data. For example preferably at least 50 to 100 tumours may be included in the range of samples analyzed. In case, that a smaller number of samples has been taken into account for determination of the frequency, a variation of the determined frequency may take place, if the range of samples is broadened.

Generally any frequency as determined may be used as a tool for the choice of a set of frameshift polypeptides according to the present invention. To ensure best results for the method according to the present invention a largest possible number of samples has to be taken into account. Yet if there are only rare data, a frequency may also be determined with respect to a restricted number of samples. This may especially be true, if the data for the restricted population of samples indicates a quite high frequency of a particular peptide, and thus implies a high therapeutic value for the respective peptide. Especially in cases, where a frequency is to be determined related to a particular tumour entity or related to particular stages of the tumorigenesis, the population of samples may be restricted.

The mutation may occur at any time during the tumour evolution and may be persistent or may be eliminated from the genome. Thus the frequency may comprise the frequency of the expression of a peptide at a particular stage of tumourigenesis or the occurrence of a genetic mutation at a particular defined stage of tumorigenesis. In one embodiment of the invention the frequency for the mutation is determined taking into account the widest possible range of samples. In this embodiment the method disclosed herein may be especially useful for the preventive vaccination of tumours. In another embodiment of the present invention the frequencies of mutation are related to specified tumour entities. In this embodiment the method according to the present invention may e.g. especially be useful for immuno-therapeutic approaches in treatment of tumours or in the preventive vaccination of particular subpopultions with an elevated risk for the occurrence of particular tumours. In a third embodiment of the present invention the frequency may be related to particular stages in tumourigenesis of particular tumours. This embodiment may be e.g. especially useful for the treatment of diagnostically defined tumours or for adjuvant treatment of tumours simultaneously or following primary treatment of tumours.

Using frameshift polypeptides, that occur with a high frequency, the probability, that a particular tumour expresses the peptide and may thus be recognized by antibodies or antigen-recognizing cells, increases. By combination of multiple such polypeptides, the probability to address particular tumour cells further is raised. Preferred frameshift polypeptides according to the present invention occur in at least 25%, more preferred frameshift polypeptides in at least 30% and most preferred frameshift polypeptides in at least 33% of the cases of the particular condition to be treated by the method according to the present invention.

According to the present invention the polypeptides are chosen to be expressed with a high frequency, thus the set of polypeptides may be limited to a number of members and nonetheless may cover a range of occurring diseases as wide as possible. For example a set of 10 polypeptides, each occurring with a frequency of more than 30 percent will statistically cover a range of more than 95% of the potentially existing disorders, as far as they have been included in the studies leading to the respective frequencies. Using 7 polypeptides requires the application of polypeptides with higher frequencies in order to cover a range of about 95% of potentially existing tumours. Using a set of polypeptides derived from enormously frequent mutated microsatellite regions may also allow for the employment of a set of only five different polypeptides without lowering the range of potentially addressed tumours. Thus the set of polypeptides comprises in a preferred embodiment at least 5, in a more preferred embodiment at least 7 and in the most preferred embodiment at least 10 different frameshift polypetides.

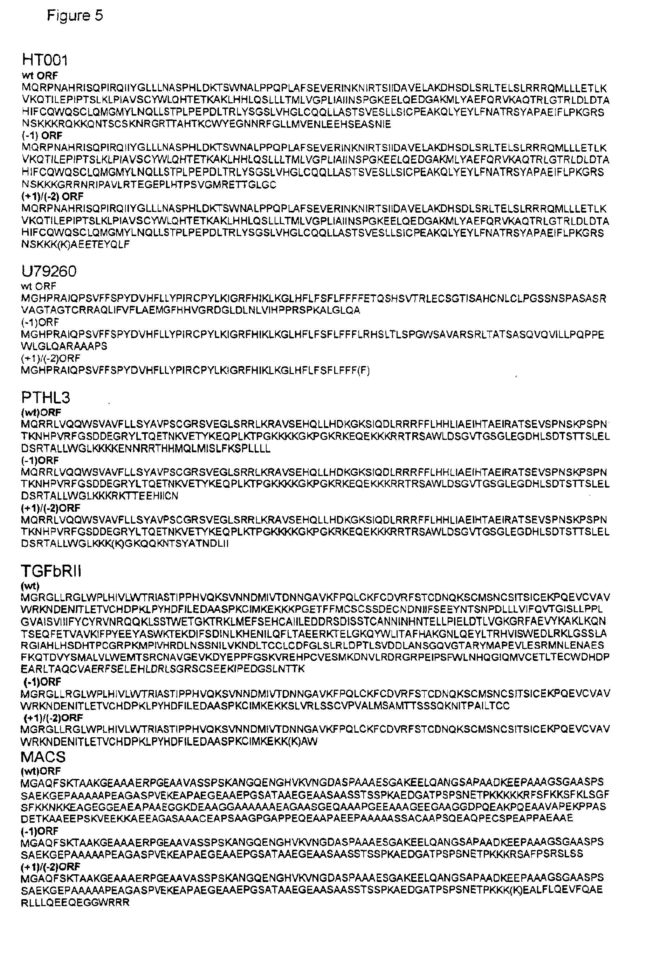

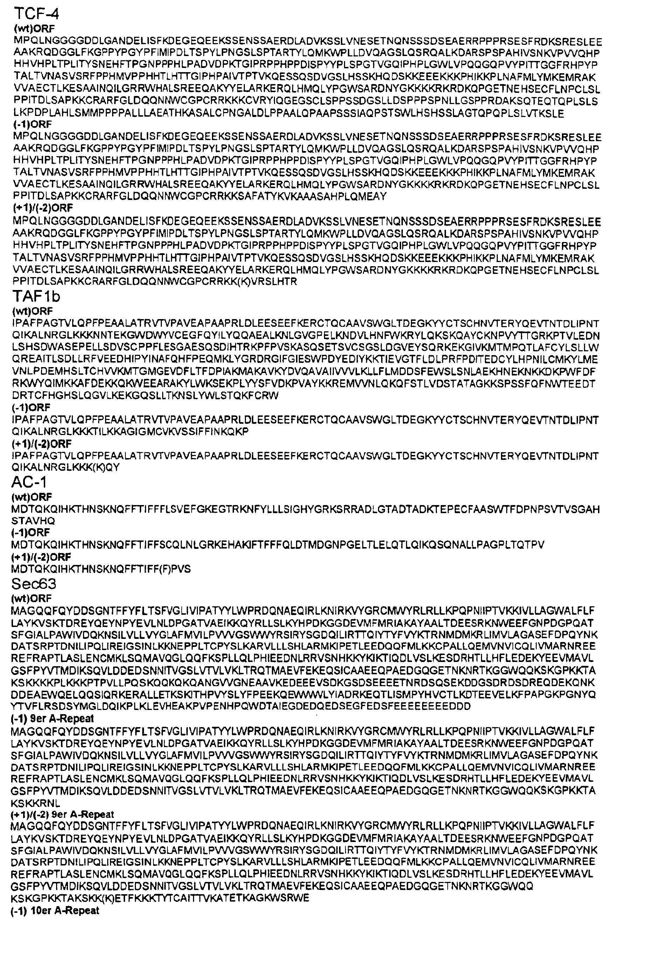

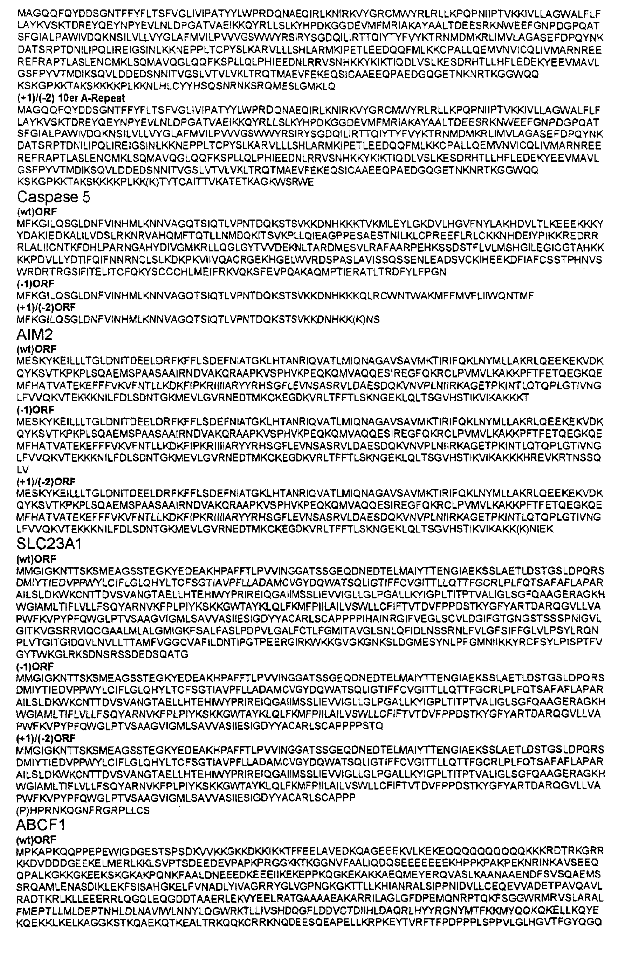

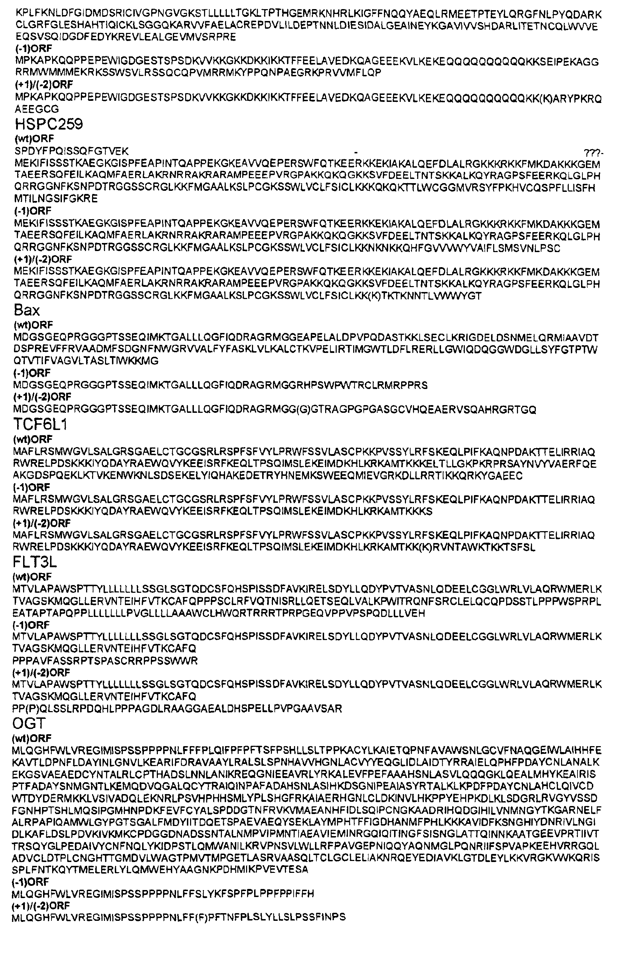

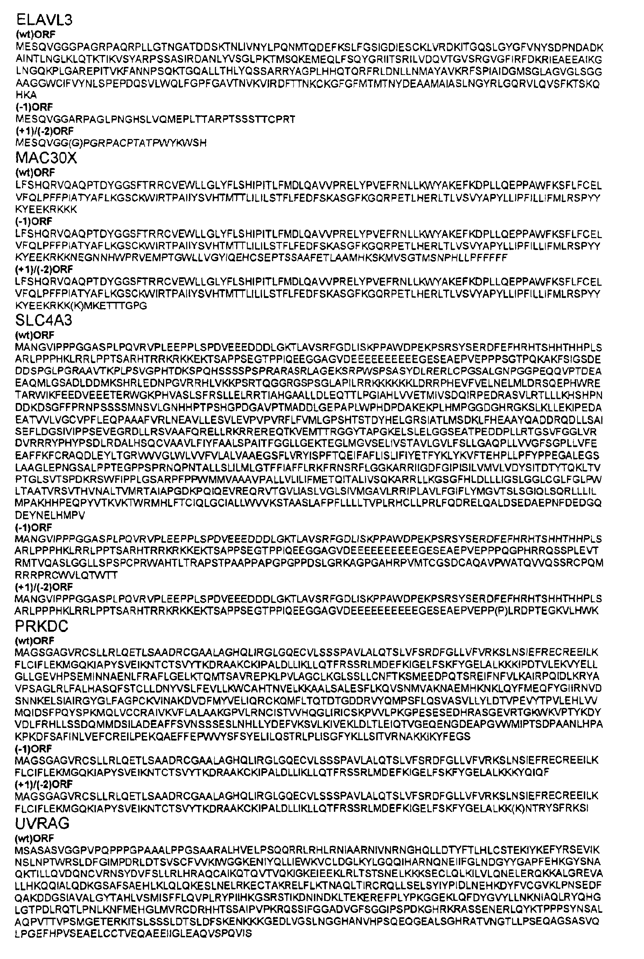

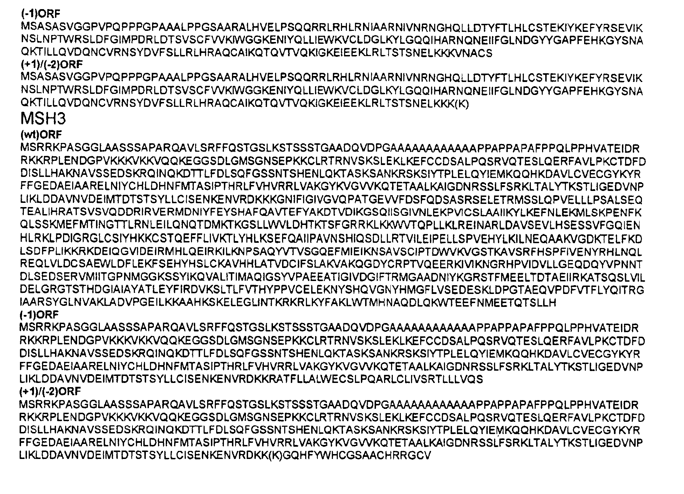

A second aspect influencing the choice of a suitable set of frameshift polypeptides concerns the type of mutation found in the microsatellite region. Frameshift mutations microsatellites are usually due to DNA polymerase slippage and may be characterized by the type of the repeat. Thus in mono nucleotide repeats this type of mutation renders 1 nt insertion or deletion. In dinucleotide repeats and tetranucleotide repeats mutations are insertions or deletions of 2 or 4 nt respectively (Polypeptides encoded by genes with coding microsatellites and the respective polypeptides encoded by genes with frameshift mutations are given in Figure 5). For example commonly (-1) mutations occur in mononucleotide repeats (MNRs). In these mutations one nucleotide is deleted such that the reading frame is shifted by one nucleotide toward the 5' end of the gene compared to the original reading frame. This type of mutation renders a reading frame identical to that produced by (+2) mutations, which arise from two nucleotide insertions in the respective microsatellites. The respective (-1) or (+2) polypeptides might differ by one amino acid. The other frameshift mutation variant leading to frameshift mutations differing from (-1) mutations concerning the resulting reading frame is the (+1) mutation, arising from one nucleotide insertion, thus rendering a reading frame shifted one nucleotide toward the 3' end of the gene compared to the original reading frame. This mutation type gives a reading frame identical to that of (-2) mutations, wherein the encoded polypeptides differ by one amino acid. (-3) and (+3) mutations are irrelevant according to the present invention, for they do not give rise to frameshift polypeptides. According to the present invention the polypeptides included within the set shall be selected as to cover the widest possible range of tumours. Thus a set comprises for example (-1 ) frameshift polypeptides and additionally (+1) frameshift polypeptides. Using more than one possible novel reading frame of the particular gene broadens the spectrum of target cells and thus may prevent escape of particular cells from being eliminated according to the present invention.

A further aspect influencing the choice of the member polypeptides included in a set of frameshift polypeptides according to the present invention is the involvement of the gene encoded for by the coding sequence containing the respective microsatellite in particular biochemical pathways. The term biochemical pathway is used with a rather broad meaning herein. Biochemical pathways as used within this document shall for example include signal transduction pathways, enzymatic pathways, metabolic pathways, the apoptosis pathway, DNA repair or polymerization pathways, the pathway of meiosis etc.. To broaden the spectrum of tumours to be addressed by the set of frameshift polypeptides, members of different pathways are included in the set. In a preferred embodiment at least 5 different pathways, in a more preferred embodiment at least 4 different pathways and in the most preferred embodiment at least 3 different pathways are represented by the frameshift polypeptides in a set according to the present invention. For example the TGFβRII as a member of a signal transduction pathway may be used in combination with the BAX gene as a member of the apoptosis pathway with additional other polypeptides associated with other pathways.

A final aspect influencing the choice of suitable frameshift polypeptides to be included in the set according to the present invention is the length of the novel (poly)peptide sequence arising from the mutation. The shift of the reading frame leads to novel stop codons. Thus the new peptides are not of the same length as the polypeptides naturally encoded by the particular gene. In most cases the new peptides are shorter or even significantly shorter than the original wild-type polypeptide. Frequently rather oligopeptides arise from the frameshift mutations. The fidelity of the immune system in recognizing "foreign" molecules reaches thus far, to identify even polypeptides, that differ from "own" polypeptides in only one single amino-acid mutation. The fragments, bound by the antigen presenting HLA molecules comprise about 12 amino acid residues. To enhance the fidelity of recognition of new polypeptides more than one single amino-acid difference should be present. A polypeptide comprising 3 consecutive amino acids differing from the wild-type amino acid sequence is reliably recognized as foreign by the immune system. This may be due to the increased probability of new amino acid combinations being present in different fragments produced by the antigen presenting machinery. Thus the frameshift polypeptides according to the present invention contain at least one new amino acid, not present in the wild-type polypeptide, in a more preferred embodiment at least 2 new amino acids and in the most preferred embodiment at least 3 new amino acids.

According to the named parameters a basic set of frameshift polypeptides may be tailored, that is suitable to address a large variety of tumours and minimizes the danger of escape of single tumour cells from the therapy. Thus the probability of survival of tumour cells in an organism following immuno-therapy can be minimized and the rate of recurrence of the cancer can be reduced.

A basic set of frameshift polypeptides includes frameshift polypeptides, that do occur with a high mutation frequency in associated disorders. Additionally the polypeptides within the set are chosen to be involved in different biochemical pathways. The mutation types are chosen, that polypeptides of a minimal length of 3 amino acid residues is expressed from the mutated nucleic acid sequence. Furthermore different mutation types of one single microsatellite may be included in the set if applicable.

Additional to the parameters given above, data concerning the particular disorder in focus are taken into account for the design of particular sets of frameshift peptides according to the present invention. Thus individual mutation frequencies, typical mutation types, relevant biochemical pathways or special immunological characteristics may contribute to the set to be used in particular cases. Furthermore in particular cases based on results of examination of particular samples of individuals vaccines may be tailored as to optimally fit the therapy of the respective disorder. In one embodiment of the present invention individual tumour vaccine compositions may be set up according to molecular profiling of individual tumours

Choosing suitable combinations of the peptides within the mixture of frameshift peptides thus enables for generation of vaccines, that elicit immune response specifically for tumours of particular organs. On the other hand it is possible to design sets of frameshift polypeptides that cover a wide range of degenerative disorders or cancers in individuals. The first possibility may be especially useful for the design of curative treatment, whereas the second variant of sets may be of special interest for the design of preventive vaccines.

The compositions and methods according to the present invention may be applied to any eukaryotic organisms exhibiting an immunologic defense system. The eukaryotic organisms are for example animals of agricultural value such as pigs, cows, sheep, etc., companion animals, such as cats, dogs, horses etc., animals employed in research purposes such as mice, rats, rabbits, hamsters etc. or humans.

According to the present invention frameshift polypeptides that comprise an immunogenic portion may be used for immunotherapy for the treatment of cancer. Immunotherapy may be broadly classified into either active or passive immunotherapy. In active immunotherapy, treatment relies on the in vivo stimulation of the endogenous host immune system to react against tumours with the administration of immune response-modifying agents (for example, tumour vaccines, bacterial adjuvants, and/or cytokines). A patient may be afflicted with disease, or may be free of detectable disease. Accordingly, the compounds disclosed herein may be used to treat cancer or to inhibit the development of cancer. The compounds are preferably administered either prior to or following primary treatment of tumours such as surgical removal of the tumours, treatment by administration of radiotherapy and/or conventional chemotherapeutic drugs or any other mode of treatment of the respective cancer or its precursors.

In passive immunotherapy, treatment involves the delivery of biologic reagents with established tumour-immune reactivity (such as effector cells or antibodies) that can directly or indirectly mediate antitumour effects and does not necessarily depend on an intact host immune system. Examples of effector cells include T lymphocytes (for example, CD8+ cytotoxic T-lymphocytes, CD4+ T-helper, tumour-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Pat. No. 4,918,164), for passive immunotherapy.

The predominant method of procuring adequate numbers of T-cells for adoptive transfer immunotherapy is to grow immune T-cells in vitro. Culture conditions for expanding single antigen-specific T-cells to several billion in number with retention of antigen recognition in vivo are well known in the art. These in vitro culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T-cell cultures in order to generate sufficient number of cells for immunotherapy. In particular, antigen-presenting cells, such as dendritic-, macrophage- or B-cells, may be pulsed with immunoreactive polypeptides or transfected with a nucleic acid sequence(s), using standard techniques well known in the art. For example, antigen presenting cells may be transfected with a nucleic acid sequence, wherein said sequence contains a promoter region appropriate for increasing expression, and can be expressed as part of a recombinant virus or other expression system. For cultured T-cells to be effective in therapy, the cultured T-cells must be able to grow and distribute widely and to survive long term in vivo. Studies have demonstrated that cultured T-cells can be induced to grow in vivo and to survive long term in substantial numbers by repeated stimulation with antigen supplemented with IL-2 (see, for example, Cheever, M., et al, "Therapy With Cultured T-Cells: Principles Revisited," Immunological Reviews, 157:177, 1997).

According to the present invention sets of frameshift polypeptides may be employed to generate and/or isolate tumour-reactive T-cells, which can then be administered to the patient. In one technique, antigen-specific T-cell lines may be generated by in vivo immunization with short peptides corresponding to immunogenic portions of the disclosed polypeptides. The resulting antigen specific CD8+ CTL or CD4+ T-helper cells clones may be isolated from the patient, expanded using standard tissue culture techniques, and returned to the patient.

Alternatively, peptides corresponding to immunogenic portions of the polypeptides of the invention may be employed to generate tumour reactive T-cell subsets by selective in vitro stimulation and expansion of autologous T-cells to provide antigen-specific T-cells which may be subsequently transferred to the patient as described, for example, by Chang et al. (Crit. Rev. Oncol. Hematol., 22(3), 213, 1996). Cells of the immune system, such as T-cells, may be isolated from the peripheral blood of a patient, using a commercially available cell separation system, such as CellPro Incorporated's (Bothell, Wash.) CEPRATE.TM. system (see U.S. Pat. No. 5,240,856; U.S. Pat. No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T-cells. The population of tumour antigen-specific T-cells is then expanded using standard techniques and the cells are administered back to the patient.

In another embodiment, T-cell receptors and/or antibodies specific for the polypeptides can be cloned, expanded, and transferred into other vectors or effector cells for use in adoptive immunotherapy.

In a further embodiment, syngeneic or autologous dendritic cells may be pulsed with peptides corresponding to at least an immunogenic portion of a polypeptide disclosed herein. The resulting antigen-expressing and/or presnting dendritic cells may either be transferred into a patient, or employed to stimulate T-cells to provide antigen-specific T-cells which may, in turn, be administered to a patient. The use of peptide-pulsed dendritic cells to generate antigen-specific T-cells and the subsequent use of such antigen-specific T-cells to eradicate tumours in a murine model has been demonstrated by Cheever et al, (Immunological Reviews, 157:177, 1997).

Monoclonal antibodies directed against frameshift peptides presented on cellular membranes may according to the present invention also be used as therapeutic compounds in order to diminish or eliminate tumours. The antibodies may be used on their own (for instance, to inhibit metastases) or coupled to one or more therapeutic agents. Suitable agents in this regard include radionuclides, differentiation inducers, drugs, toxins, and derivatives thereof. Preferred radionuclides include 90Y, 1231, 1251, 1311, 186Re, 188Re, 211At, and 212Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

Pharmaceutical compositions useful in immuno-therapy according to the present invention may comprise a set of at least 5 to 10 frameshift polypeptides (or variants thereof), and a physiologically acceptable carrier. The vaccines may additionally comprise a non-specific immuneresponse enhancer, wherein the non-specific immune response enhancer is capable of eliciting or enhancing an immune response to an exogenous antigen. Examples of non-specific-immune response enhancers include adjuvants, biodegradable microspheres (e.g., polylactic galactide) and liposomes (into which the polypeptide is incorporated). Pharmaceutical compositions and vaccines may also contain other epitopes of tumour antigens, either incorporated into a fusion protein or present within a separate polypeptide.

Alternatively, a pharmaceutical composition or vaccine suitable for immunotherapy according to the present invention may contain nucleic acids, that code for one or more frameshift polypeptides according to the present invention. Nucleic acids may for example include single-stranded (sense or antisense) or double-stranded molecules, and may be DNA (genomic, cDNA or synthetic) or RNA. RNA molecules comprise as well HnRNA (containing introns) as mRNA (not containing introns). According to the present invention the polynucleotides may also be linked to any other molecules, such as support materials or detection marker molecules, and may, but need not, contain additional coding or non-coding sequences. The nucleic acid may be administered in a way that allows the polypeptides to be generated in situ. Suitable expression systems are known to those skilled in the art. The expression of the polypeptides may for example be persistent or transient. In pharmaceutical compositions and/or vaccines, providing for in-situ expression of polypeptides, the nucleic acids may be present within any suitable delivery system known to those of ordinary skill in the art, including nucleic acid expression systems, bacteria and viral expression systems. Appropriate nucleic acid expression systems comprise the necessary regulatory nucleic acid sequences for expression in the patient, such as suitable promoters, terminators etc.. Bacterial delivery systems may for example employ the administration of a bacterium that expresses an epitope of a cell antigen on its cell surface. In a preferred embodiment, the nucleic acid may be introduced using a viral expression system such as e.g., vaccinia virus, retrovirus, or adenovirus, which may involve the use of a non-pathogenic, replication competent virus. Suitable systems are known to those of ordinary skill in the art and are disclosed, for example, in Fisher-Hoch et al., PNAS 86:317-321, 1989; Flexner et al., Ann. N.Y. Acad Sci. 569:86-103, 1989; Flexner et al., Vaccine 8:17-21, 1990; U.S. Pat. Nos. 4,603,112, 4,769,330, and 5,017,487; WO 89/01973; U.S. Pat. No. 4,777,127; GB 2,200,651; EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207,1993.

In another embodiment transgenic mammalian cells may be used for delivery and/or expression of the nucleic acids. The methods for producing nucleic acid constructs suitable for in-situ expression of polypeptides are known to those of skill in the art.

Furthermore the nucleic acid may be administered as naked nucleic acids. In this case appropriate physical delivery systems, which enhance the uptake of nucleic acid may be employed, such as coating the nucleic acid onto biodegradable beads, which are efficiently transported into the cells. Administration of naked nucleic acids may for example be useful for the purpose of transient expression within a host or host cell.

The pharmaceutical compositions used for immuno-therapy according to the present invention may be administered by any suitable way known to those of skill in the art. The administration may for example comprise injection, such as e.g., intracutaneous, intramuscular, intravenous or subcutaneous injection, intranasal administration for example by aspiration or oral administration. A suitable dosage to ensure the pharmaceutical benefit of the treatment should be chosen according to the parameters, such as age, sex, body weight etc. of the patient, known to those of skill in the art.

The type of carrier to be employed in the pharmaceutical compositions of this invention, will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (e.g., polylactic glycolide) may also be employed as carriers for the pharmaceutical compositions of this invention. Suitable biodegradable microspheres are disclosed, for example, in U.S. Pat. Nos. 4,897,268 and 5,075,109. Any of a variety of immune-response enhancers may be employed in the vaccines of this invention. For example, an adjuvant may be included. Most adjuvants contain a substance designed to protect the antigen from rapid catabolism, such as aluminum hydroxide or mineral oil, and a nonspecific stimulator of immune response, such as lipid A, Bordetella pertussis or Mycobacterium tuberculosis. Such adjuvants are commercially available as, for example, Freund's Incomplete Adjuvant and Complete Adjuvant (Difco Laboratories, Detroit, Mich.) and Merck Adjuvant 65 (Merck and Company, Inc., Rahway, N.J.).

The pharmaceutical compositions or immuno-therapeutic methods according to the present invention may be used for the treatment of degenerative disorders or cancers. For example the compositions and methods may be employed in the therapy of diagnosed cancers in order to eliminate the tumour cells from the affected organism. As well primary tumours as metastases or disseminated tumour cells within an organism may be targets to the therapeutic compounds and methods disclosed herein.

Furthermore the compositions and methods of the invention may be employed in the treatment of pre-neoplastic conditions. In this case the pre-neoplastic cells or tissues may be directly addressed by the immuno-therapeutic compositions or methods, or may be hindered from evolving into neoplastic or dysplastic conditions. For example in this case the pre-neoplastic condition may be treated preventively. By the vaccination the immune response may be elicited, so that emerging neoplasms may be destroyed.

The methods and compositions according to the present invention may also be used for the prevention of degenerative disorders or cancers associated with frameshift mutations in coding microsatellites. For this purpose a vaccination of a population of organisms or of subgroups of said population may be performed. The subgroups may be built by suitable parameters such as hereditary predisposition for the emergence of degenerative disorders, exposure to factors, that increase the risk of being affected by said disorders etc.

The present invention provides compositions and methods for enhanced immunotherapy of disorders associated with MSI⁺ related occurrence of frameshift peptides. The invention provides sets for the immuno-therapy of said disorders, that address a wide range of different types of disorders in an organism and may thus be employed as a preventive vaccine against said disorders. Furthermore the invention also provides sets of polypeptides useful for the treatment of particular types of disorders. The use of sets of frameshift polypeptides for the immuno-therapy according to the present invention provides the means for a reliable treatment of degenerative disorders and cancers associated with frameshift mutations in coding microsatellites reducing the risk of escape of several tumour cells or of a population of tumour cells from being addressed by the therapy. Thus the method of the invention reduces the risk of recurrence of tumours after immuno-therapy employing CTLs, T-helper cells and possibly specific antibody producing B-cells raised against frameshift polypeptides characteristic for tumour cells.

### Brief description of the drawings:

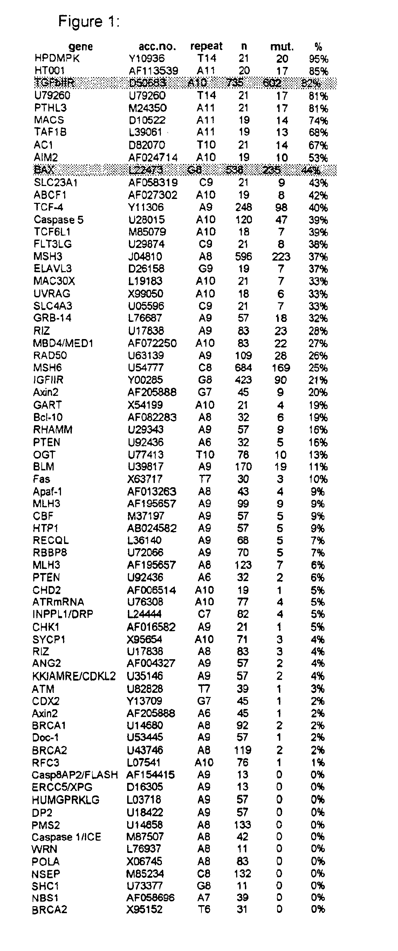

- Figure 1:: **Coding microsatellite genes investigated in MSI-H colorectal cancer.** Repeat: type of nucleotide and length of repeat tract, n: number of samples investigated, mut.: number of samples mutated, %: percentage of mutated samples,

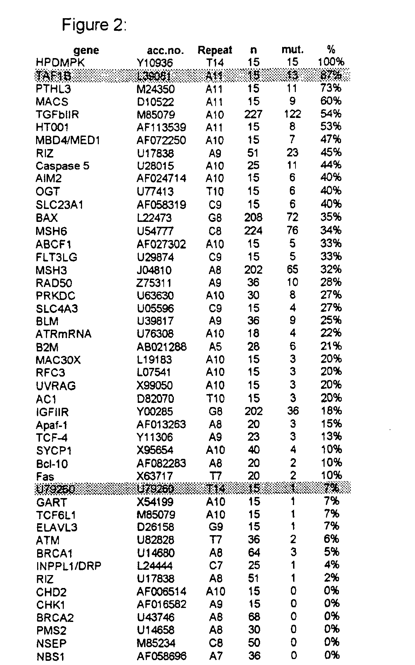

- Figure 2:: **Coding microsatellite genes investigated in MSI-H gastric cancer.** Repeat: type of nucleotide and length of repeat tract, n: number of samples investigated, mut.: number of samples mutated, %: percentage of mutated samples

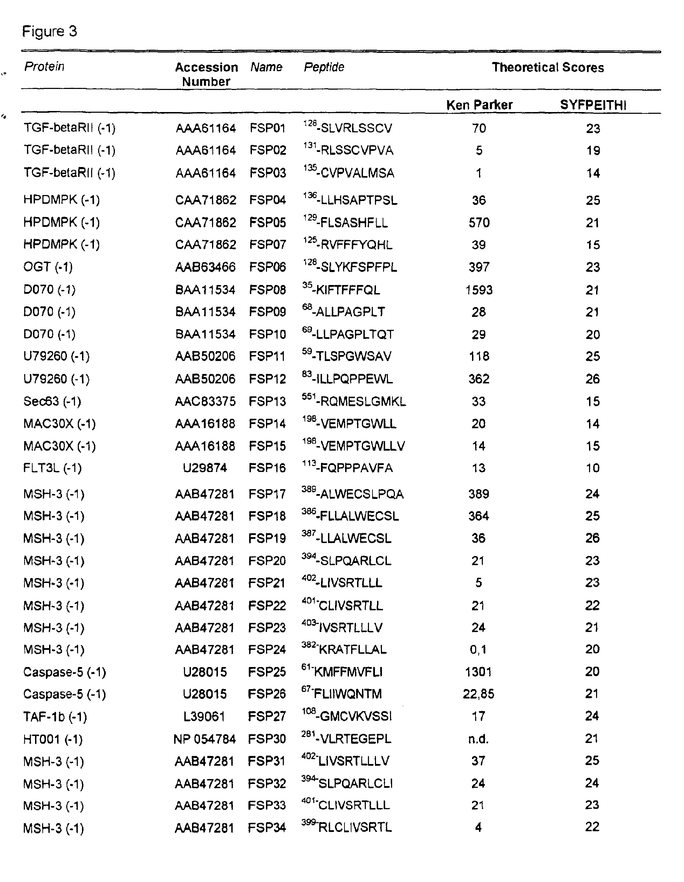

- Figure 3:: **Frameshift Peptides analysed for in vitro stimulation of a cellular immune response;** All analysed peptides are derived from (-1) mutations in microsatellites of the cognate proteins; The protein or nucleotide accession numbers are indicated within the table; the position of the start amino acid in the protein is indicated in the tables; the predicted binding scores to HLA-A2.1 using computer assisted analysis; for experimental details see Example 1

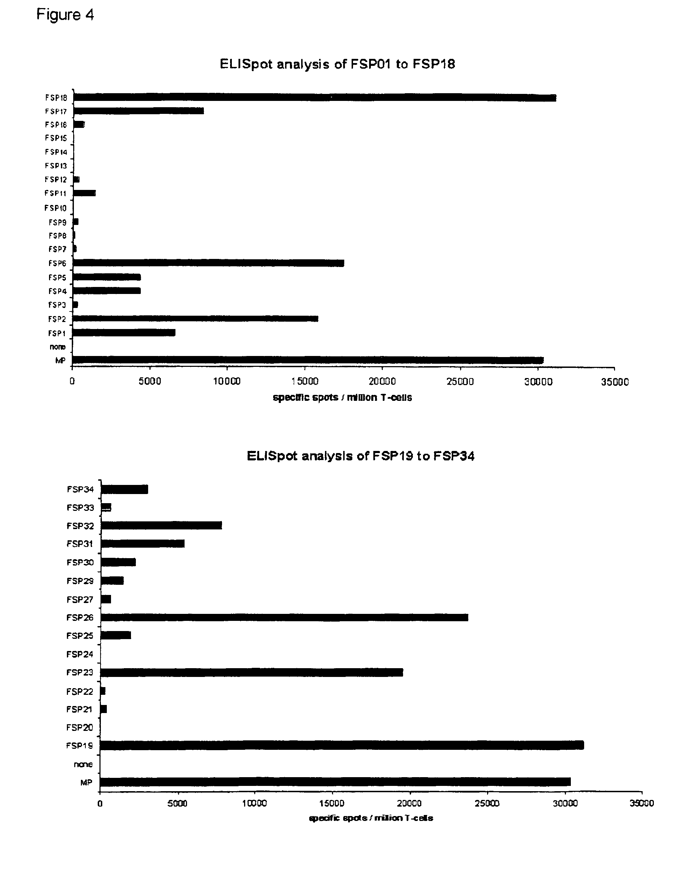

- Figure 4:: **ELISpot-analysis of FSP T-cell lines** (FSP01-FSP34). Titrated amounts of T-cells were incubated overnight with 3.5x10⁴ peptide loaded T2 cells per well. The number of IFN-γ-releasing activated T-cells (spots) among the total number of cells analyzed (10⁶) is depicted for each frameshift peptide. For experimental details see Example 3.
- Figure 5:: **Listing of sequences of polypeptides encoded by genes with coding microsatellites;** the sequences of polypeptides arising from different possible frameshift mutations are depicted. for each polypeptide the sequence expressed from the wild type open reading frame is given (wtORF); Furthermore the sequences expressed from (-1) mutations and, from (-2/+1) mutations are given.

The following examples are given for illustration of the invention only and are not intended to limit the scope of the invention.

### Example 1: Analysis of the mutation frequency of genes harbouring repeat tracts including data from publications and own data

In order to extract cMNRs and cDNRs, we screened the 109.289 entries with human sequences in the EMBL database (EMBL Rel. 62, Mar. 2000). Using various Perl scripts (Wall, Christiansen, & Schwartz 1996), we obtained annotated coding sequence (a total of 46.520 entries) and applied a rigorous redundancy check at the 98% level, thus detecting a total of 33.595 coding sequences in these entries. cDNA entries were compared to genomic DNA to identify corresponding mRNA. If both informations were available for a particular gene, priority was given to the genomic sequence, since repeats in cDNA might span several exons. We finally considered 22.577 cDNAs (mRNAs) and 11.018 coding sequences in genomic entries for further analysis.

In the coding regions, a minimum repeat length of 6 mononucleotides and 4 dinucleotides was required as lower limit of the repeats. In regards to the dinucleotide repeats, all 12 different types of dinucleotide repeats were taken into consideration. cDNA and genomic DNA entries were analyzed separately. Using several filters, repeat tracts within pseudogenes, vector sequences as well as homopolymeric nucleotide stretches at the most 5' or 3' ends of sequences were excluded. All candidate sequences were stored in a relational database for further analysis. Subsequently, two independent BLASTN analyses were performed: initial database analysis was done using the BLASTN included in the HUSAR program package of the German Cancer Research Center in Heidelberg, followed by an Advanced BLAST analysis of the high throughput genomic sequencing (htgs) database (http://www.ncbi.nlm.nih.gov/). Exon-intron boundaries were determined by MALIGN analysis, matching a candidate cDNA with homologous genomic DNA sequences. Finally, true coding microsatellites were verified by direct sequencing of repeat candidates amplified from genomic DNA.

Published mutation data of genes harboring repeat tracts were collected. Numbers of tumour samples analyzed and the number of mutated samples found were sorted by gene and repeat type in a spreadsheet. Individual sample numbers and numbers of mutated samples from each single publication for each repeat tract within a gene were summarized. Subsequently tissue specific cumulative mutation frequencies were calculated. If mutation data were not reported separately for specific repeats, i.e. represented mixed data or if mutation data on tumour cell lines and primary tumour samples were shown as one item, these data were omitted. In order to minimize sampling errors only repeat mutation data obtained from at least 10 tissue samples were considered for statistical analysis.

Additionally investigations were performed regarding the mutation frequencies of unpublished coding microsatellite regions. Nine novel coding microsatellites residing in genes not yet analyzed for frameshift mutations in MSI colorectal, endometrial or gastric tumours have been examined in the course of the studies leading to the present invention. They include three genes containing A11 repeats (TAF1B, MACS, HT001), five genes with A10 repeats (CHD2, UVRAG, TCF6L1, ABCF1, AIM2) and one gene harboring a G9 repeat (ELAVL3). The MSI status of these specimen was determined using the NCI ICG-HNPCC microsatellite reference marker panel (8). PCR reactions were performed as follows:

Genomic DNA was isolated from 5-8 haematoxylin and eosin stained 5 µm sections after microdissection, using the Qiamp Tissue Kit (Qiagen, Hilden, Germany). Preparation of DNA from cell lines was performed according to standard protocols.

PCR primers were designed to closely flank the target sequence, yielding short amplimeres of about 100 base pairs thus allowing precise fragment sizing and robust amplification from archival tissues. PCR reactions were performed in a total volume of 25 µl containing 50 ng genomic DNA, 2.5 µl 10 x reaction buffer (Life Technologies, Karlsruhe, Germany), 1.5 mM MgCl₂, 200 µM dNTPs, 0.3 µM of each primer, and 0.5 U Taq DNA polymerase (Life Technologies) and using the following conditions: initial denaturation at 94 °C for 4 min, followed by 35 cycles of denaturation at 94 °C for 30 s, annealing at 58 °C for 45 s, and primer extension at 72 °C for 30 s. The final extension step was carried out at 72 °C for 6 min. PCR fragments were analyzed on an ALF DNA sequencing device (Amersham Pharmacia Biotech, Freiburg, Germany) using 6.6 % polyacrylamide/ 7 M urea gels. Size, height and profile of microsatellite peaks were analyzed using the AlleleLinks software (Amersham Pharmacia Biotech). Coding microsatellite instability was scored, if smaller or larger-sized amplimeres were detected in tumour DNA compared to DNA from non-neoplastic cells. Allele intensities were determined and ratios of wild-type and novel alleles in normal and tumour tissues were calculated, defining a two-fold difference as threshold for allelic shifts. Similarly, unstable alleles in tumour cell lines were identified by comparison to 36 unmatched normal mucosae. In order to determine the predicted repeat type and length amplified coding microsatellites were subjected to Big Dye terminator cycle sequencing (Perkin Elmer, Darmstadt, Germany) and subsequent analysis on an ABI 310 sequencing device.

The frequencies of mutations in a particular microsatellite region examined herein are given in figure 1. The mutation rates are calculated with respect to the total number of samples included in the studies. The mutations frequencies resulting from studies covering a larger number of samples may be handled to be more significant then those referring to a smaller number of analysed samples.

Overall the results show, that a number of the analysed microsatellite sites is mutated with high frequency over the range of examined samples as well as over the range of published data. This suggests, that according to the present invention a set of frameshift peptides, arising from frequent mutations should be expressed in high percentage of tumours.

### Example 2: Detection of the expression of frameshift mutated mRNA from genes harboring coding microsatellite regions using PCR

Samples of colon, gastric and endometrial carcinomas are used to determine the expression of mRNA showing frameshift mutations in coding microsatellite regions using PCR and subsequent sequencing of the amplified nucleic acid products.

Tumours are collected, snap frozen, and stored at -80°C. They are verified to be composed predominantly of neoplastic cells by histopathological analysis. mRNA is isolated from tumours using Qiagen reagents (Qiagen, Hilden, Germany), and single-stranded cDNA is synthesized using Superscript II (Life Technologies, Inc.).

PCR reactions were performed in a total volume of 25 µl containing 50 ng cDNA, 2.5 µl 10 x reaction buffer (Life Technologies, Karlsruhe, Germany), 1.5 mM MgCl₂, 200 µM dNTPs, 0.3 µM of each primer, and 0.5 U Taq DNA polymerase (Life Technologies) and using the following conditions: initial denaturation at 94 °C for 4 min, followed by 35 cycles of denaturation at 94 °C for 30 s, annealing at 58 °C for 45 s, and primer extension at 72 °C for 30 s. The final extension step was carried out at 72 °C for 6 min. PCR fragments were analyzed on an ALF DNA sequencing device (Amersham Pharmacia Biotech, Freiburg, Germany) using 6.6 % polyacrylamide/ 7 M urea gels.

The experiments described show, that in the cases of the tested genes TGFβRII, BAX, OGT, U79260, CASP 5, MSH 3, HPDMPK, HT001, PTHL3, MACS, TCF4, TAF1B, AC1 and SEC63 mutated coding microsatellite regions are transcribed into mRNA.

These results indicate, that the cells harboring mutations in coding microsatellite regions express neo-polypeptides derived from frameshift mutations.

### Example 3: Stimulation of cellular immune response by frameshift peptides

The present experiments were performed in order to determine, whether the frameshift peptides arising from mutations in coding microsatellite regions according to the present invention are suited to stimulate a cellular immune response. The experiments were performed as follows:

Peptides displaying HLA-A2.1-binding motifs were selected by taking advantage of specific computer programs [(Parker, Bednarek, & Coligan 1994); http://bimas.dcrt.nih.gov/molbio/hla_bind/ and (Rammensee et al. 1999); http://134.2.96.221/scripts/MHCServer.dll/home.htm]. Peptides were purchased from the peptide synthesis unit of the DKFZ. Stock solutions (10mg/ml in DMSO) were stored at -70°C and diluted to 1mg/ml in PBS before use. T2 cells were pulsed with 50µg/ml peptide and 5µg/ml β2-microglobulin (Sigma; Deisenhofen, Germany) overnight at 37°C. The expression of HLA-A2.1 was then analysed by flow cytometry using mAb BB7.2 followed by incubation with FITC-conjugated (Fab')2 goat antimouse Ig (Vonderheide et al. 1999).

Peripheral blood was obtained from a healthy HLA-A2.1 + donor and collected in heparinized tubes. PBMNC were isolated by Ficoll-density gradient centrifugation. Whole CD3+ T-cells were isolated from PBMNC by magnetic depletion of non-T-cells using the MACS Pan T-Cell Isolation Kit (Miltenyi; Bergisch Gladbach, Germany) according to manufacturer's instructions. Preparations contained at least 97% of CD3+ cells as assessed by immunophenotypic analysis.

CD40 Bs of a HLA-A2.1+ donor were incubated with peptide (10µg/ml) and human β2-microglobulin (3µg/ml; Sigma) in serum-free Iscov's DMEM medium for one hour at room temperature, washed twice to remove excess of peptide, were irradiated (30Gy) and added to purified CD3+ autologous T-cells (>97% CD3+) at a ratio of 4:1 (T:CD40 Bs) in Iscov's MEM containing 10% human AB-serum, supplements (1:100) and hIL-7 (10ng/ml, R&D). Cells were plated at a density of 2 x 10⁶ T-cells/well in 1ml of medium. After three days in culture they were fed with 1ml complete medium. For restimulation of T-cells, this was repeated weekly. IL-2 was first given at day 21 (10IU/ml, R&D), also at day 24 and from day 28 on only hIL-2 was used instead of hIL7.

ELISpot assays were performed as described elsewhere (Meyer et al. 1998). Briefly, nitrocellulose-96-well plates (Multiscreen; Millipore, Bedford, USA) were covered with mouse anti-human IFN-g monoclonal antibody (Mabtech, Sweden) and blocked with serum containing medium. Varying numbers of effector cells were plated in triplicates with 3,5x10⁴ peptide-loaded T2 cells per well as targets. After incubation for 18h, plates were washed, incubated with biotinylated rabbit anti-human IFN-g second antibody, washed again, incubated with streptavidin coupled alkaline phosphatase, followed by a final wash. Spots were detected by incubation with NBT/BCIP (Sigma) for 45min, reaction was stopped with water, after drying spots were counted using the KS-ELISpot reader (Zeiss Kontron; Göttingen, Germany).

These procedures were performed for peptides derived from mutations in the coding regions of the following genes: TGFβRII, OGT, U79260, CASP 5, MSH 3, HPDMPK, HT001, TAF1B, D070, MAC30X, FLT3L and SEC63. Peptides are listed in Figure 3. Results for ELIspot analysis is shown in Figure 4.

The analysis shows, that the used peptides are suited to raise an immune response. Peptides arising from frameshift mutations thus may be used to raise immune response for example in the course of vaccinations according to the present invention.

### Example 4: Vaccination therapy using a set of 10 preselected frameshift polypeptides

Each five patients suffering from gastric cancer, colorectal cancer and pancreatic cancer are included in this study. The study is designed as a an open label non randomized study.

A set of 10 frameshift polypeptides preselected by rational considerations is used for carrying out the immunotherapy. The set of polypeptides consisted of frameshift peptides derived from mutations in the coding regions of the following genes: TGFβRII, U79260, CASP 5, HT001, PTHL3, MACS, TCF4, TAF1B, AC1 and SEC63

The peptide-mixture used for vaccination consists of equimolar amounts of the different (10) freeze-dried peptides. The peptides are dissolved in 0.5ml sterile 0.9% NaCl at a final concentration of 1-1.6mg/ml for each peptide.

Vaccination is carried out using 100µl peptide mixture (5 x 10⁻⁵ M concentration of each peptide). The mixture is administered intracutaneously about 20min after injection of 100µl (0.4mg/ml) recombinant human GM-CSF (i.e. Leucomax®, Schering-Plough). 100µl of peptide mixture will be injected intracutaneously at a separate site without GM-CSF as a DTH-test. A DTH-test with each individual peptide is performed at week 6 to distinguish between positive and negative reactions to the individual peptides. The DTH-test is considered positive if the area of the skin reaction has a diameter of at least 5mm.

Vaccination regimen includes 4 vaccinations carried out weekly. The first vaccination is performed a minimum of four weeks after other treatments have been stopped.

The assessment of the tumor response is carried out by CT or X-Ray examination as applicable in the particular case. The assessment is performed two months prior to the first vaccination and two months after the last vaccination.

In the majority of the tested cases a significant reduction of the tumor sizes are detected. In no case included in the study a progression of the tumor is stated. Furthermore no more new lesion emerged during the time course of the study.

The experiments show, that the mixture of the named ten frameshift polypeptides is suitable for immunotherapy of a wide variety of tumor entities.

Further tumor entities to which the presented set will be applicable include breast cancer, cancer of the brain, endometrial cancer and others.

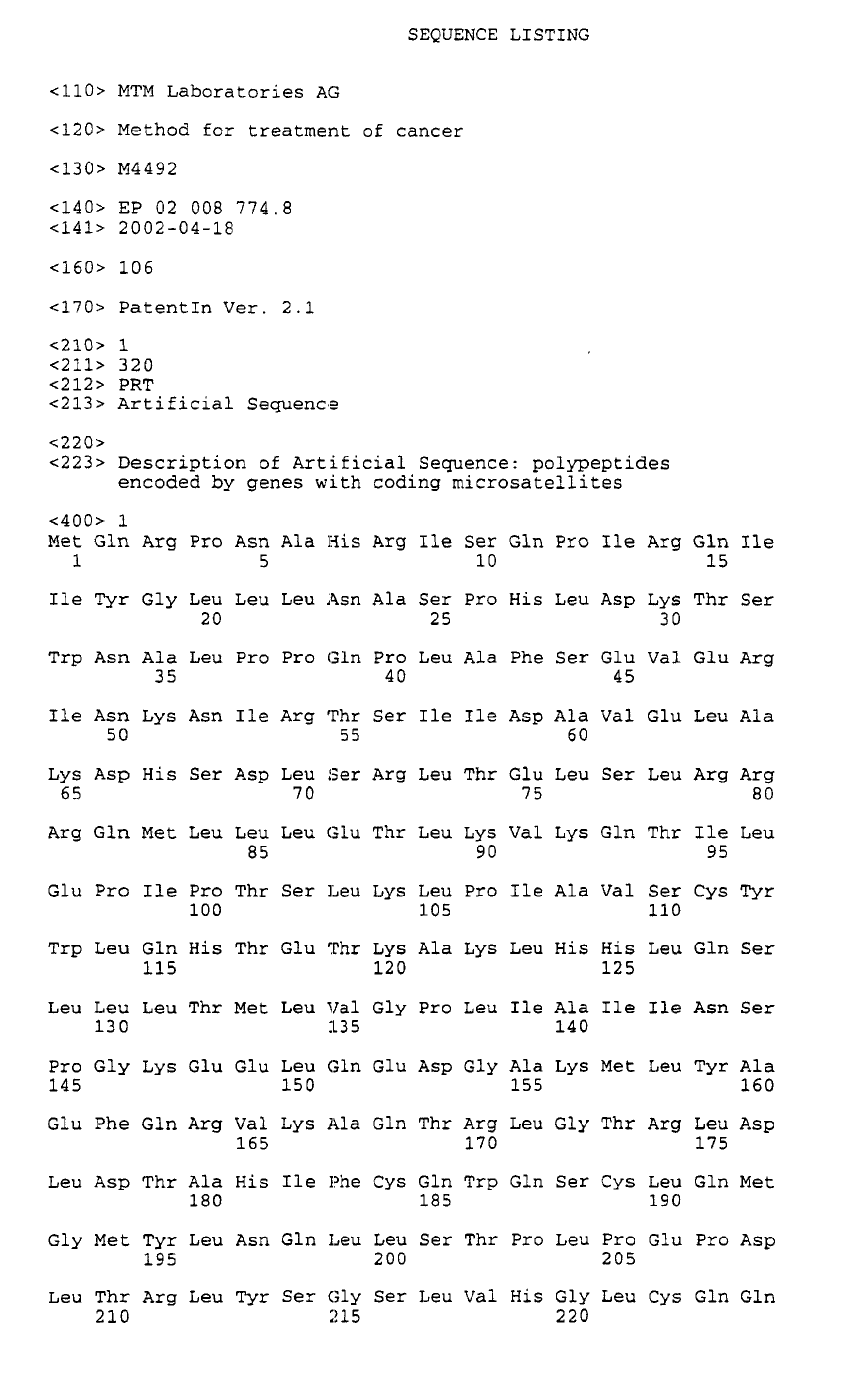

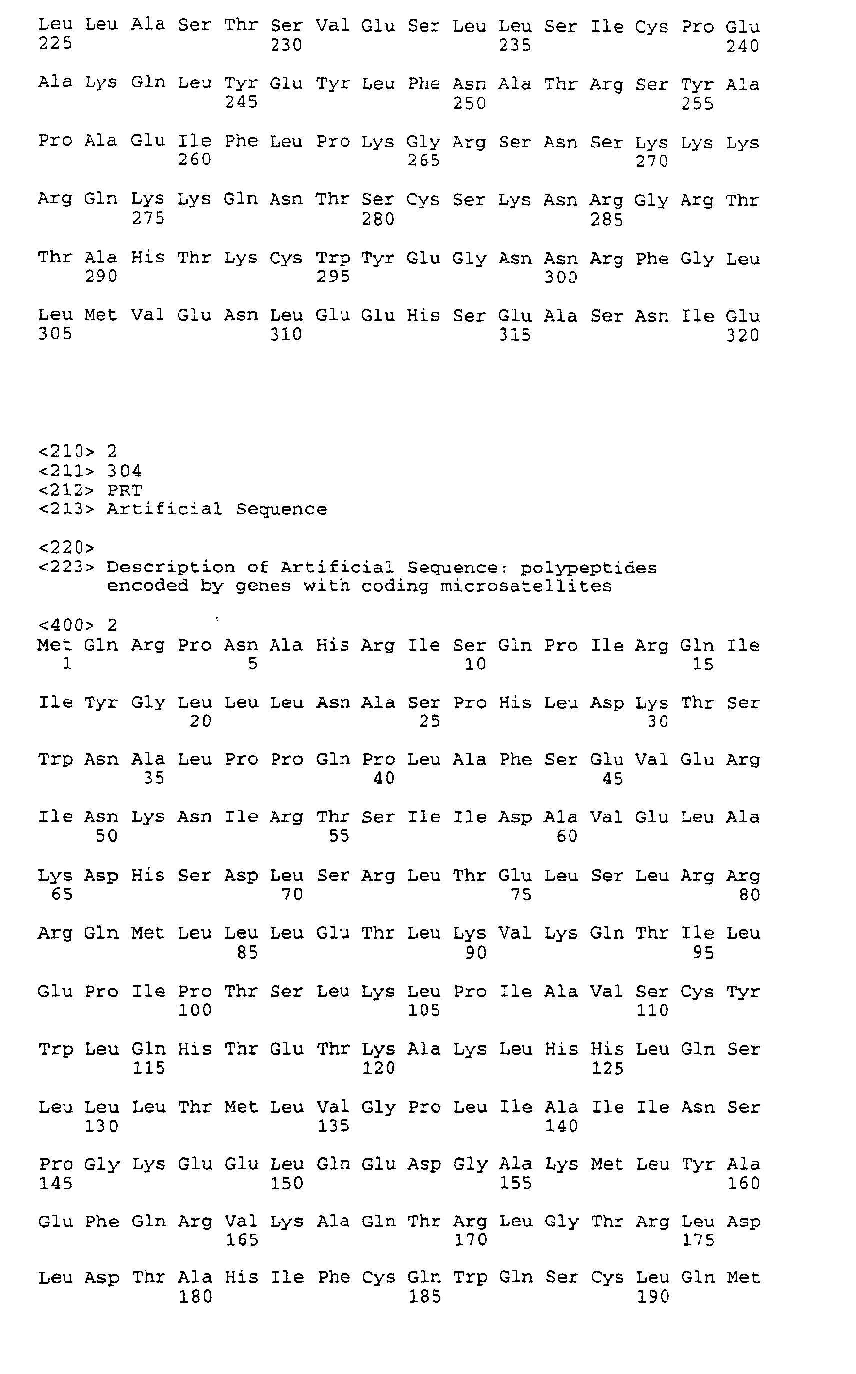

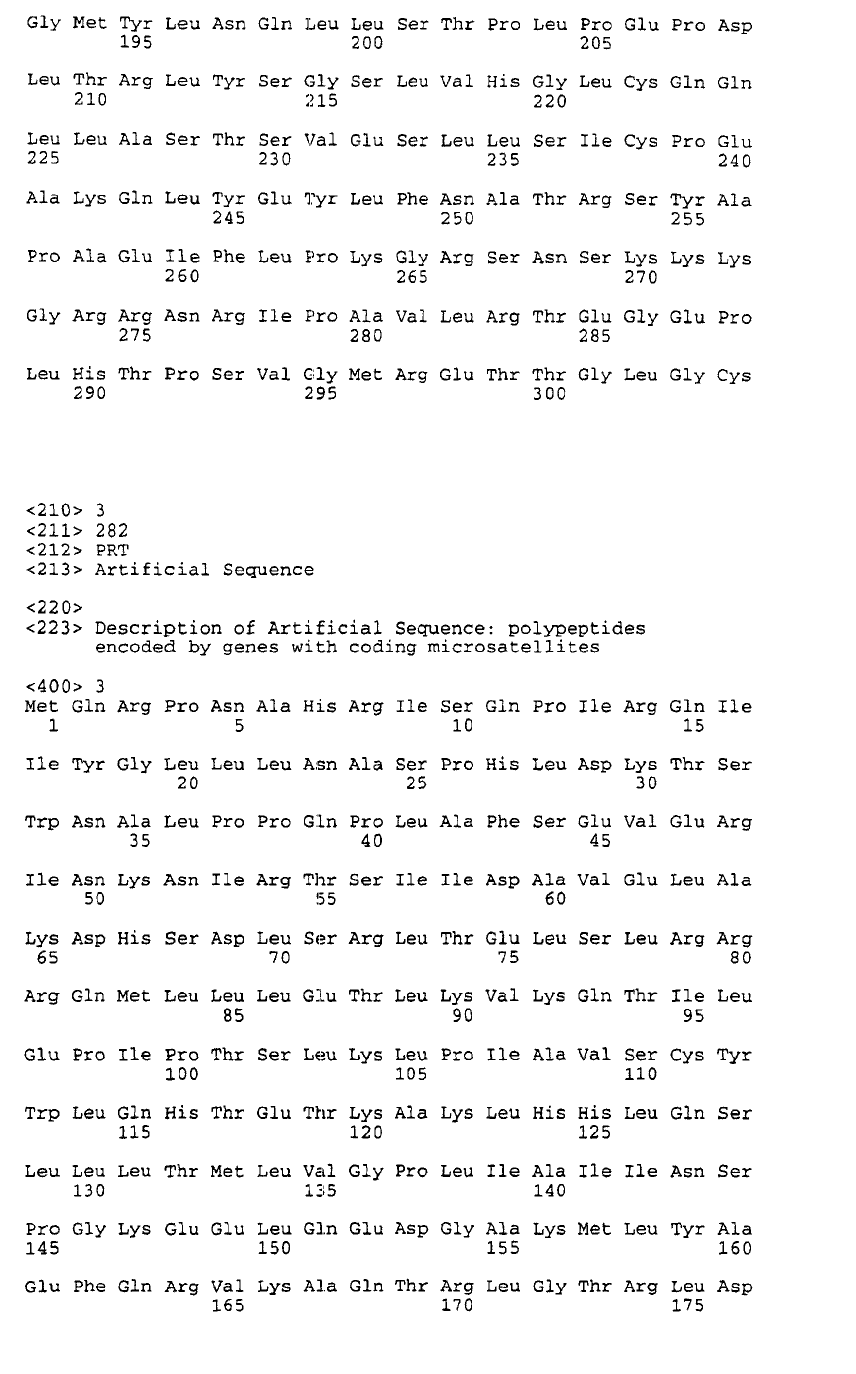

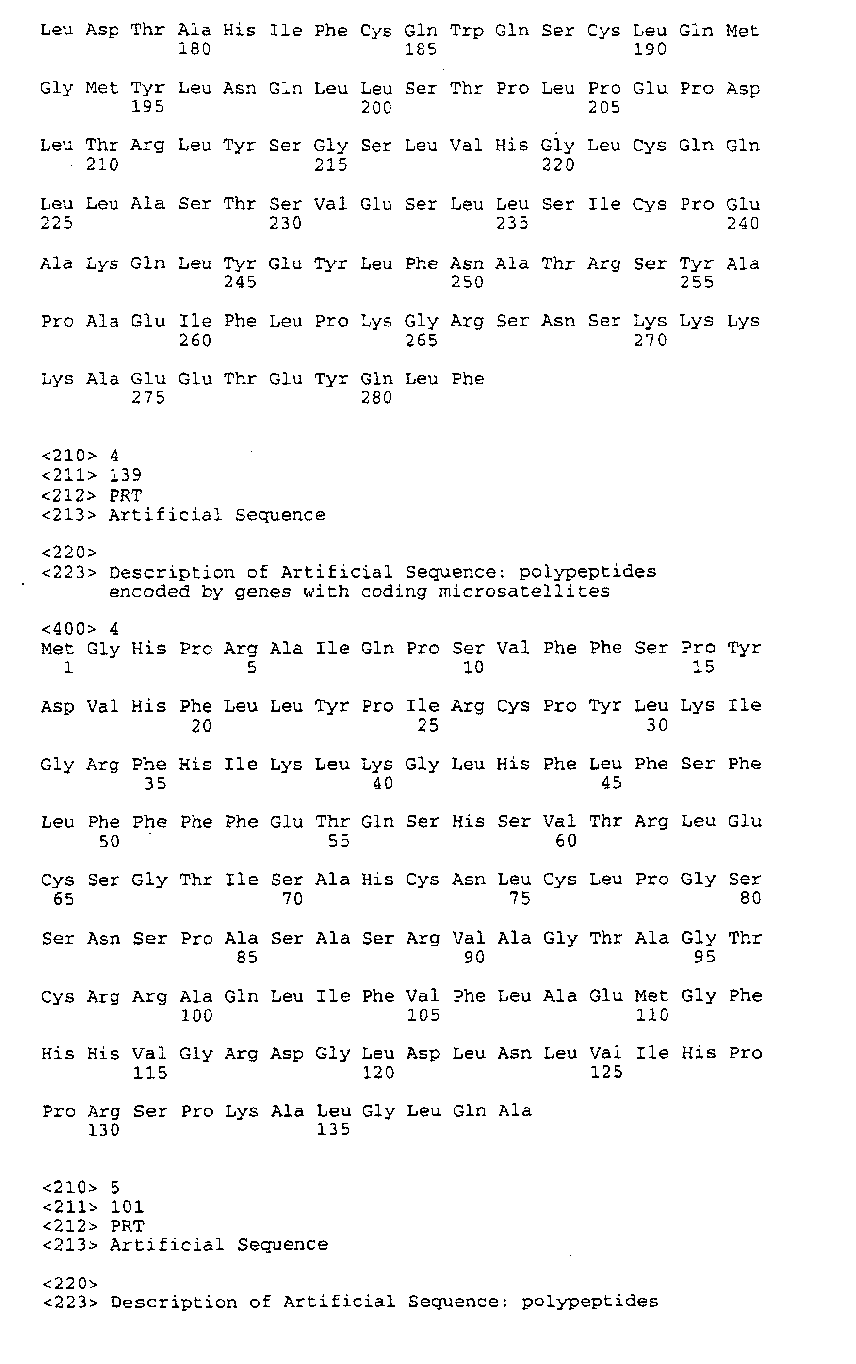

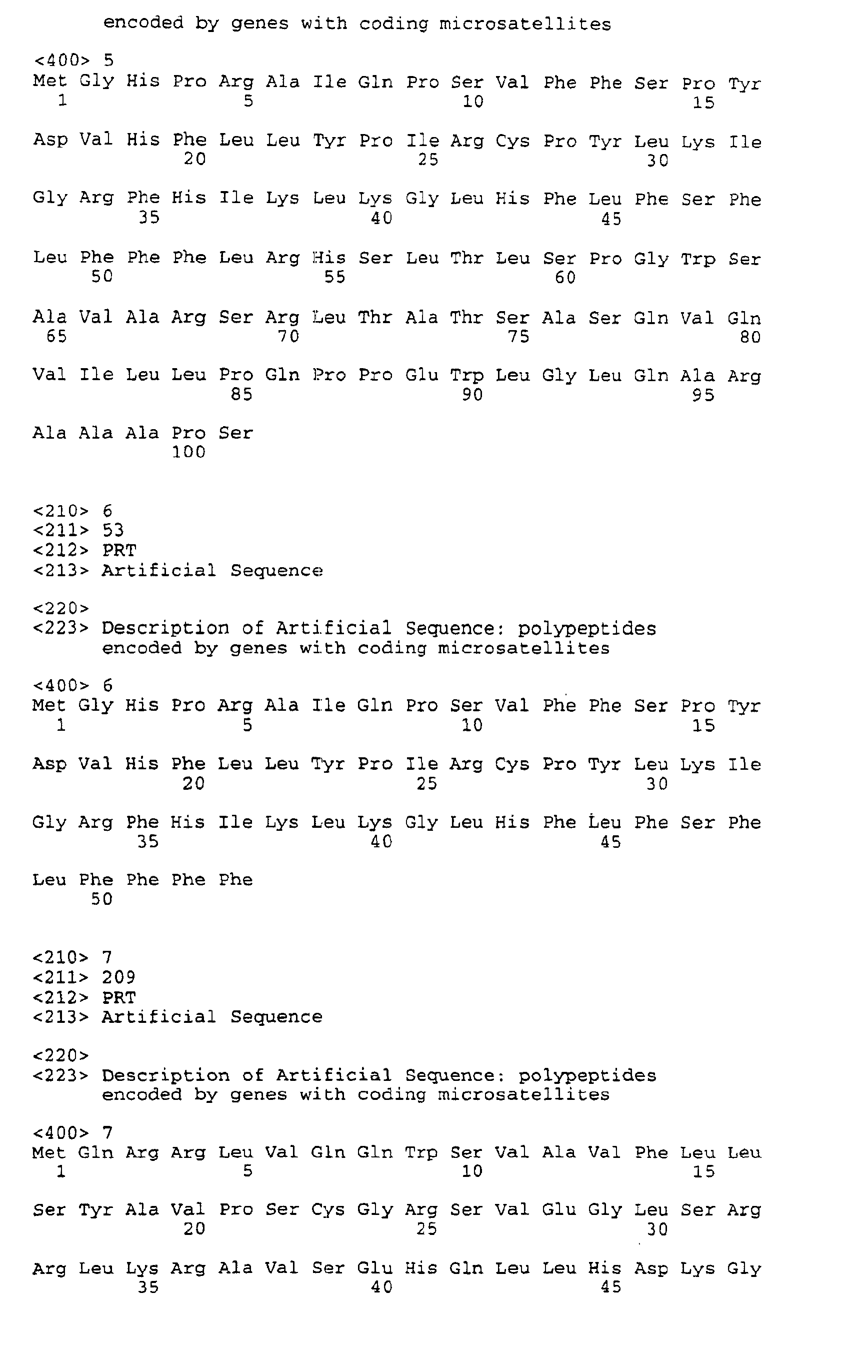

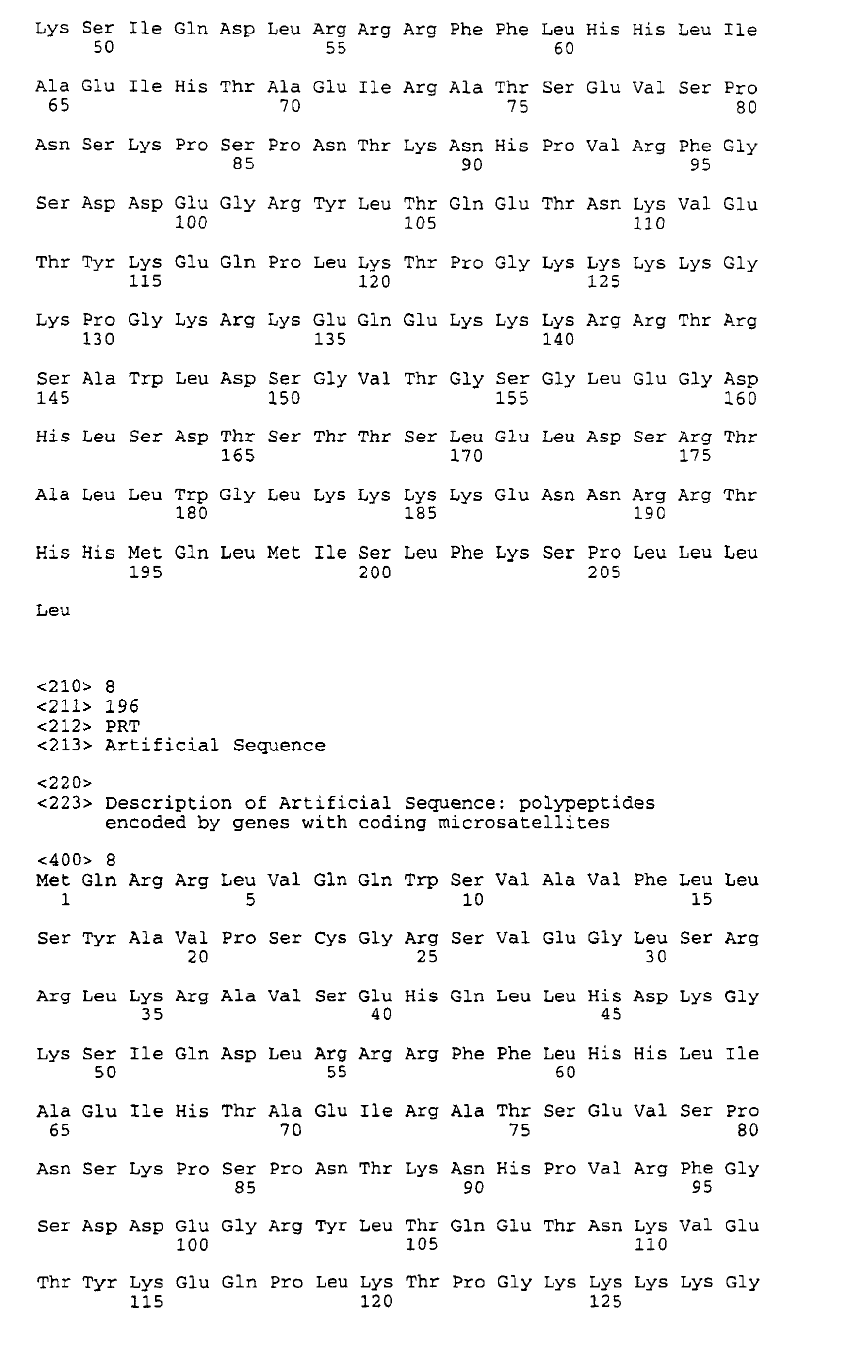

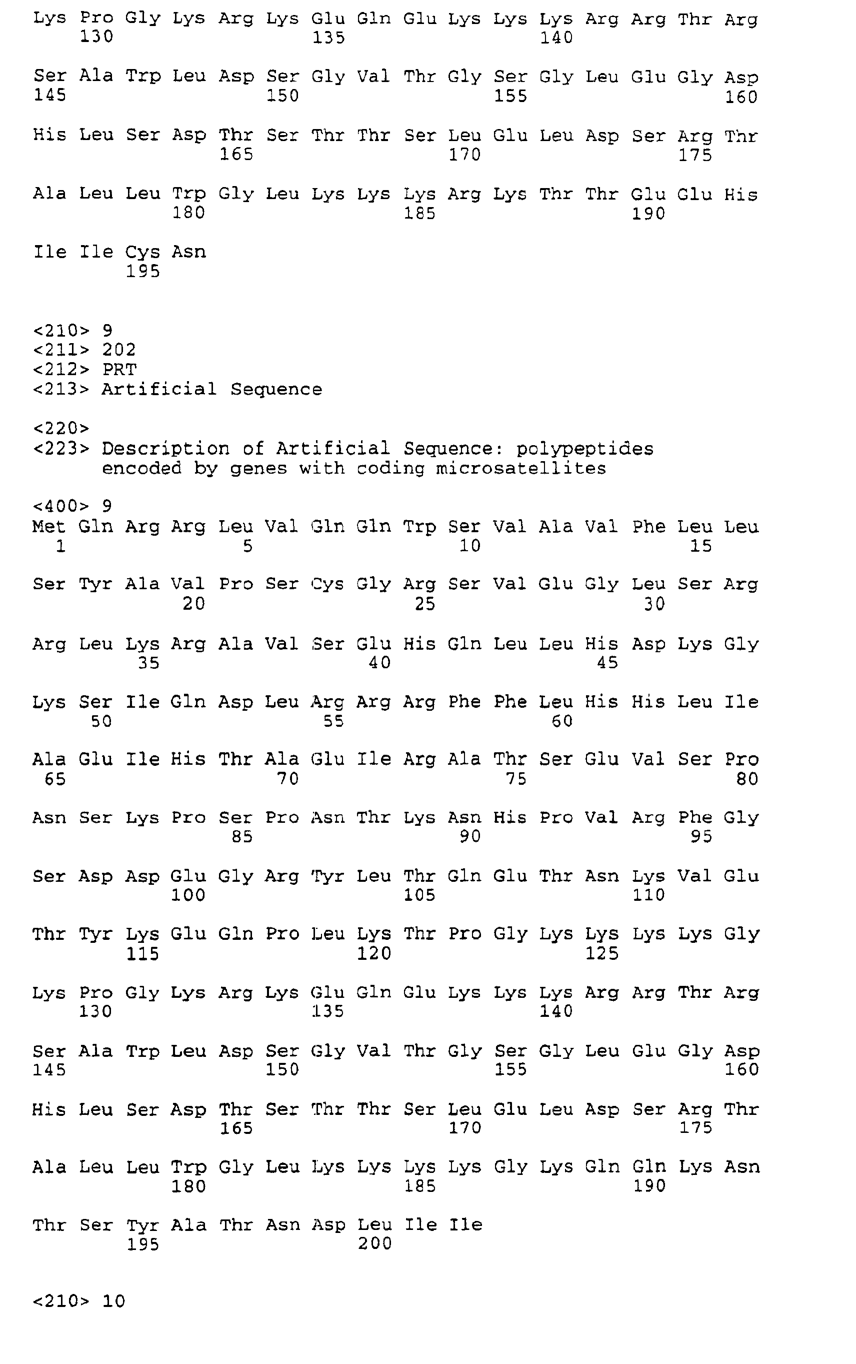

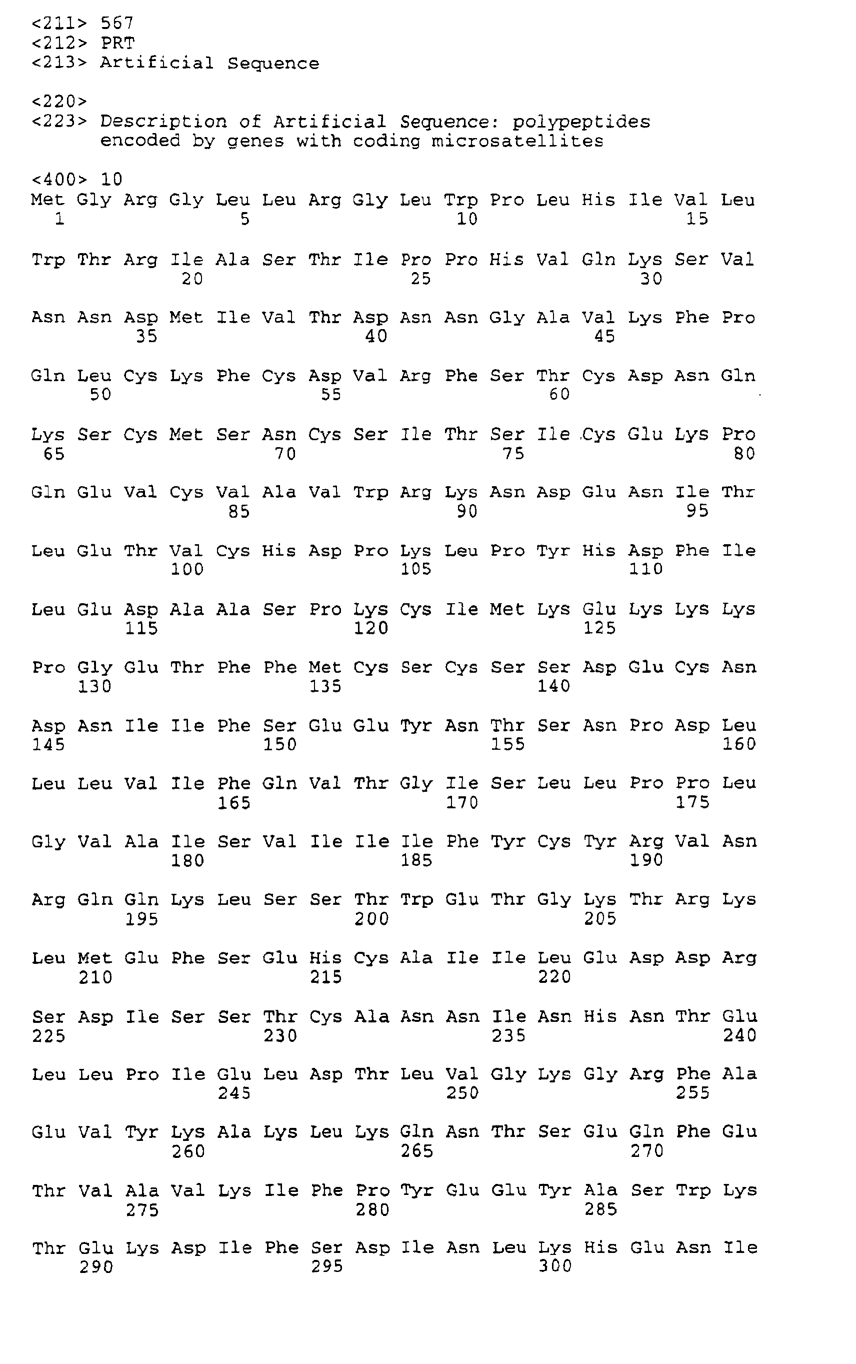

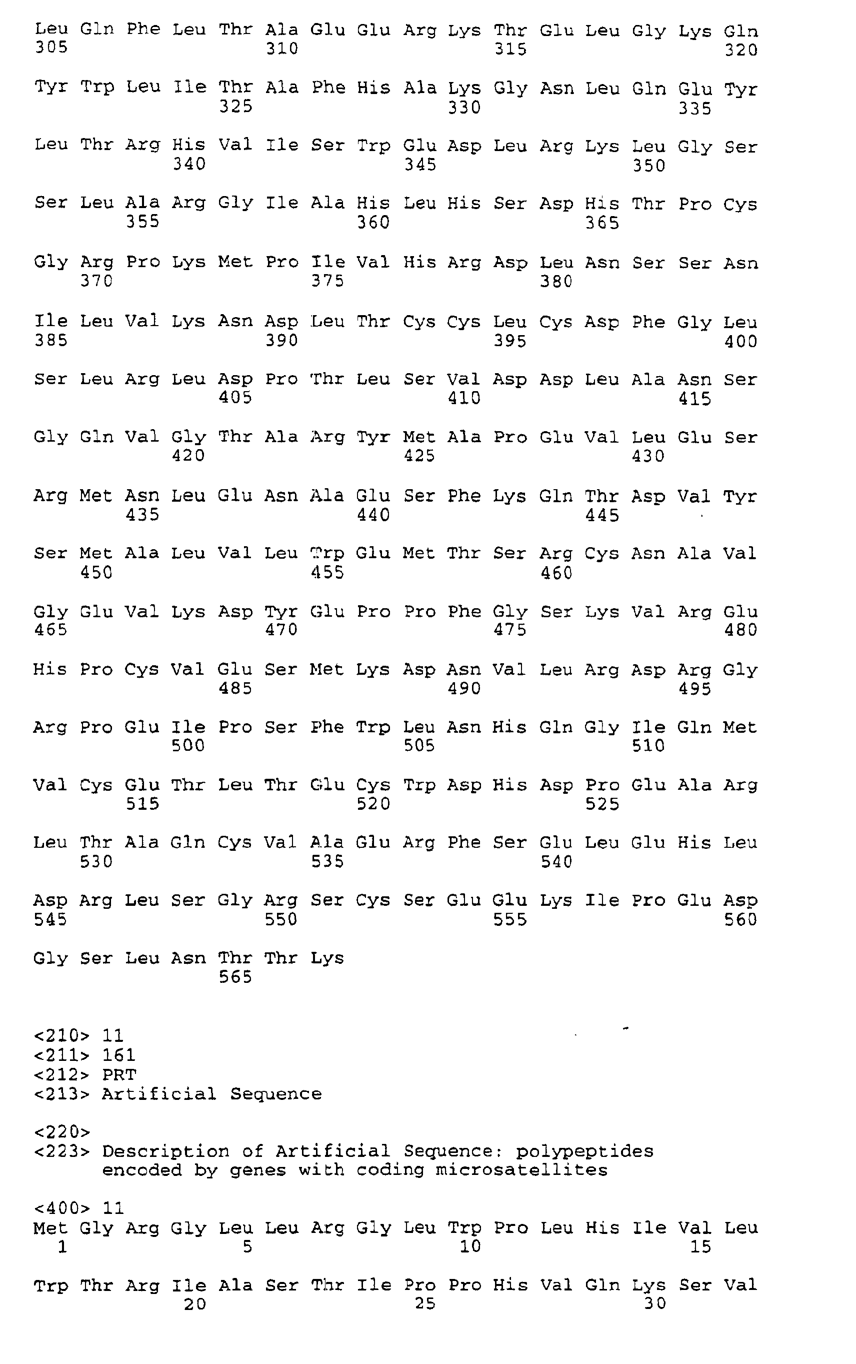

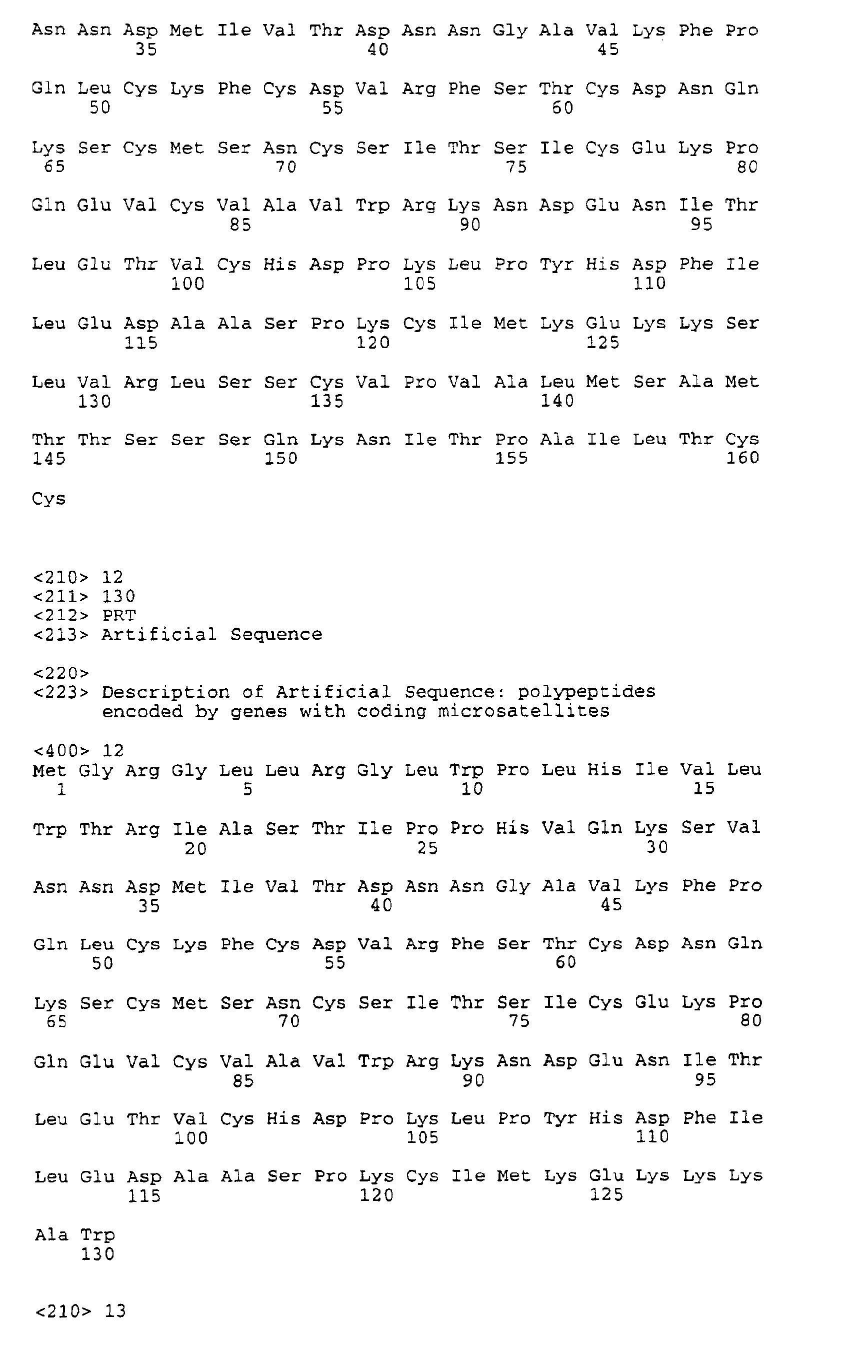

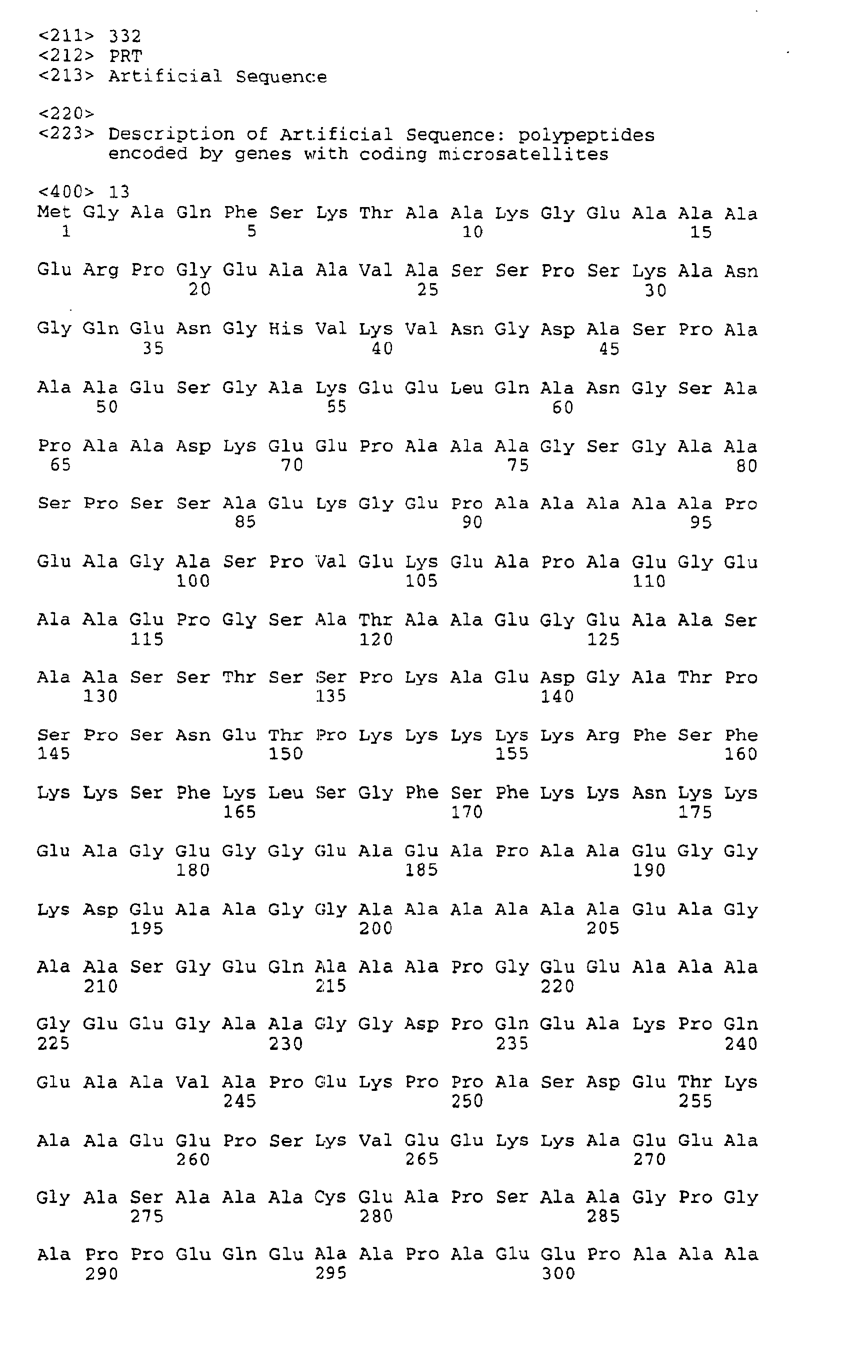

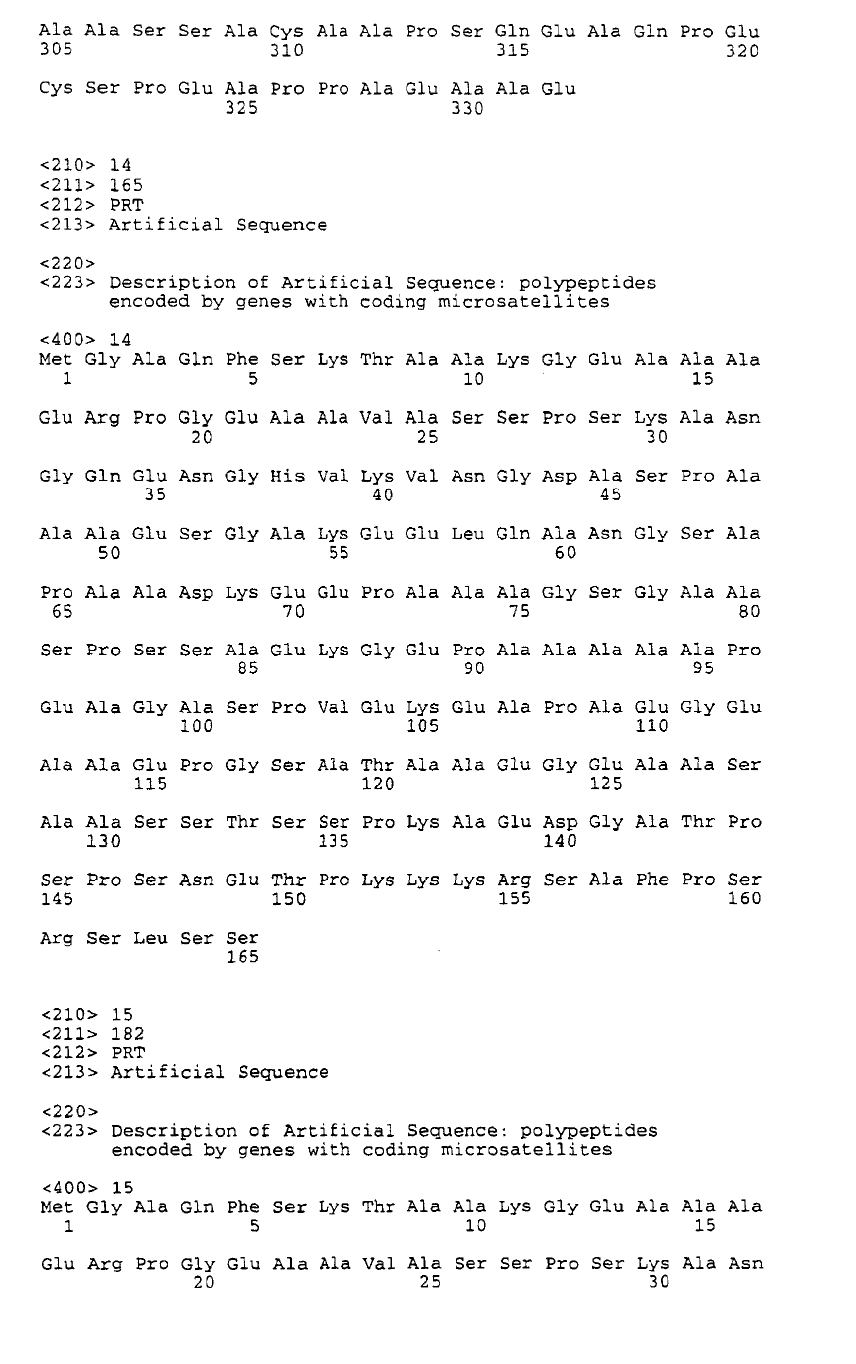

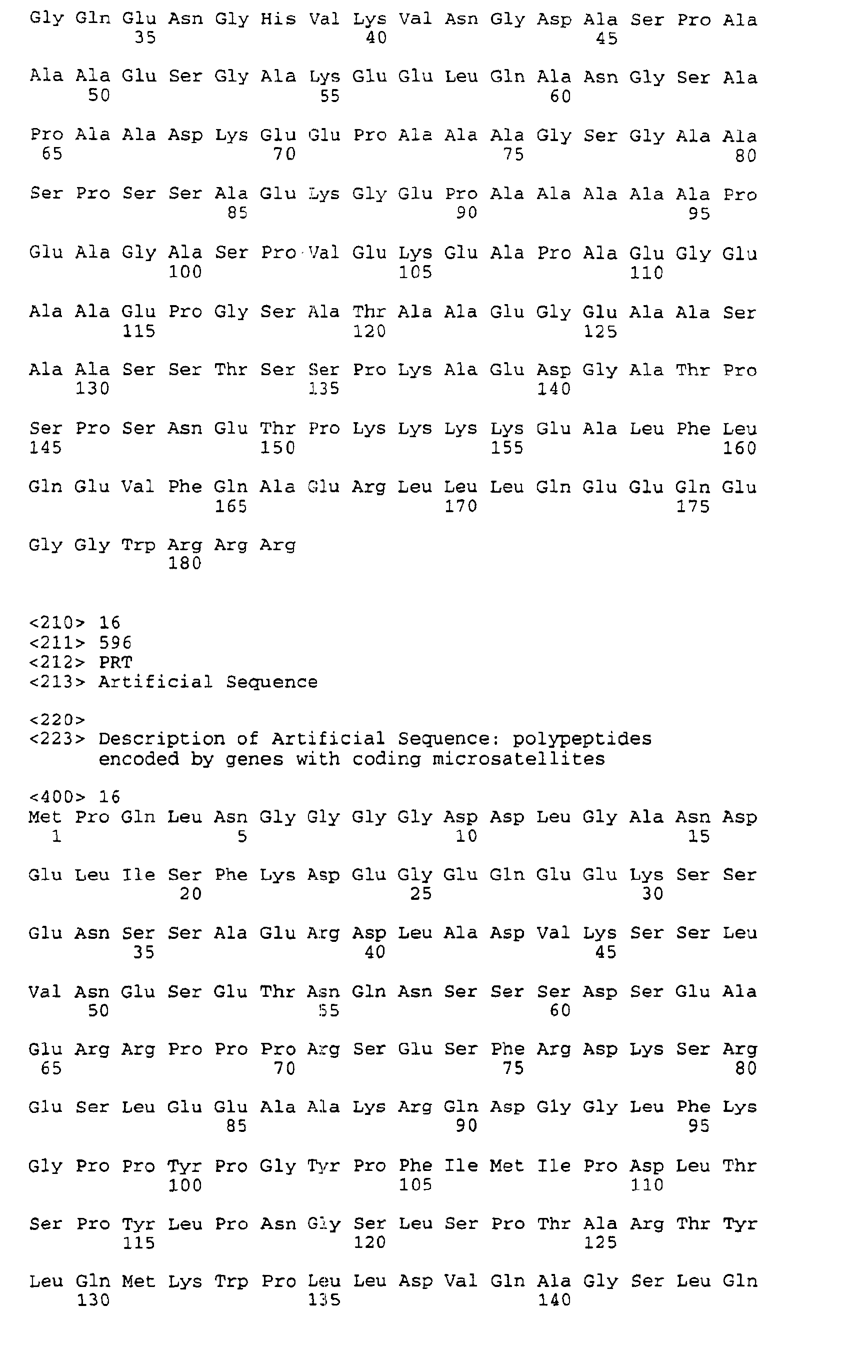

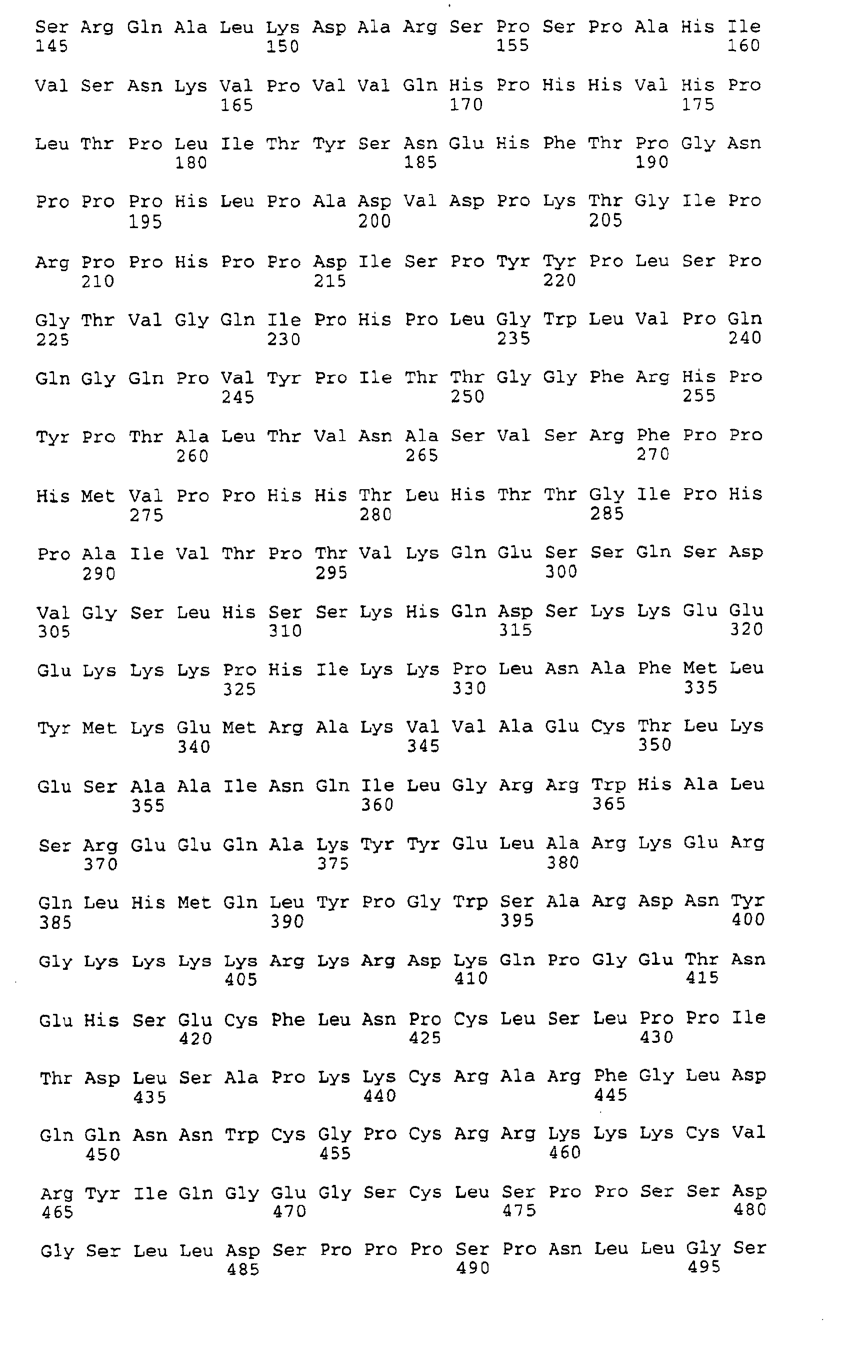

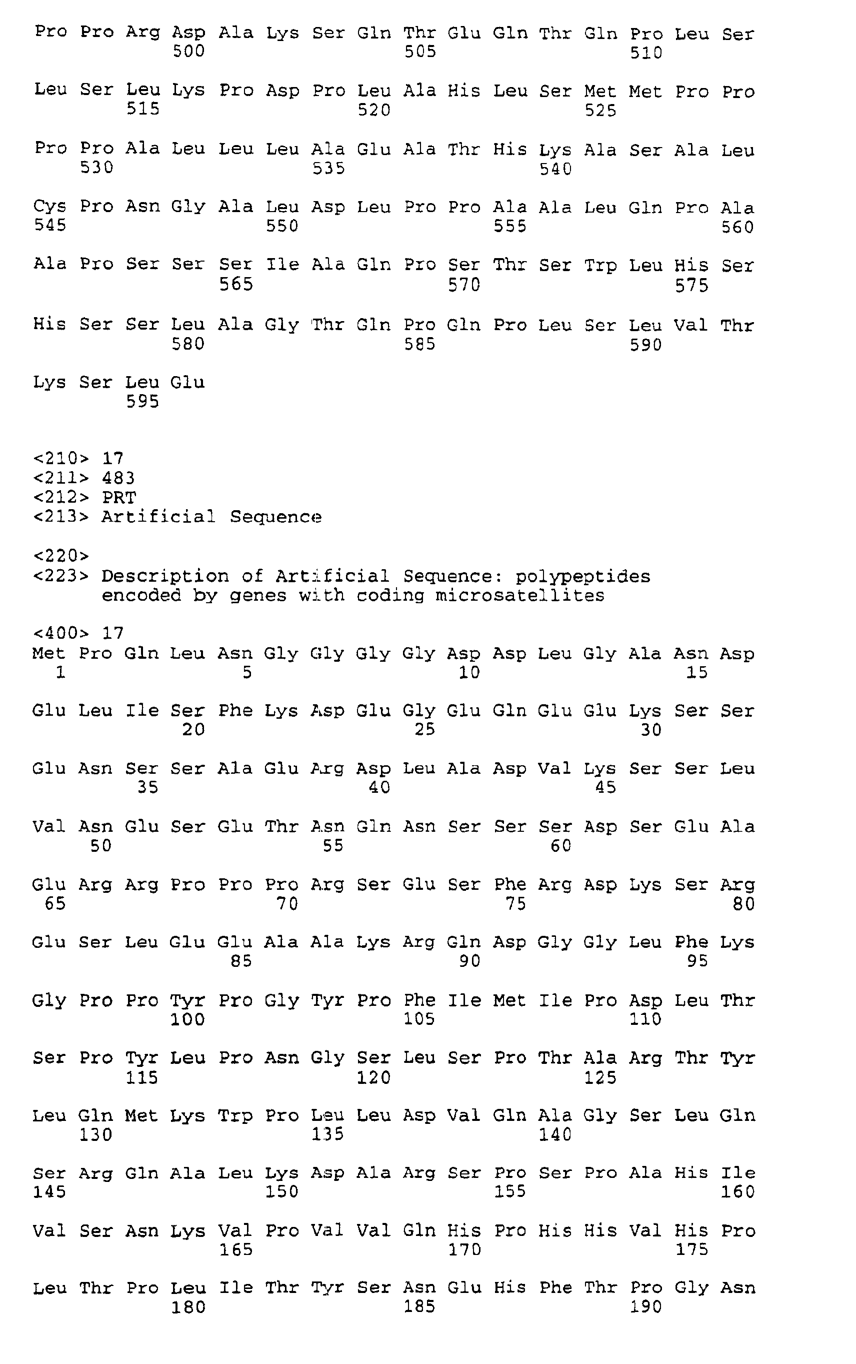

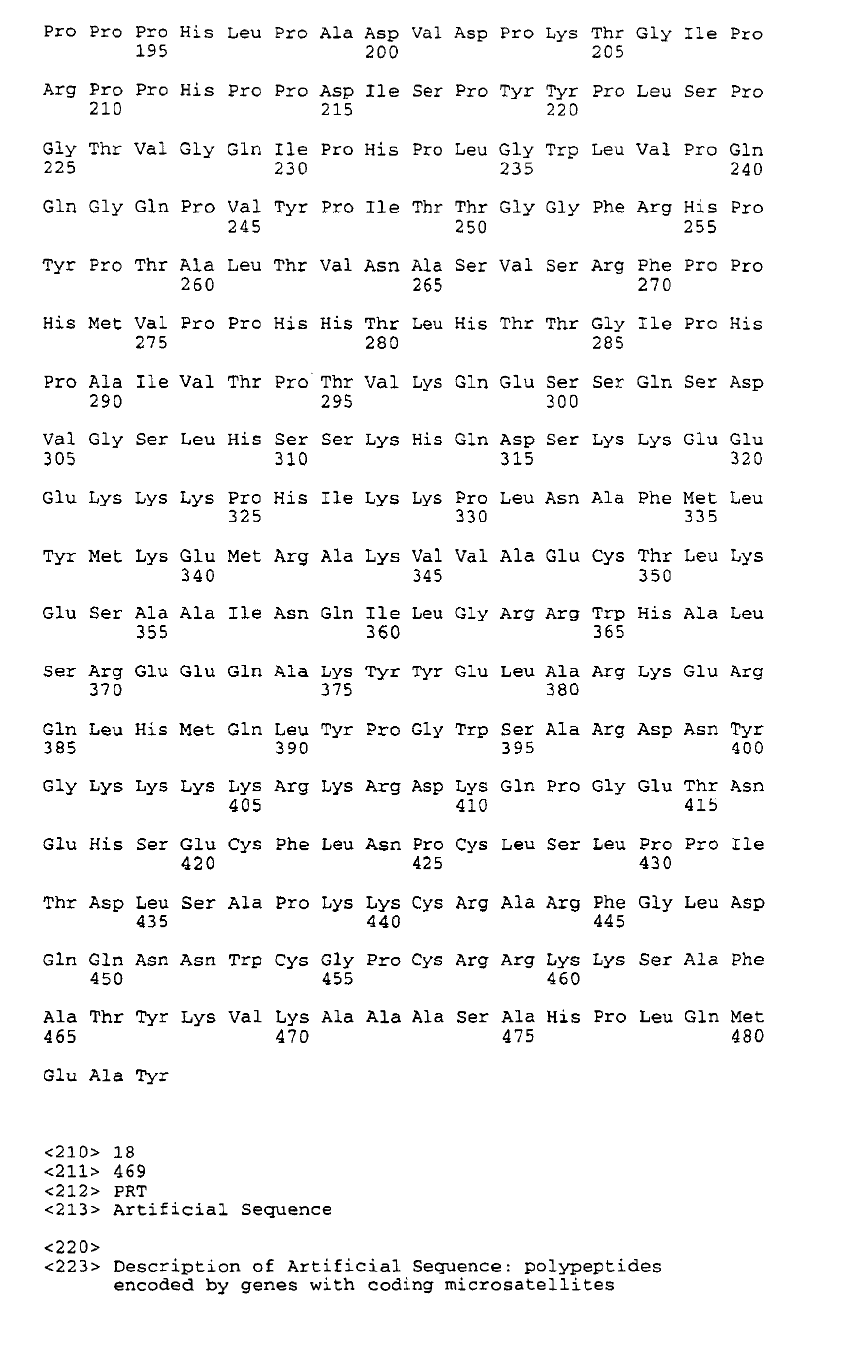

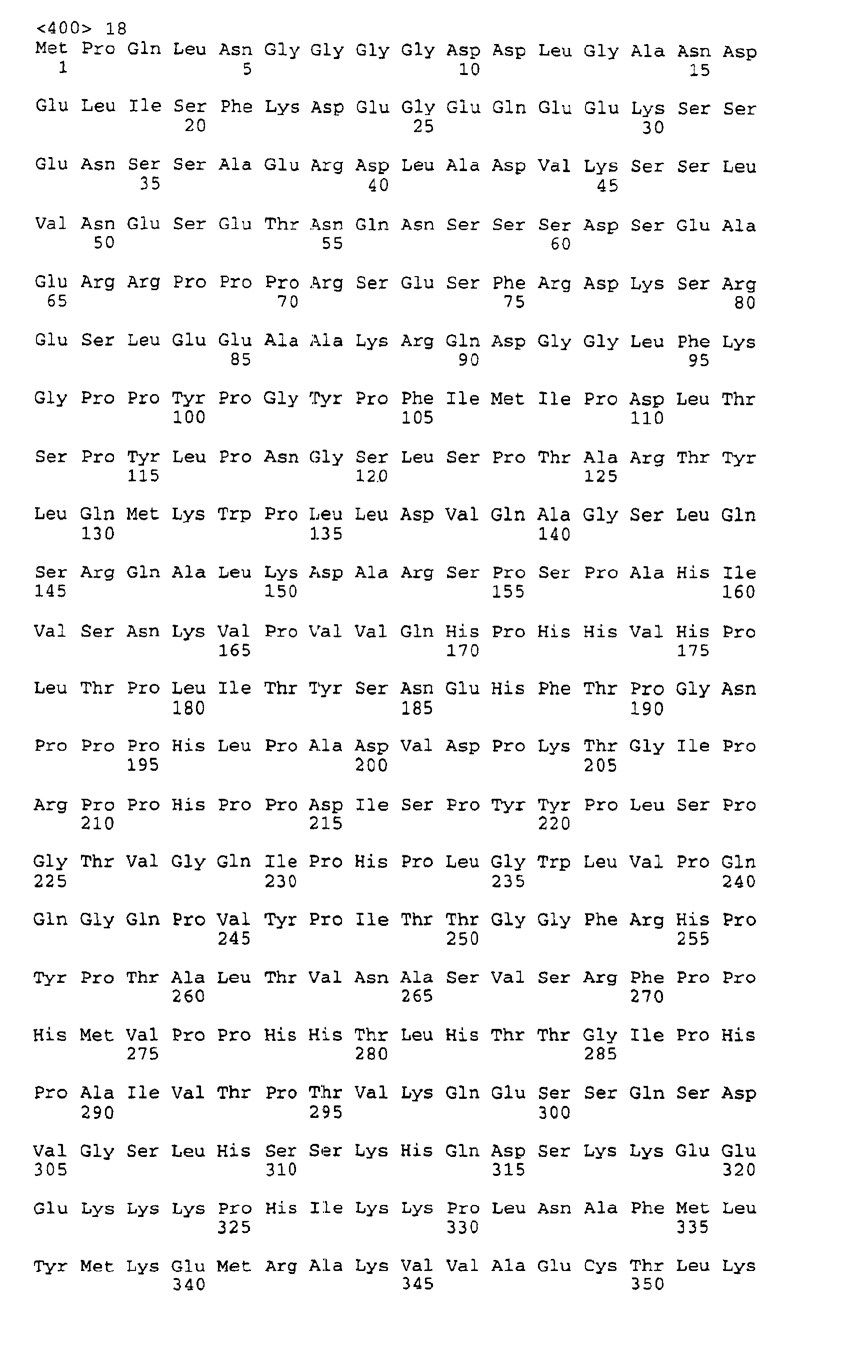

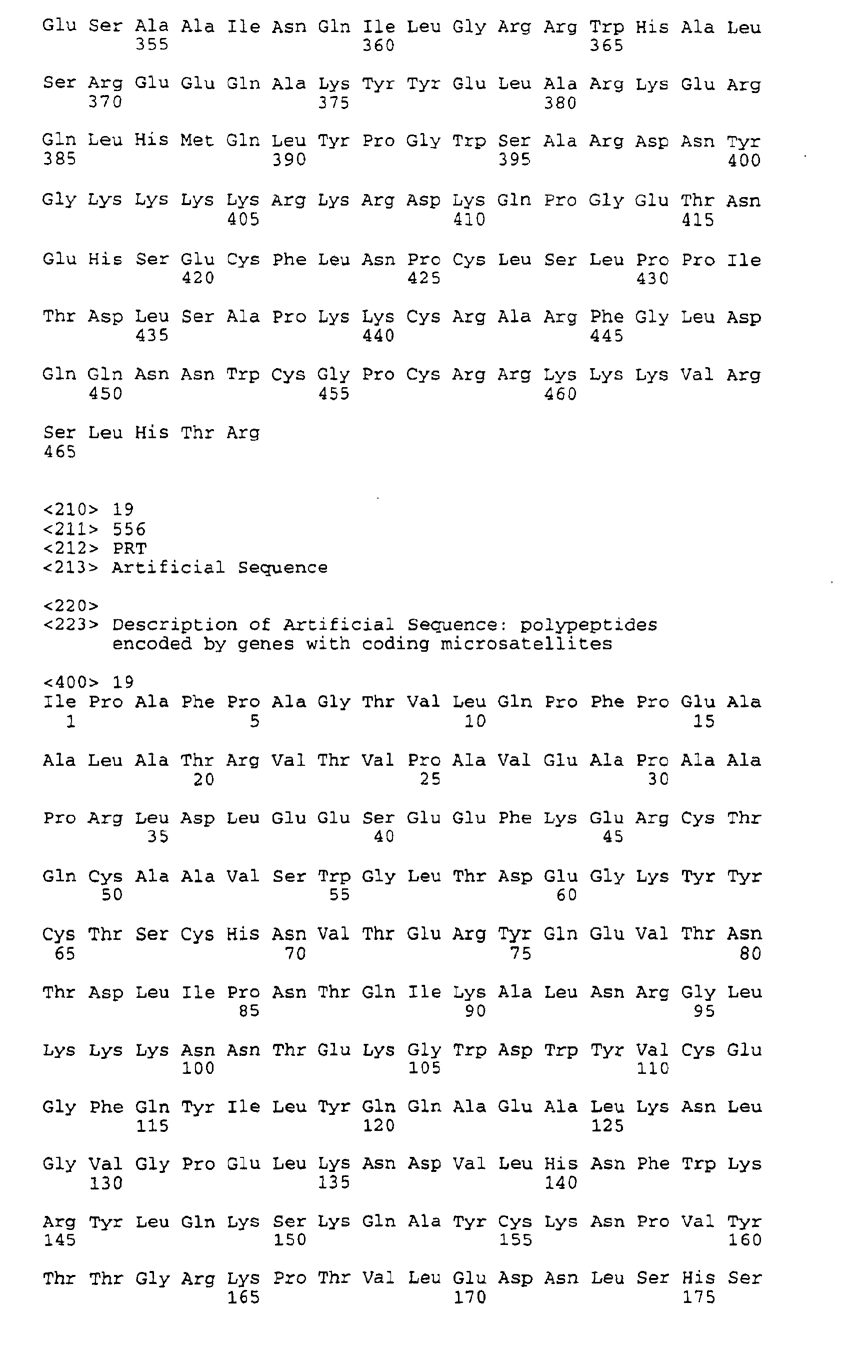

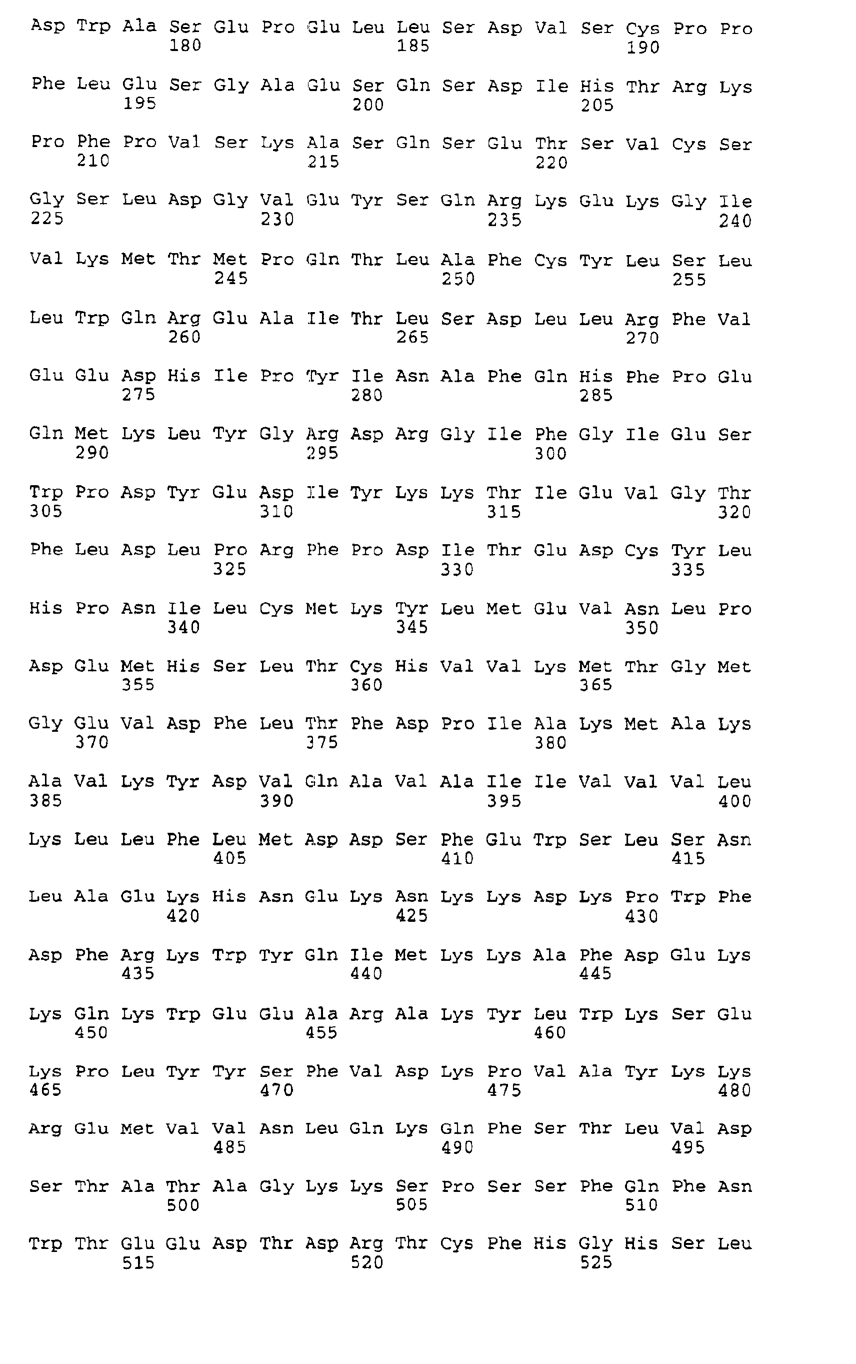

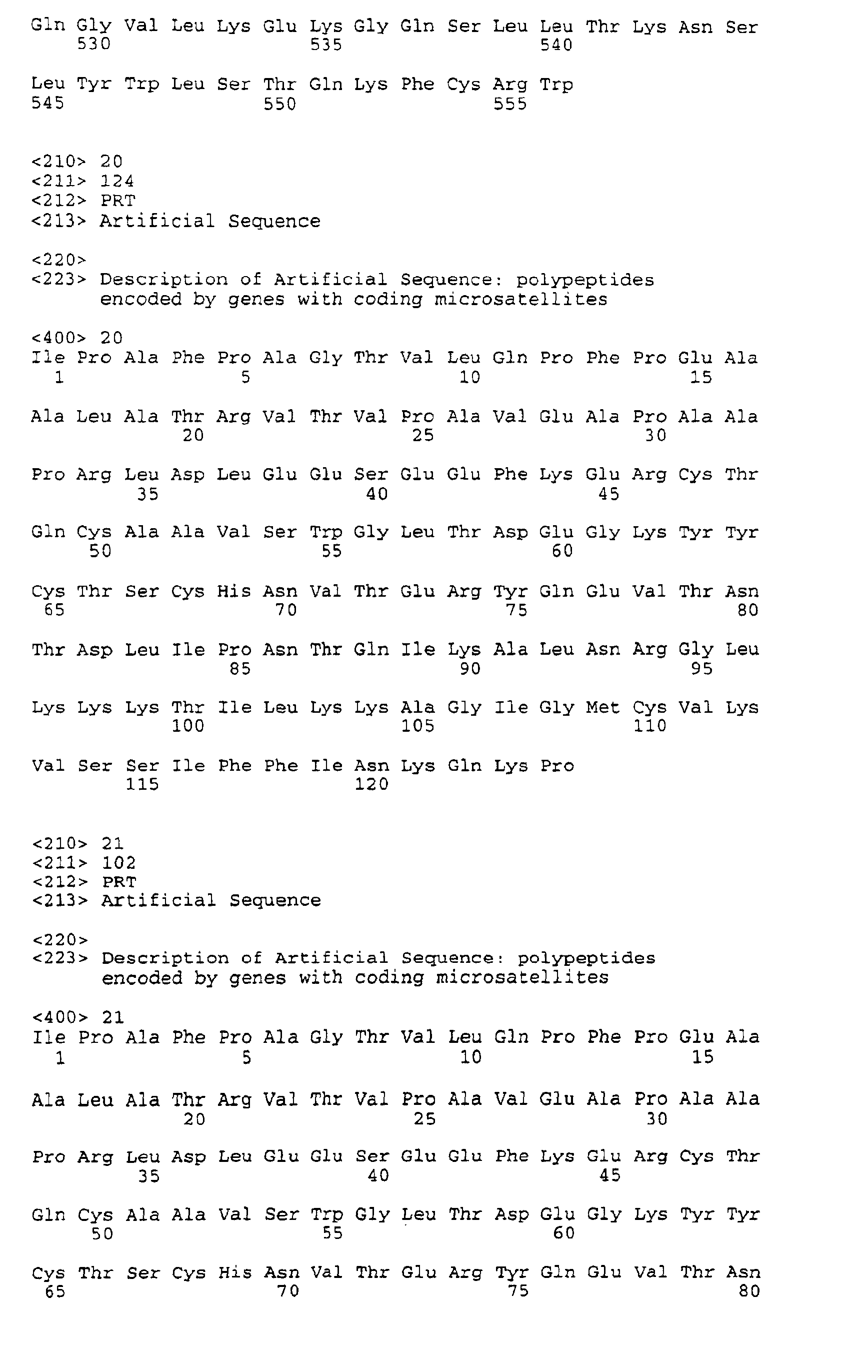

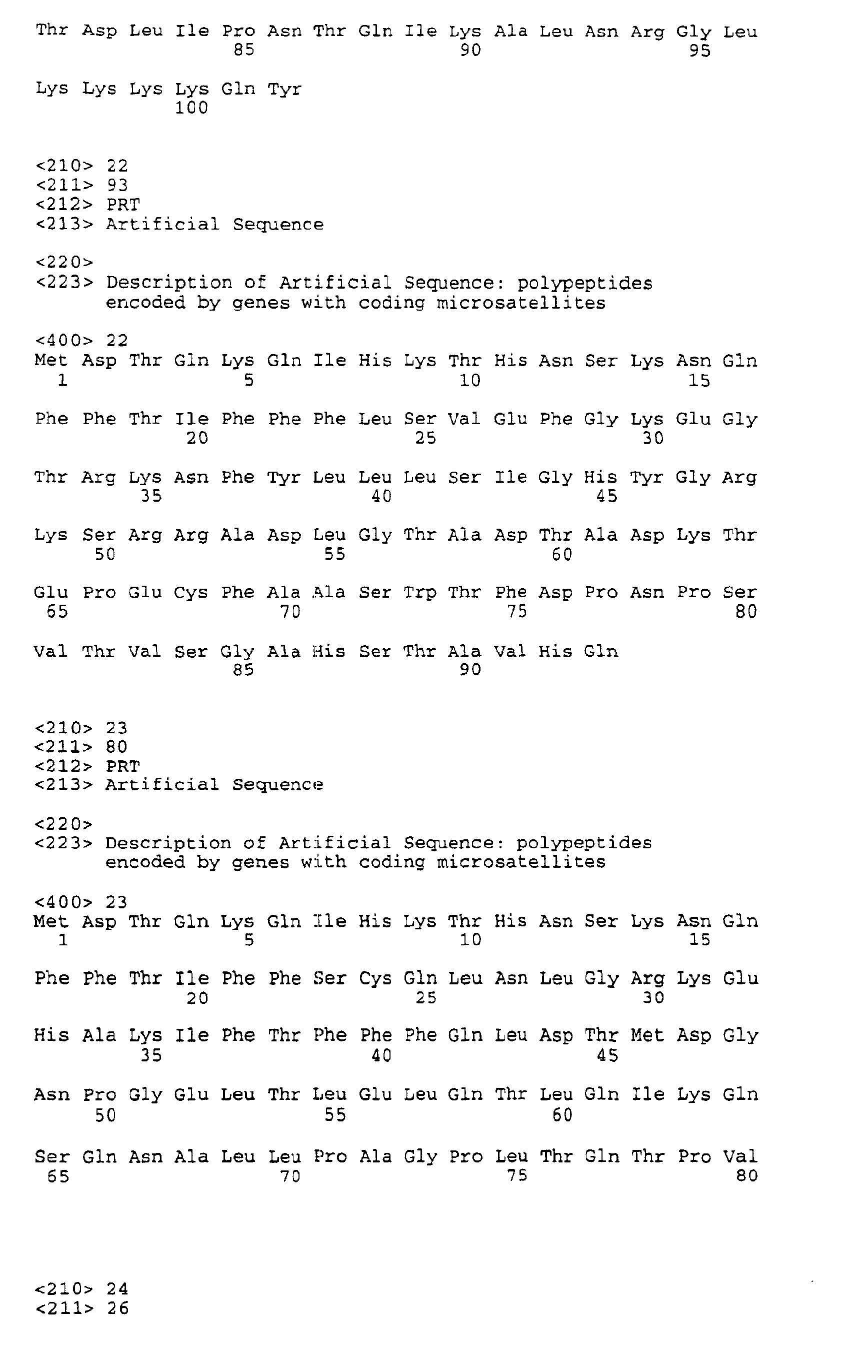

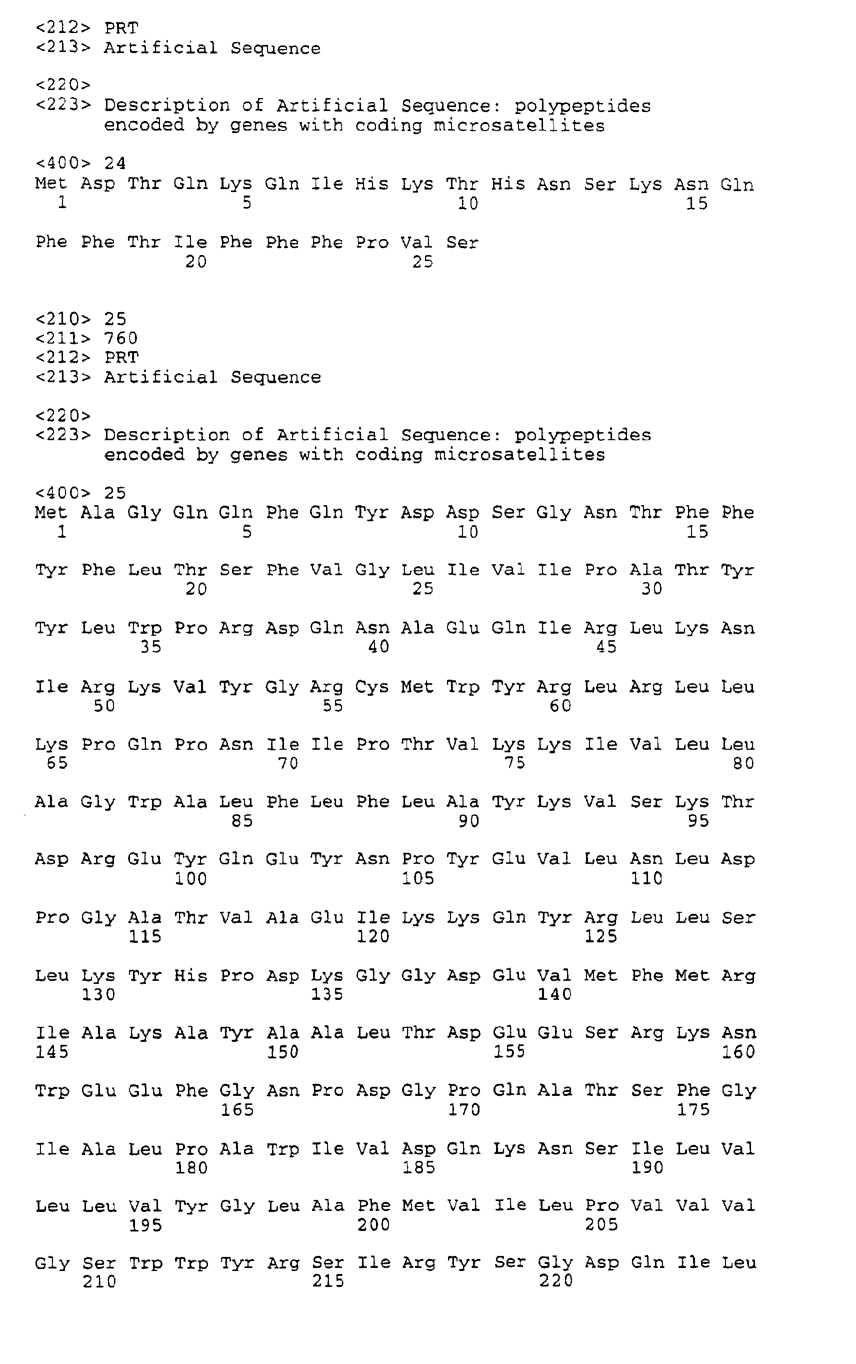

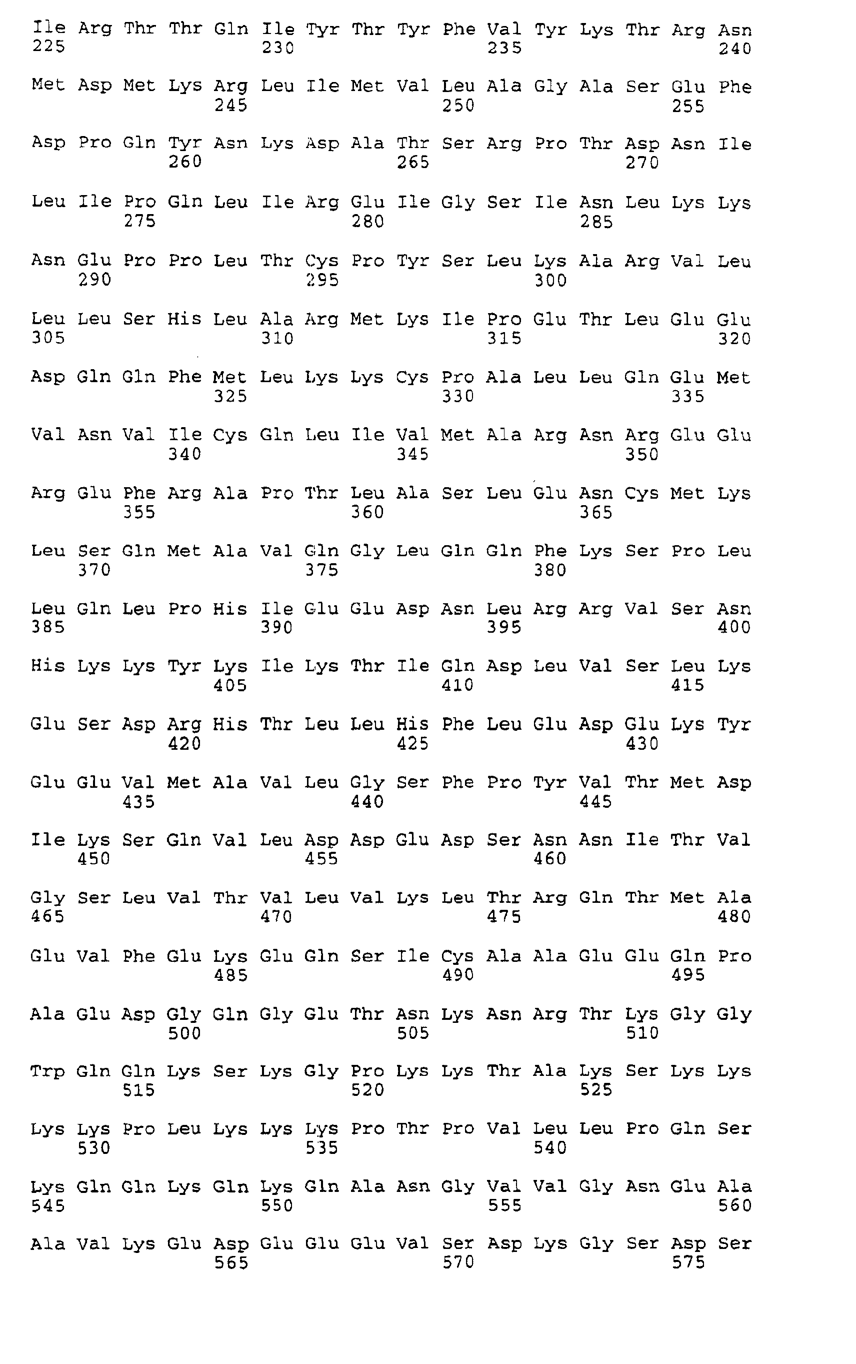

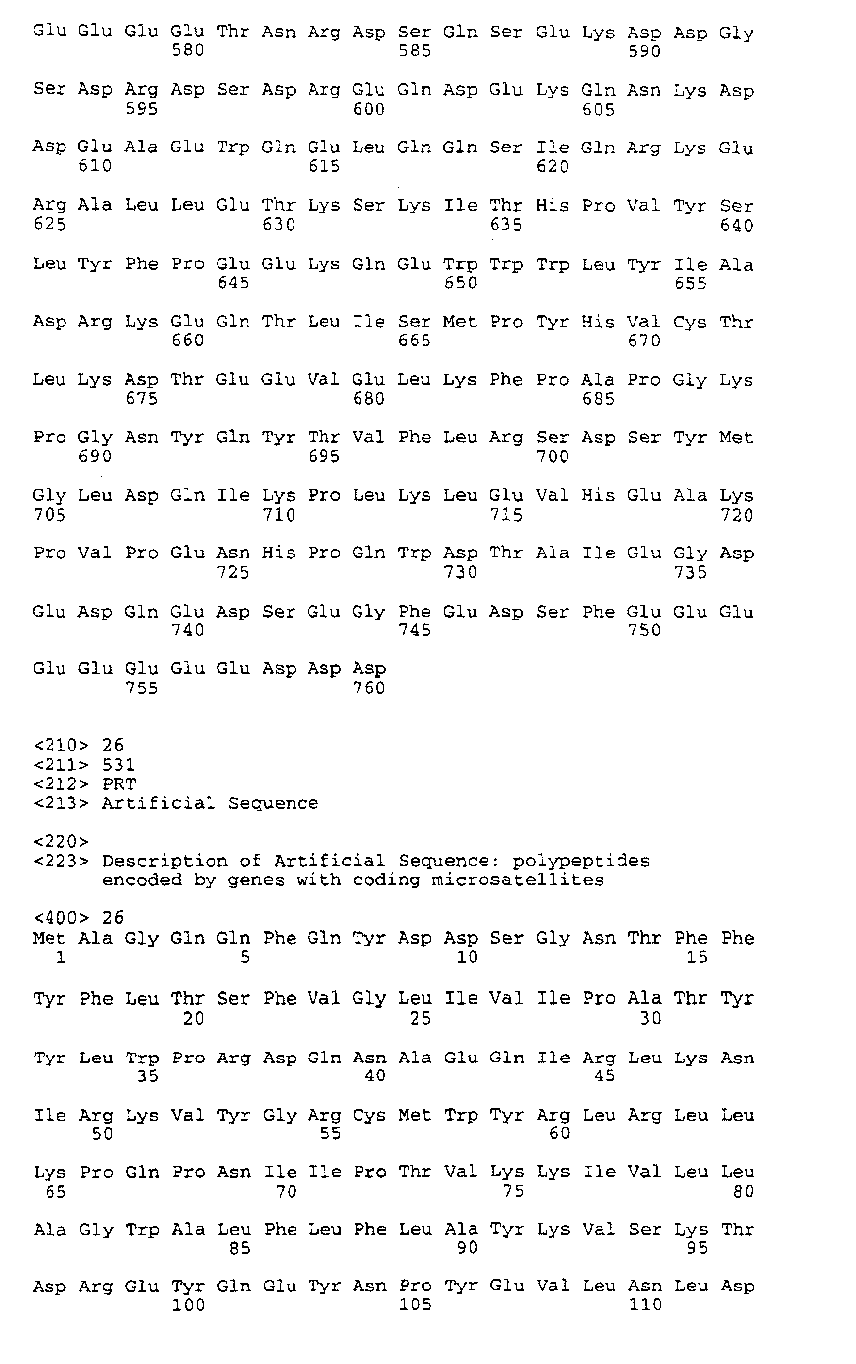

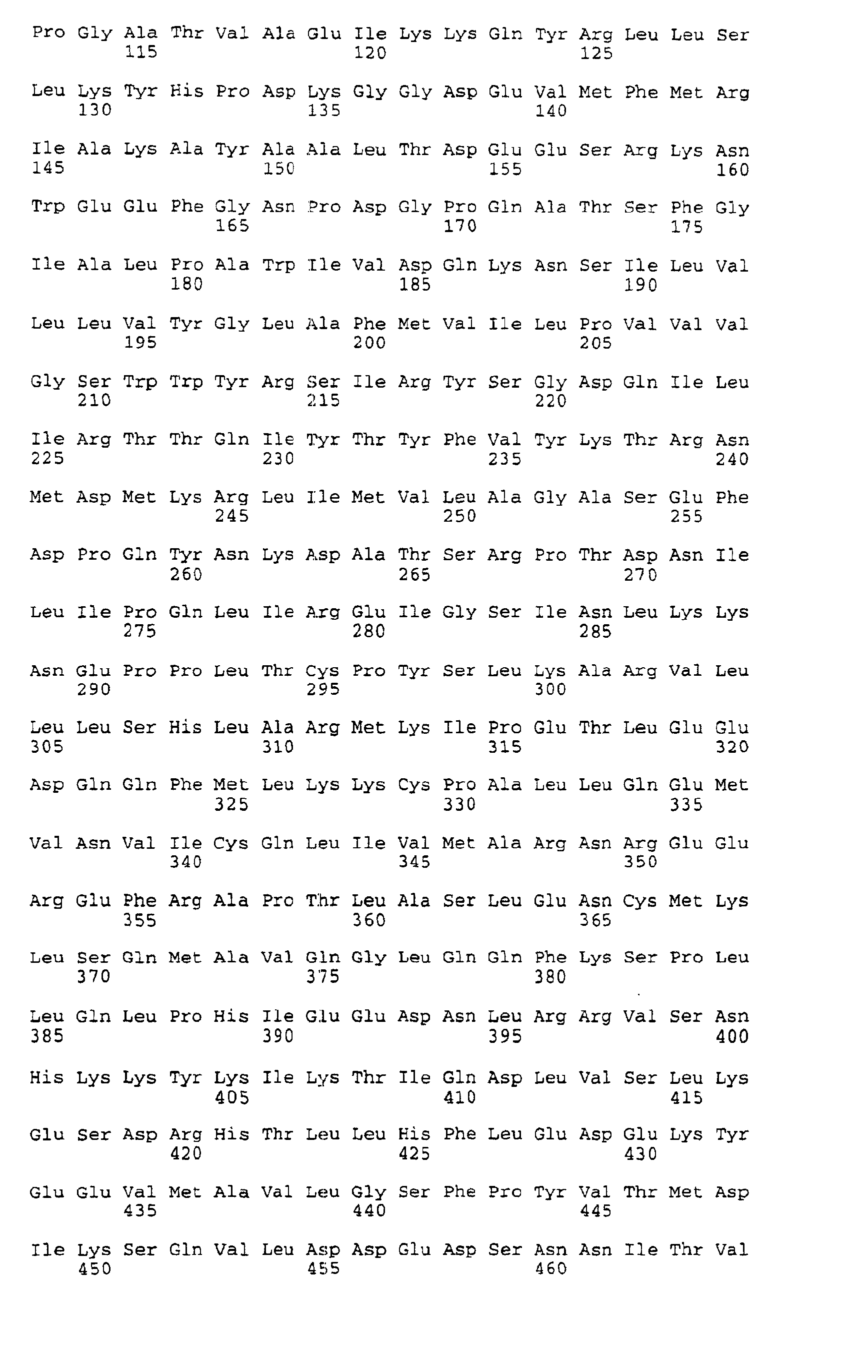

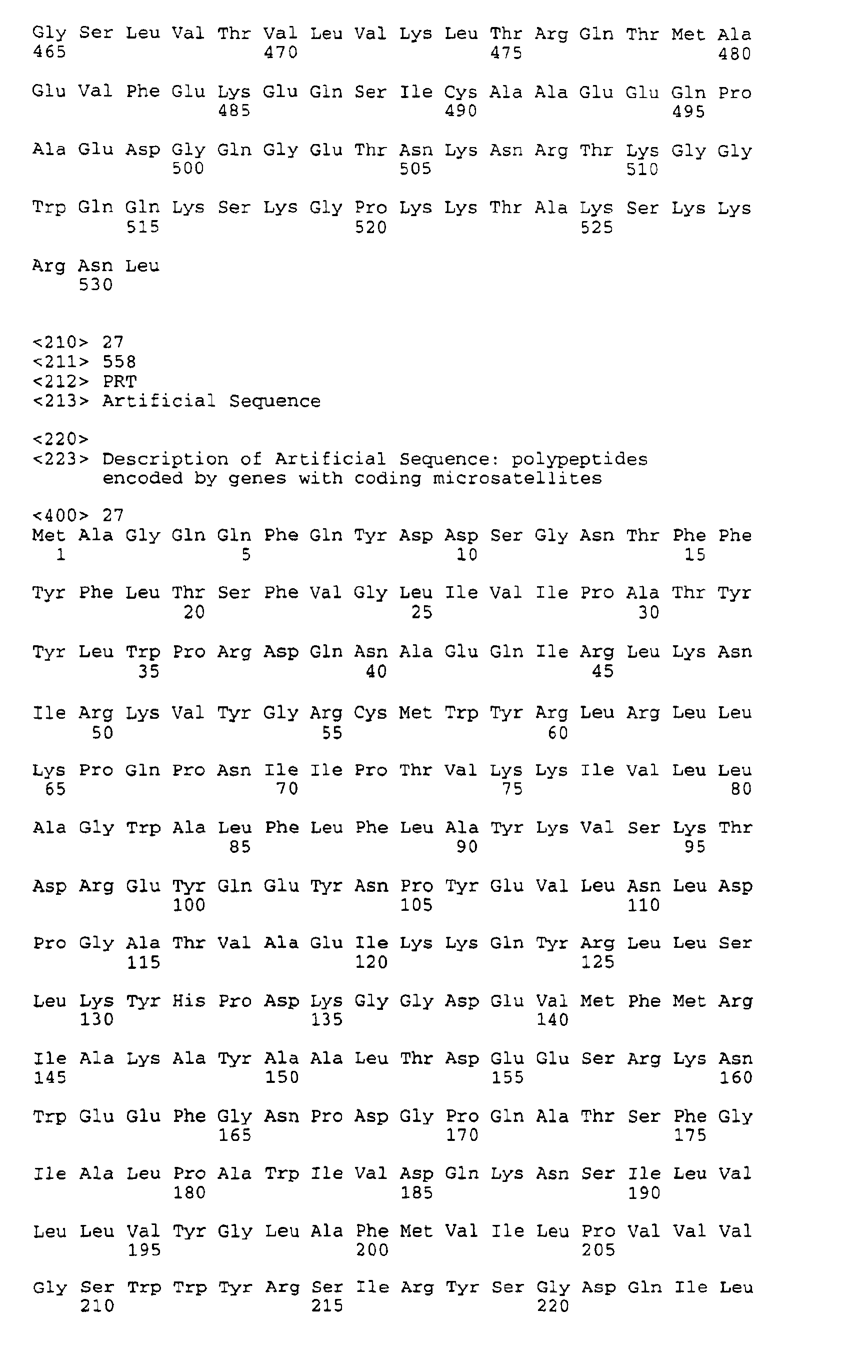

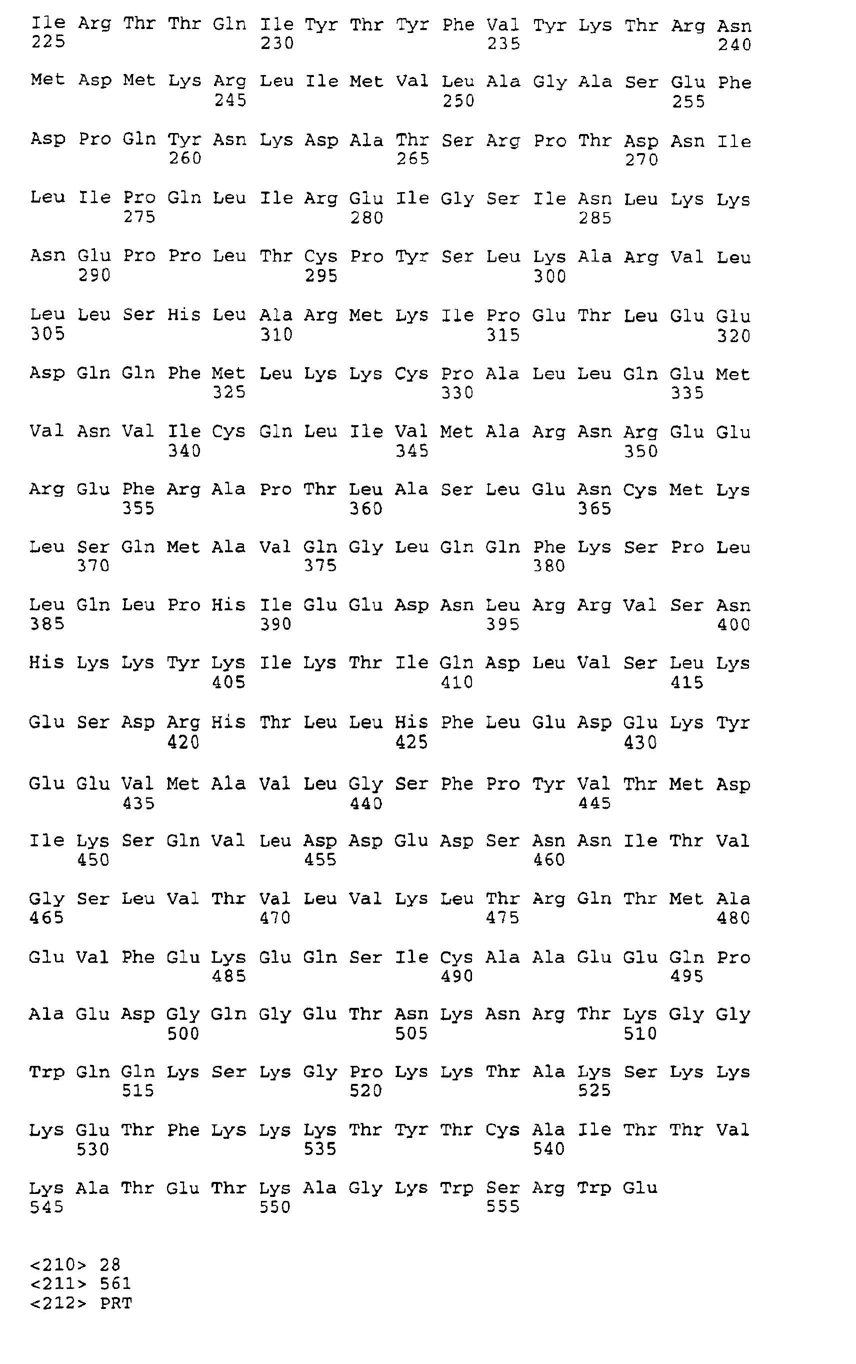

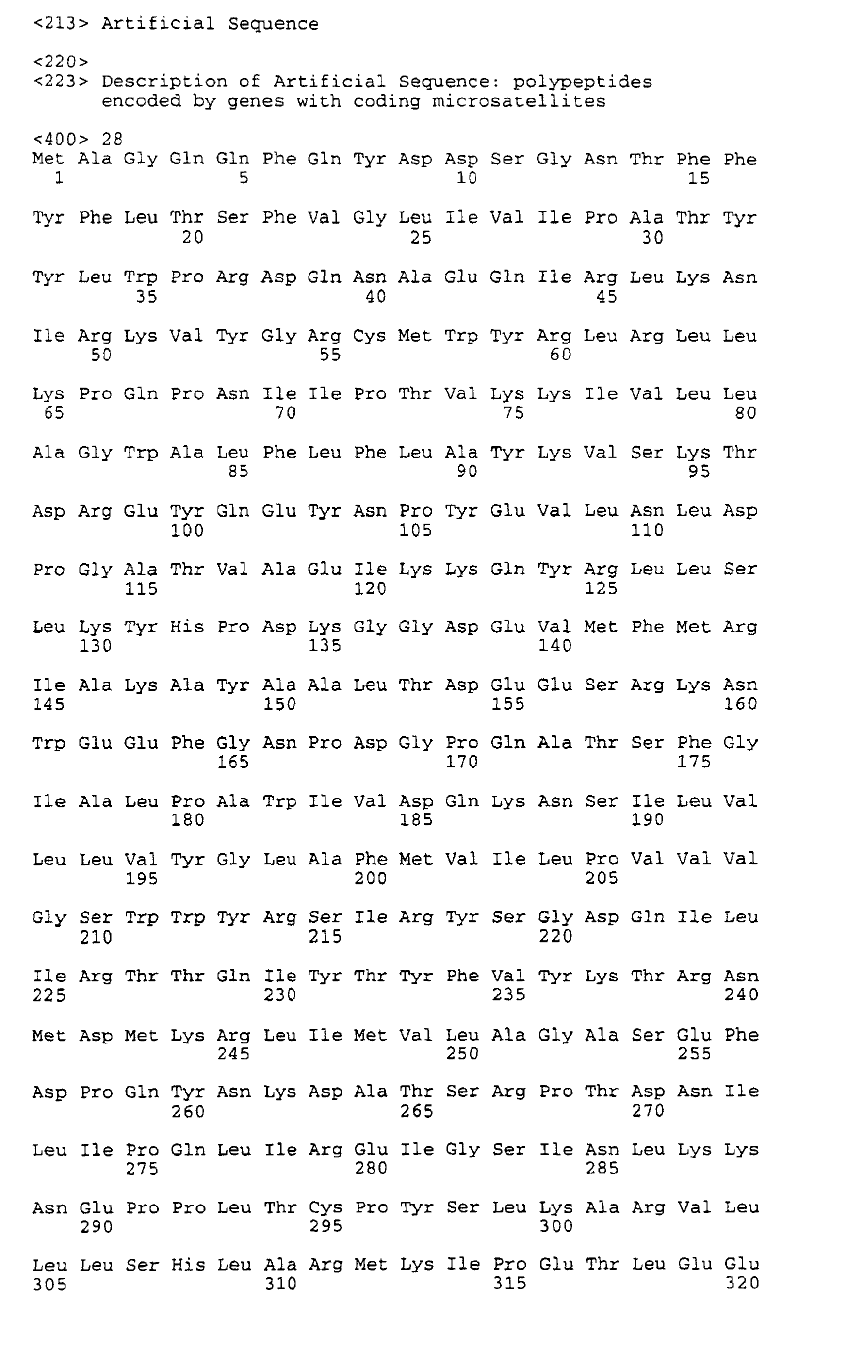

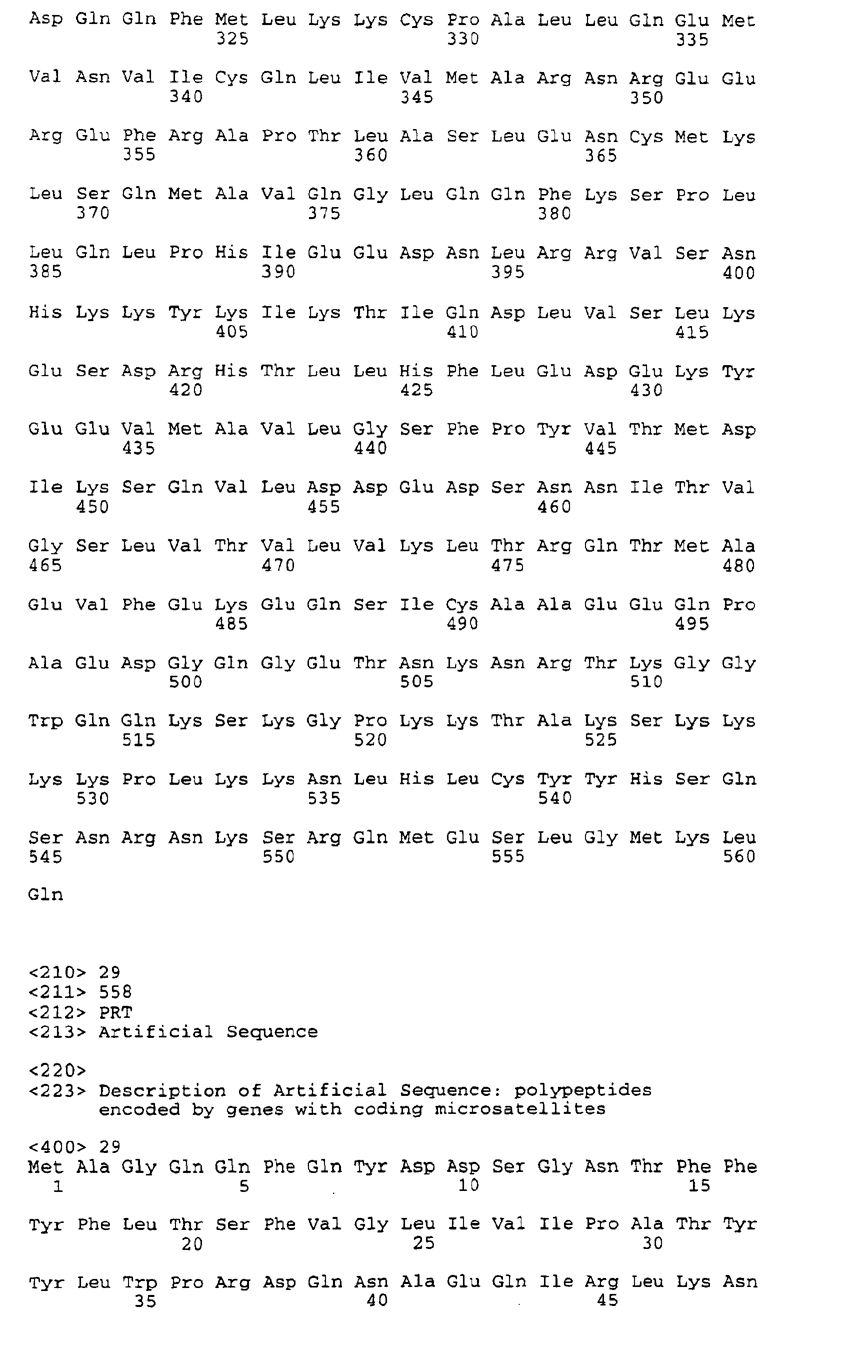

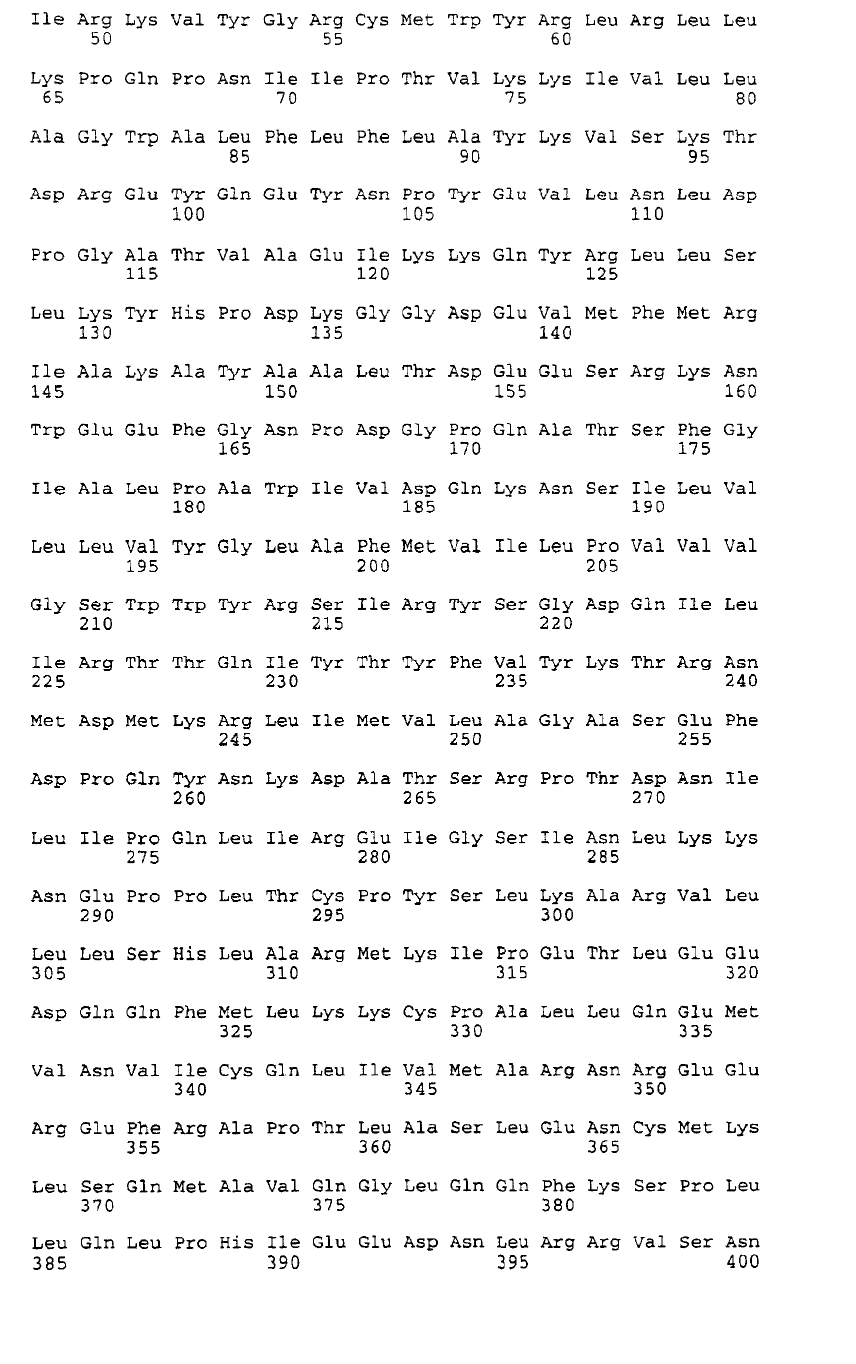

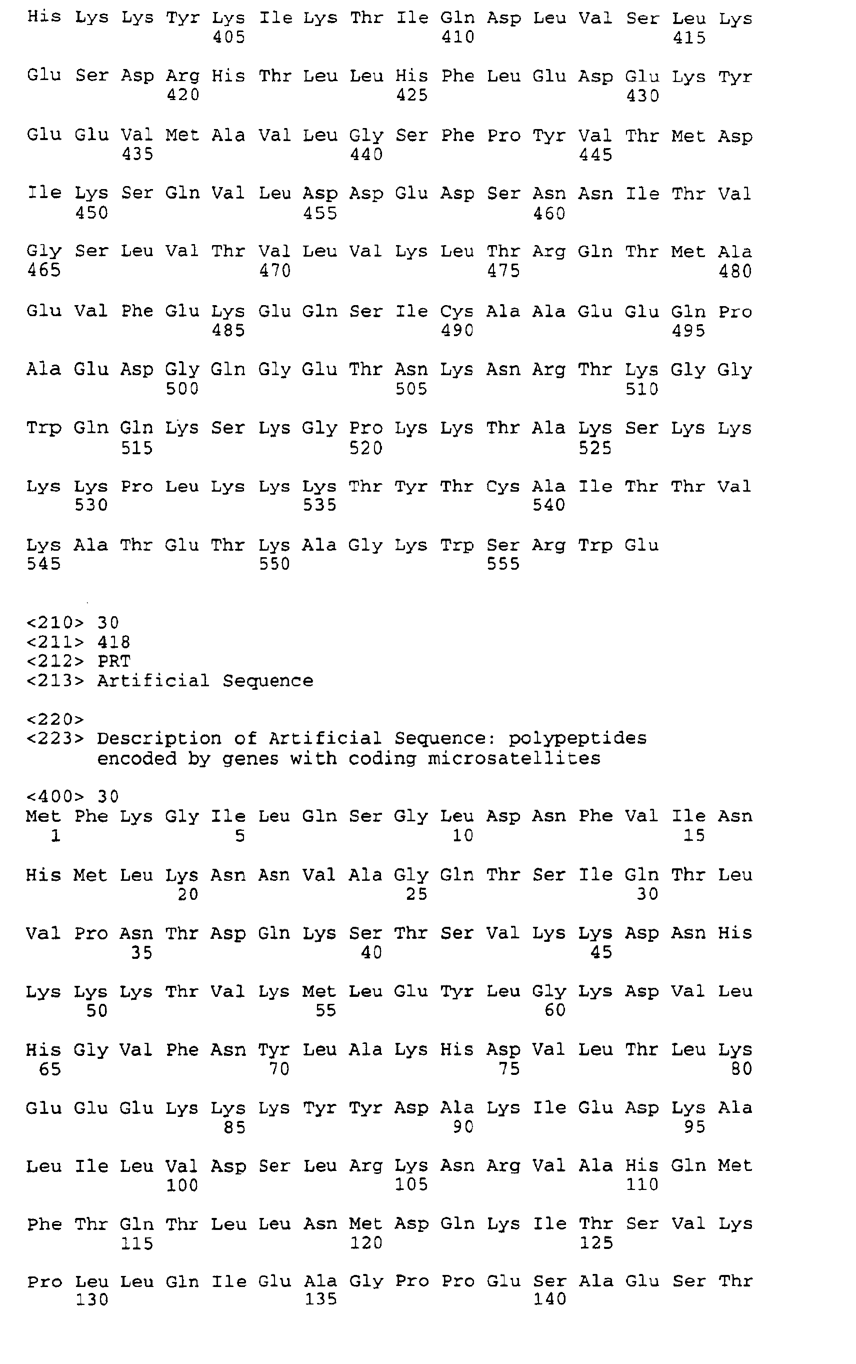

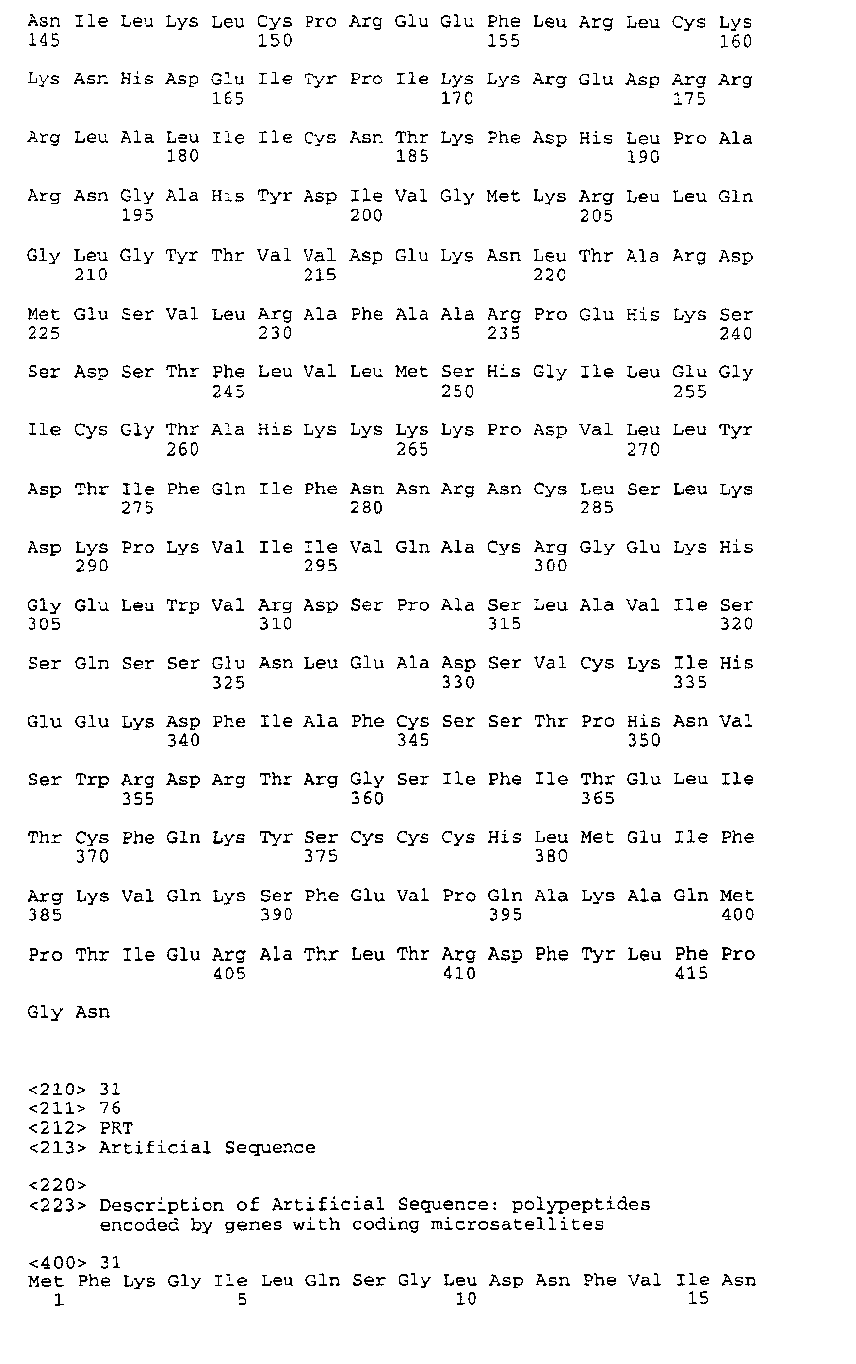

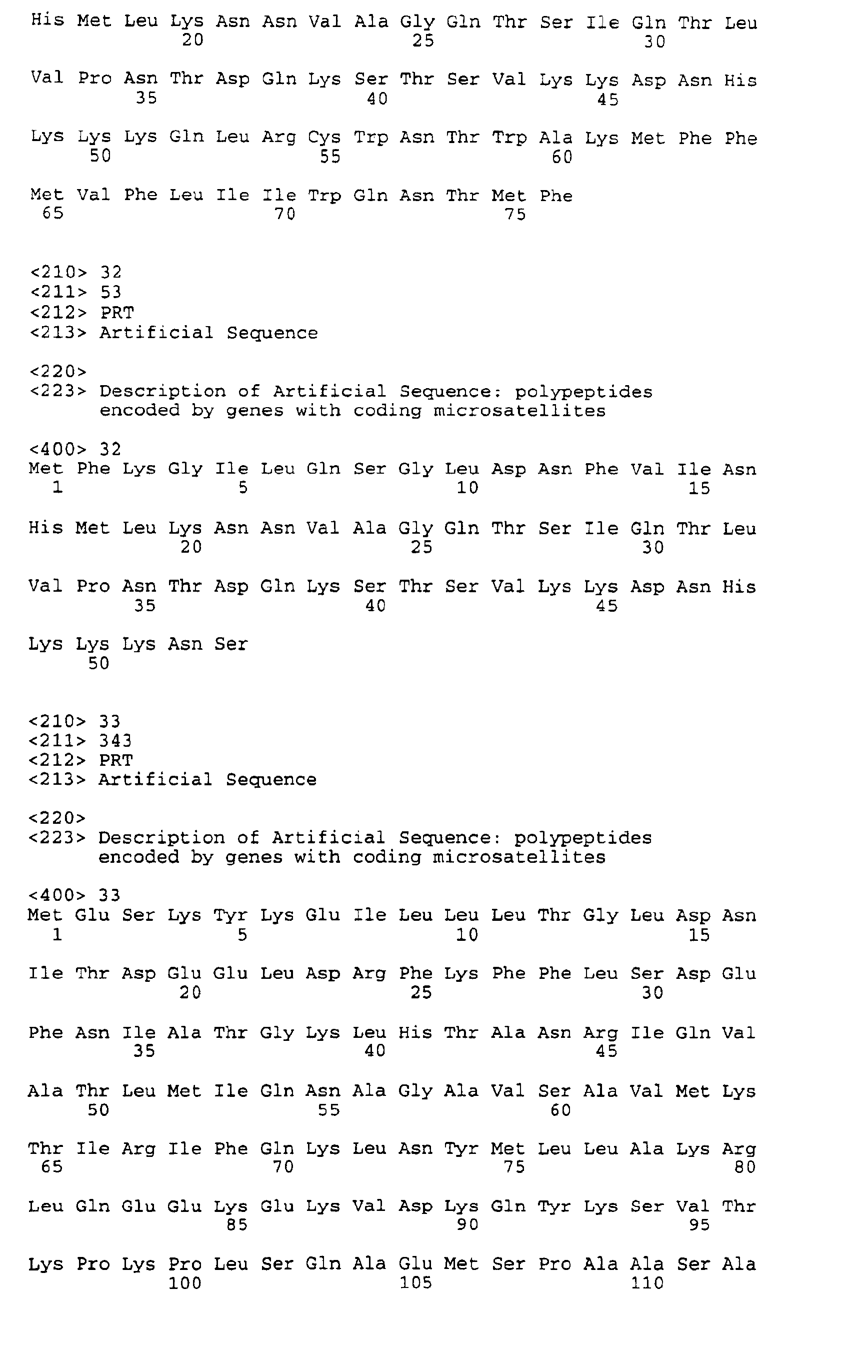

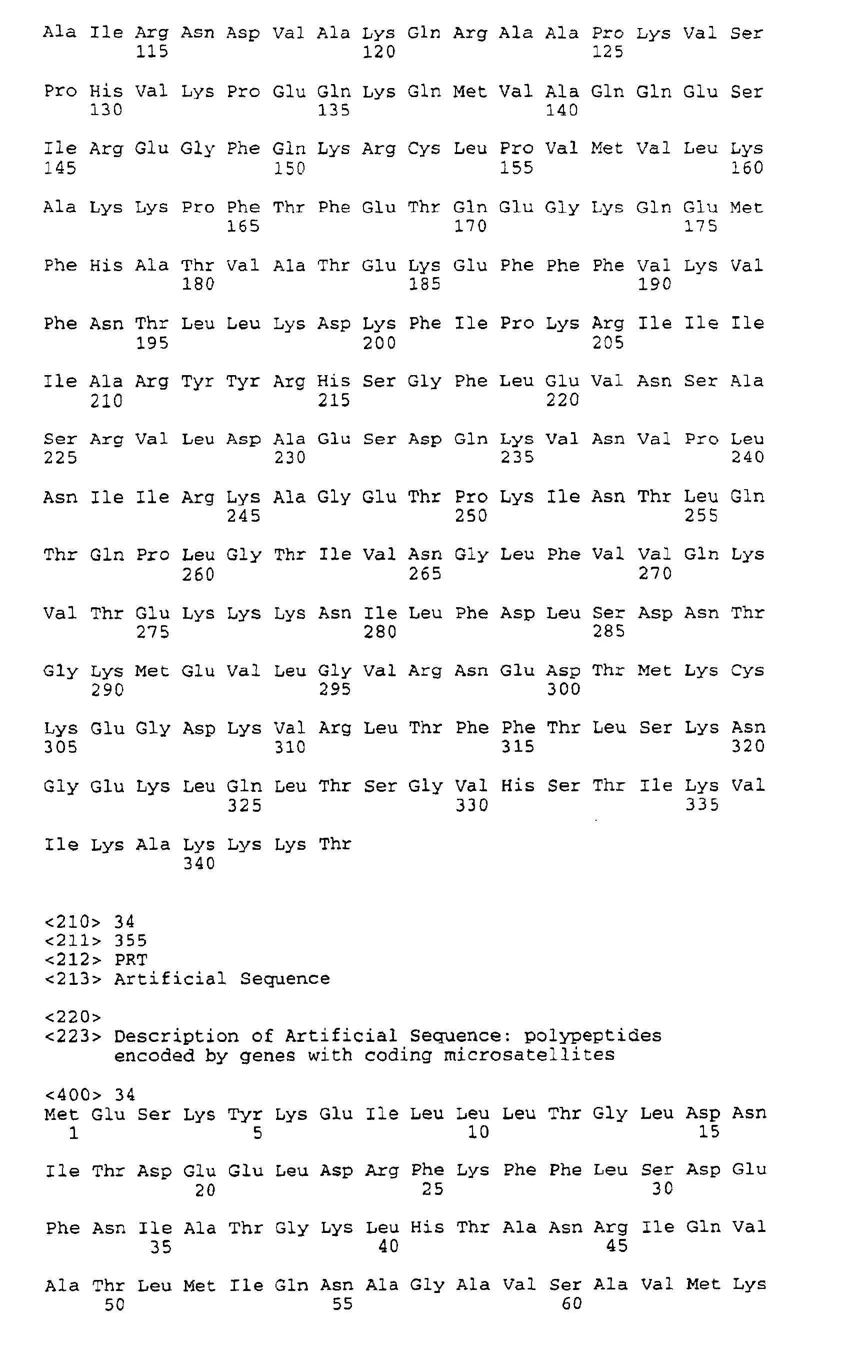

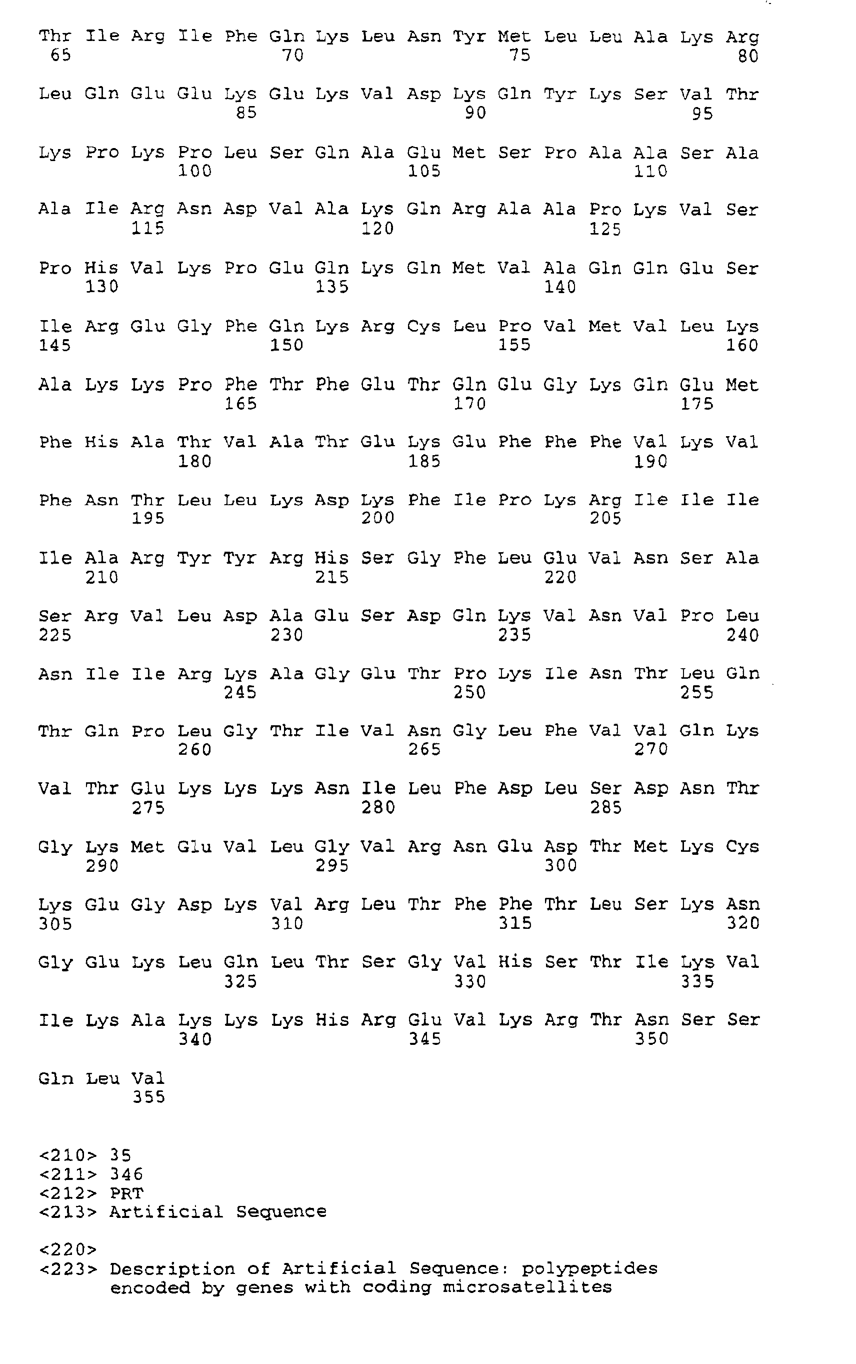

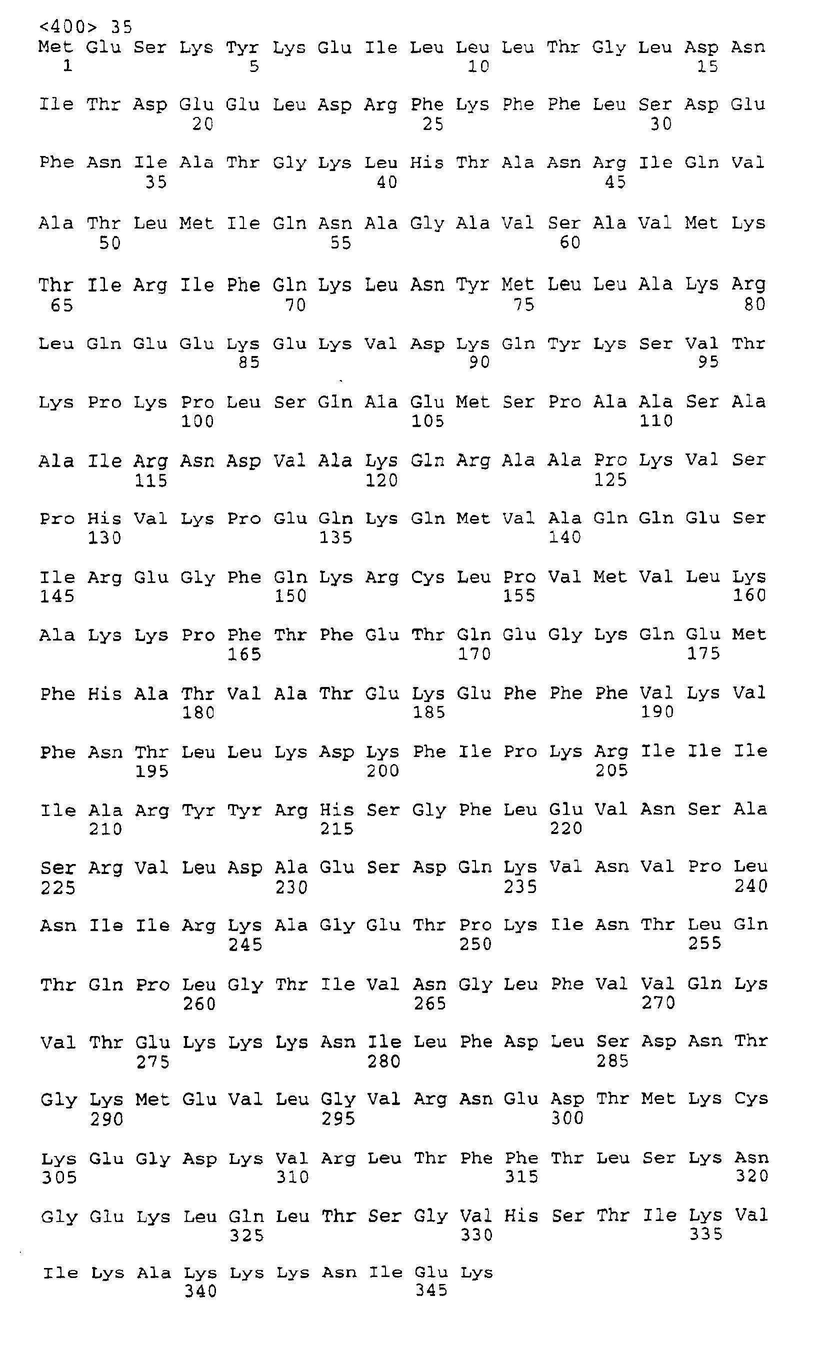

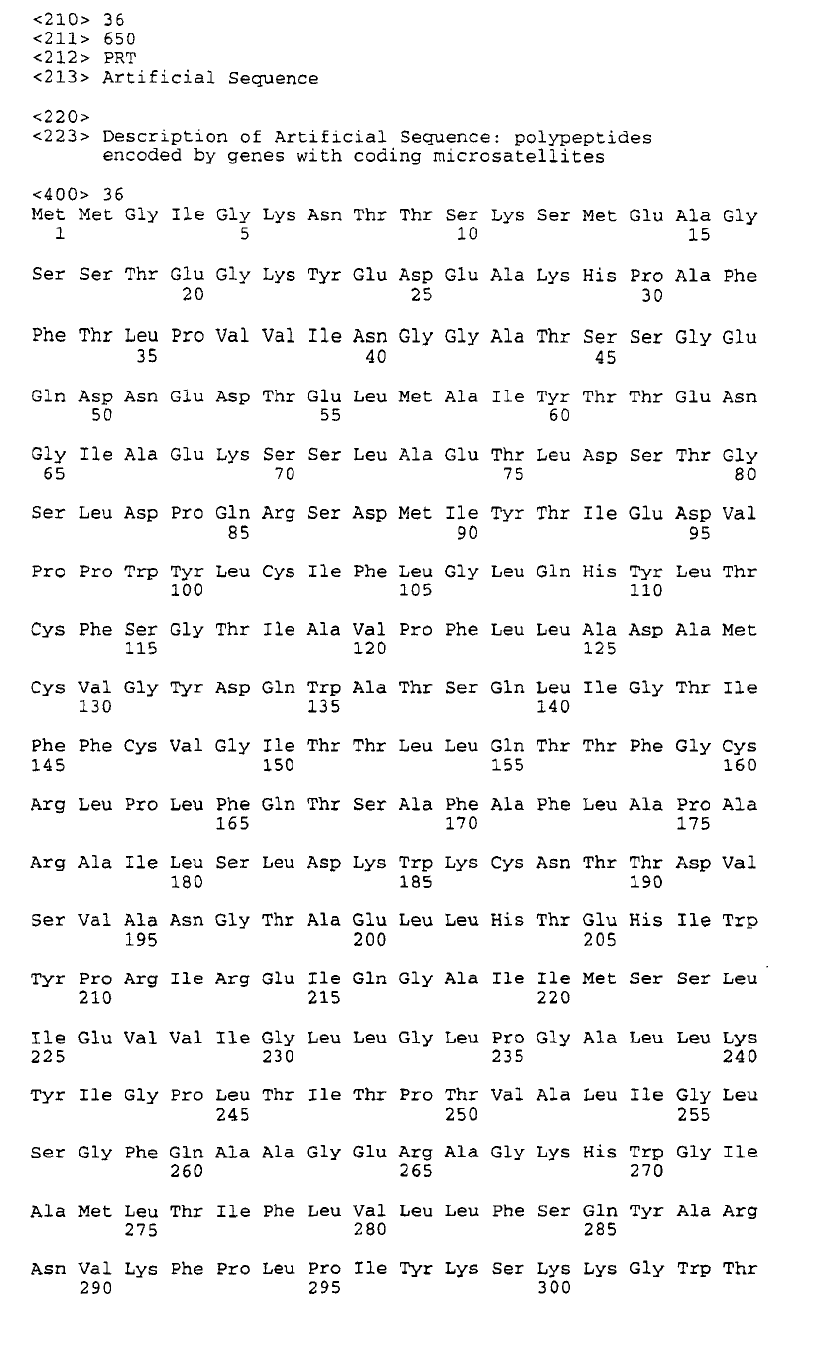

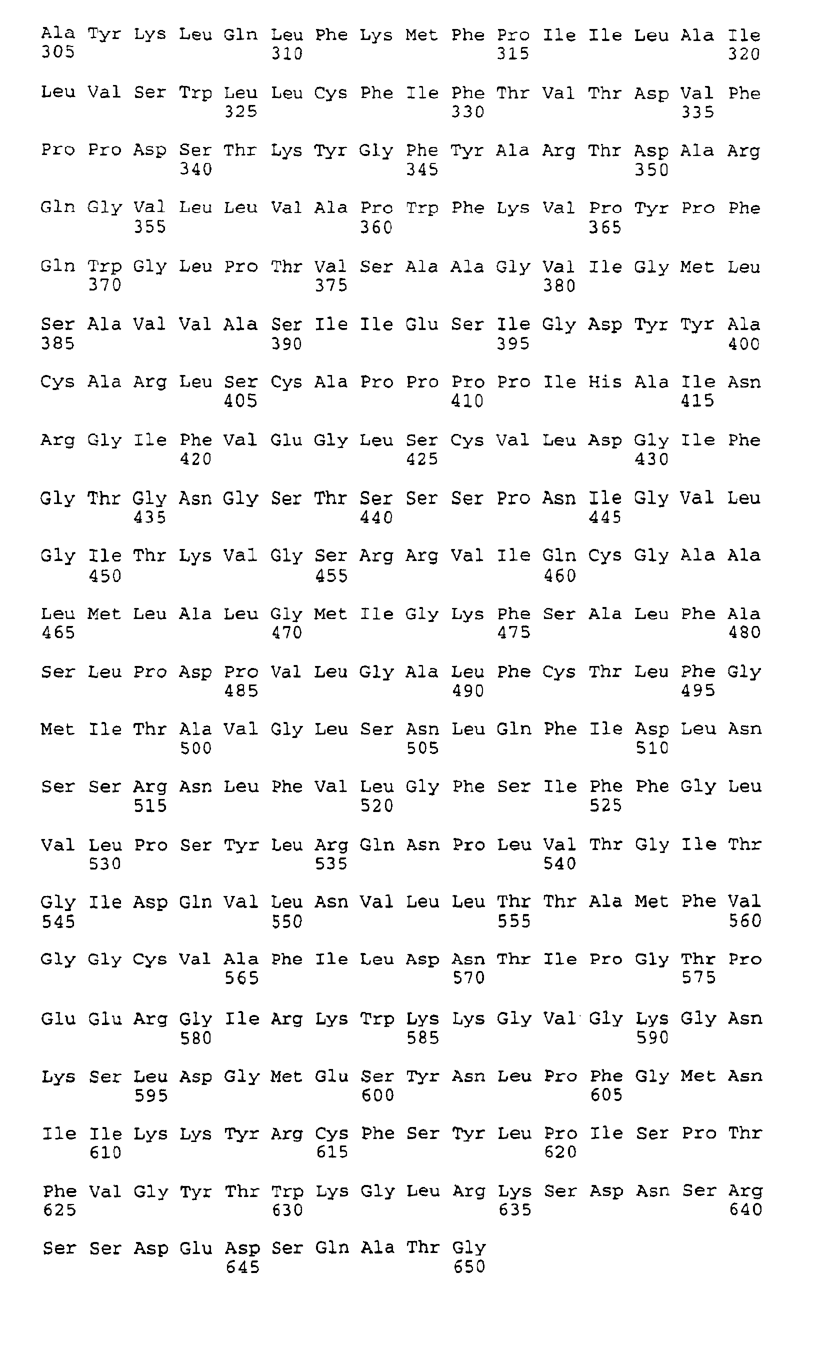

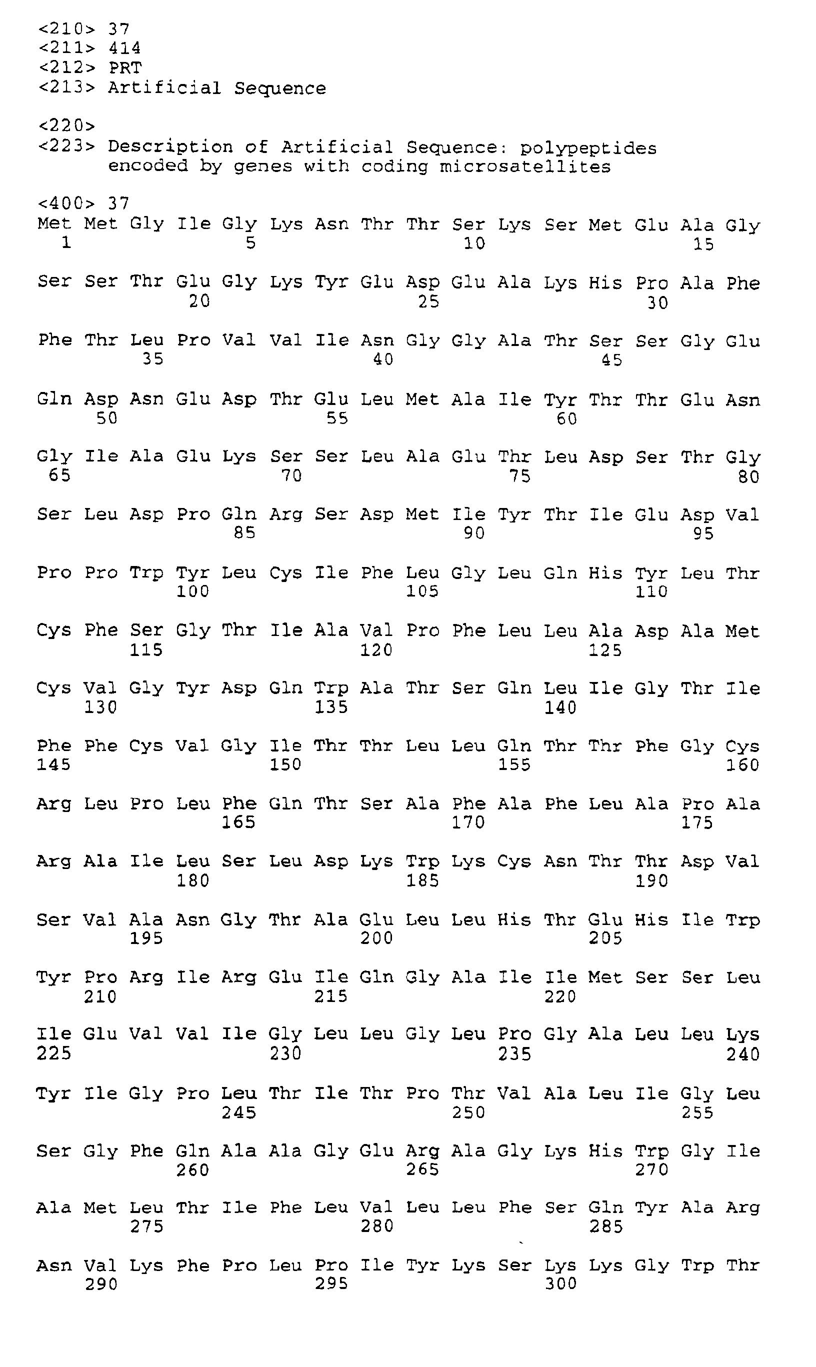

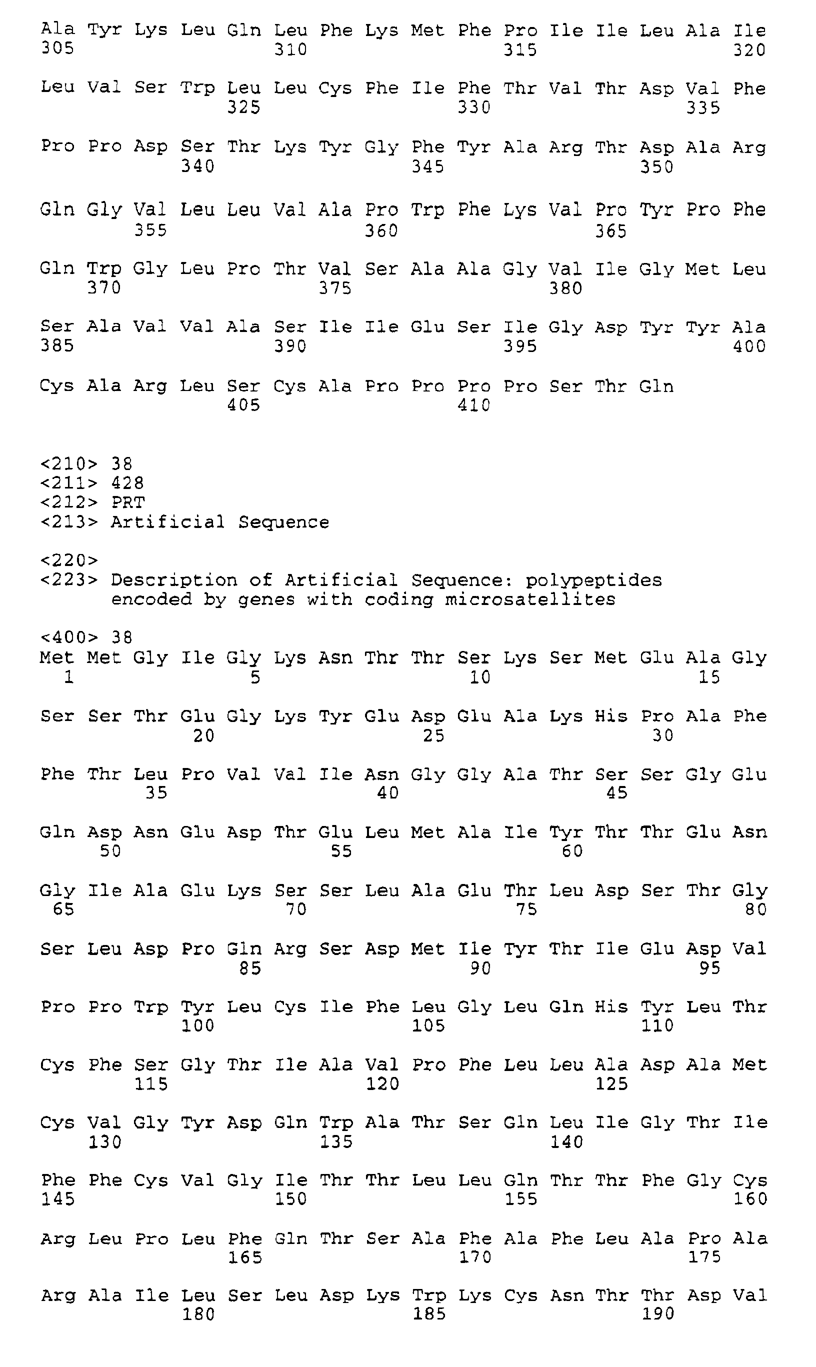

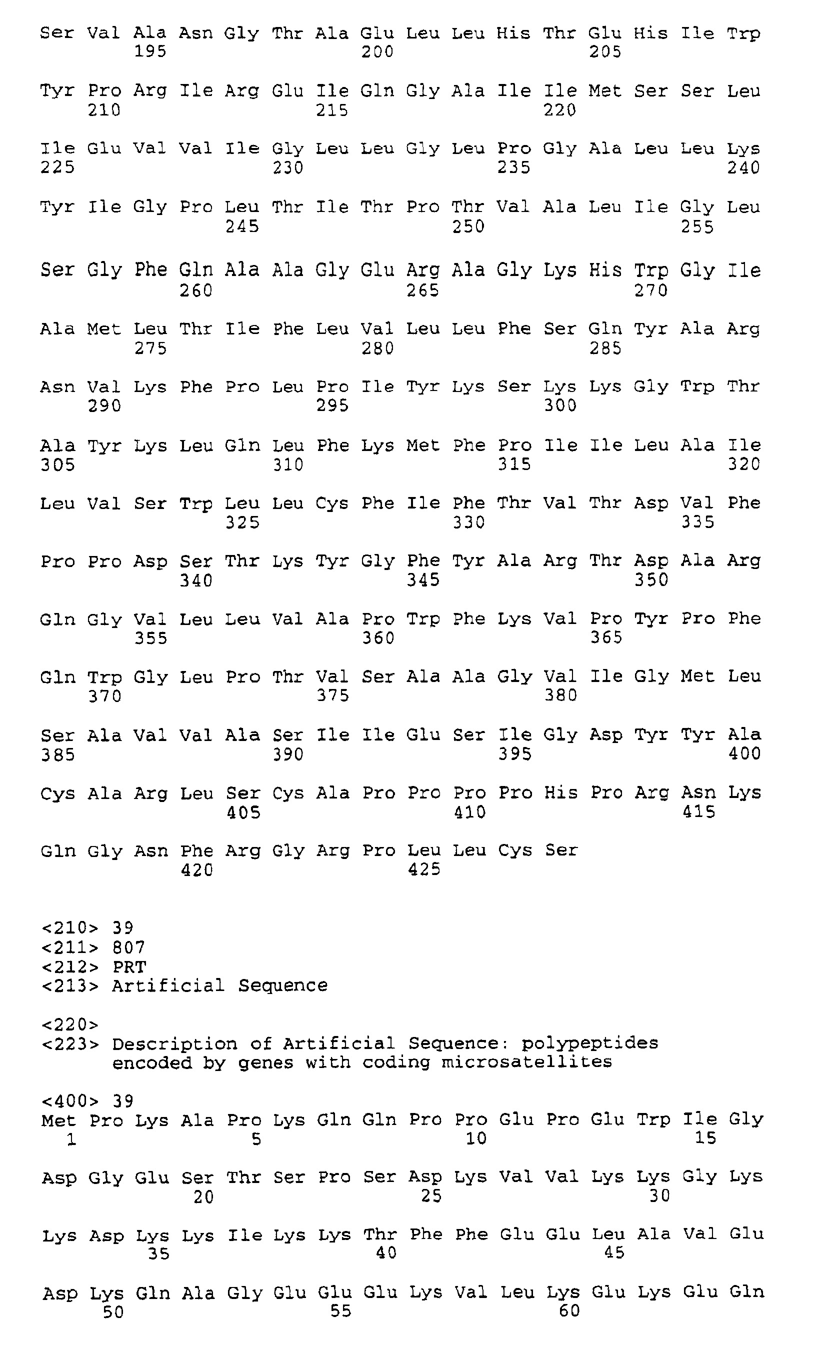

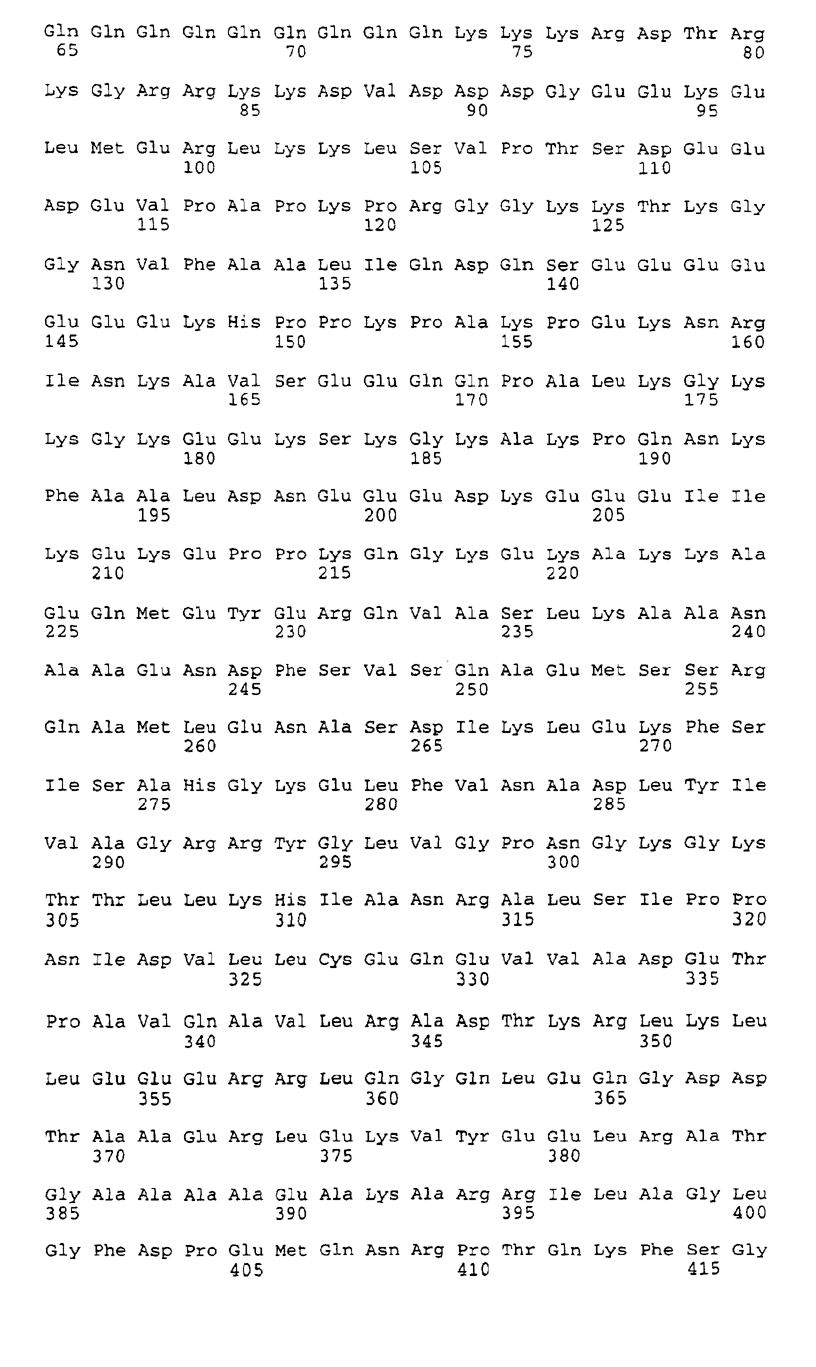

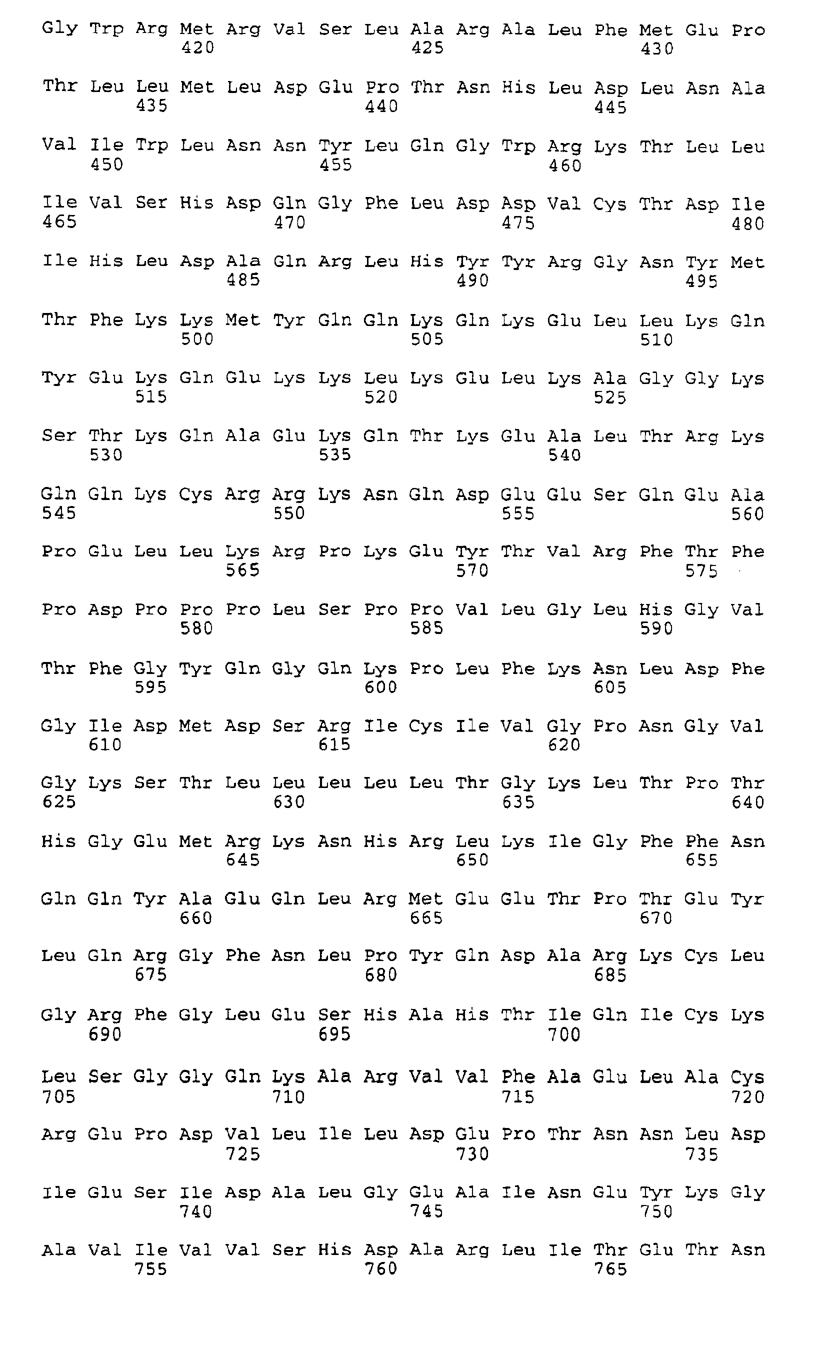

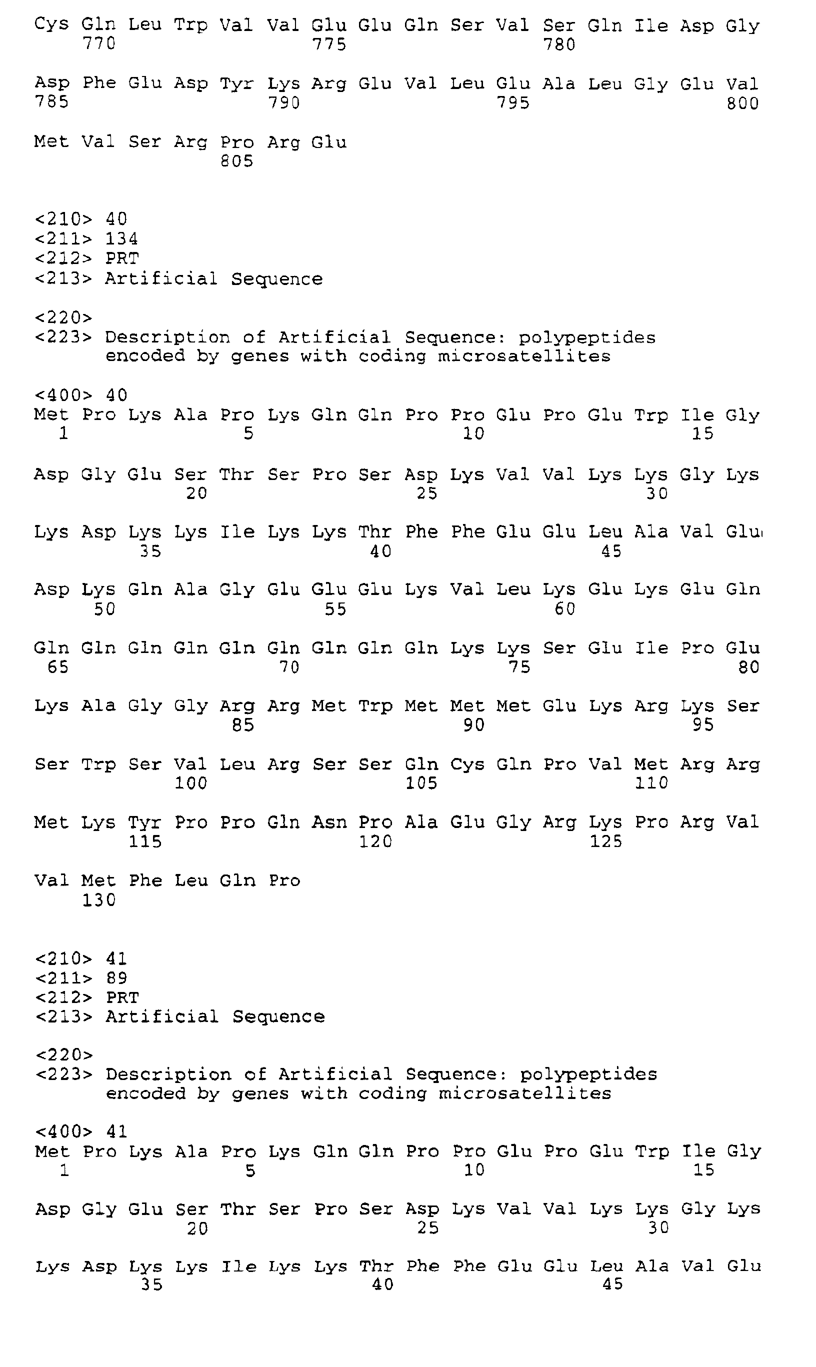

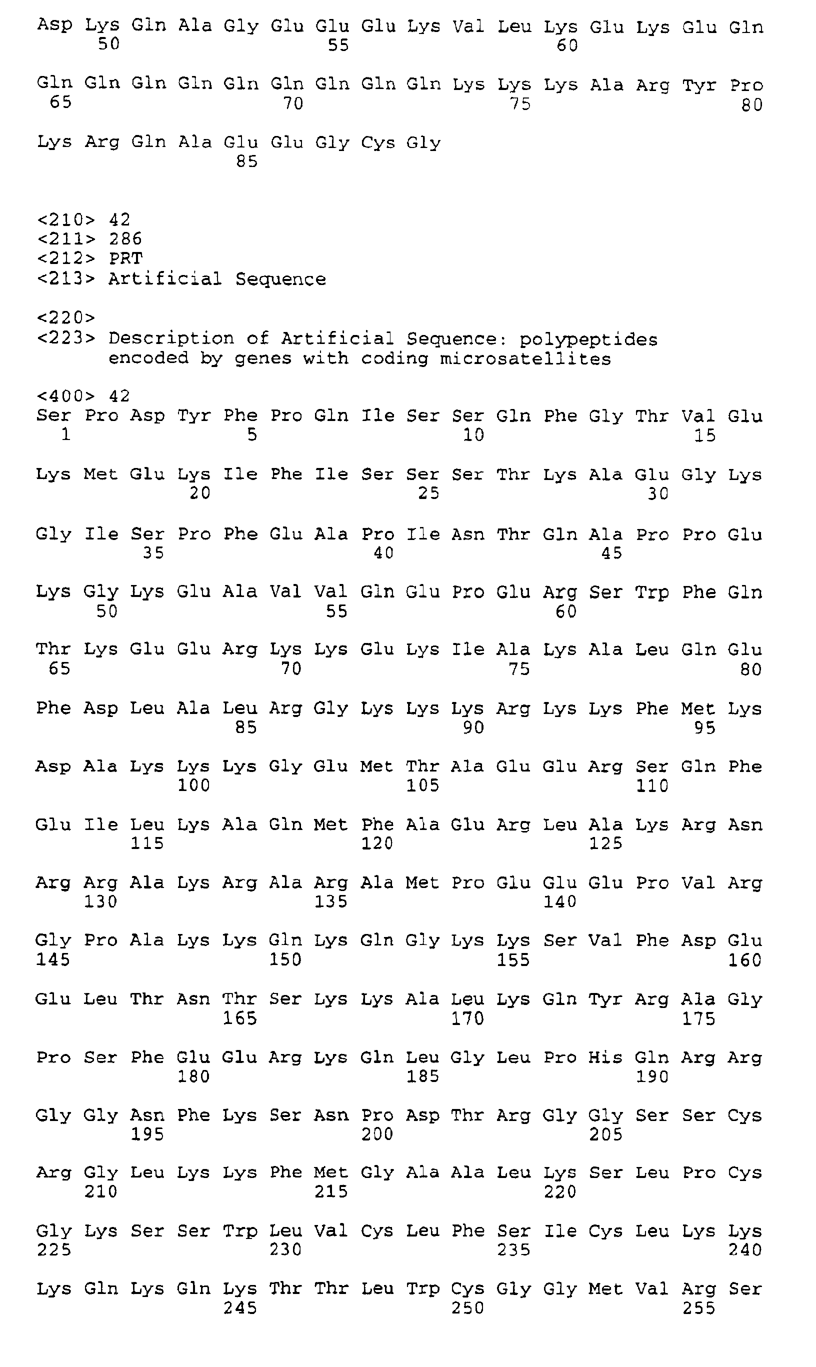

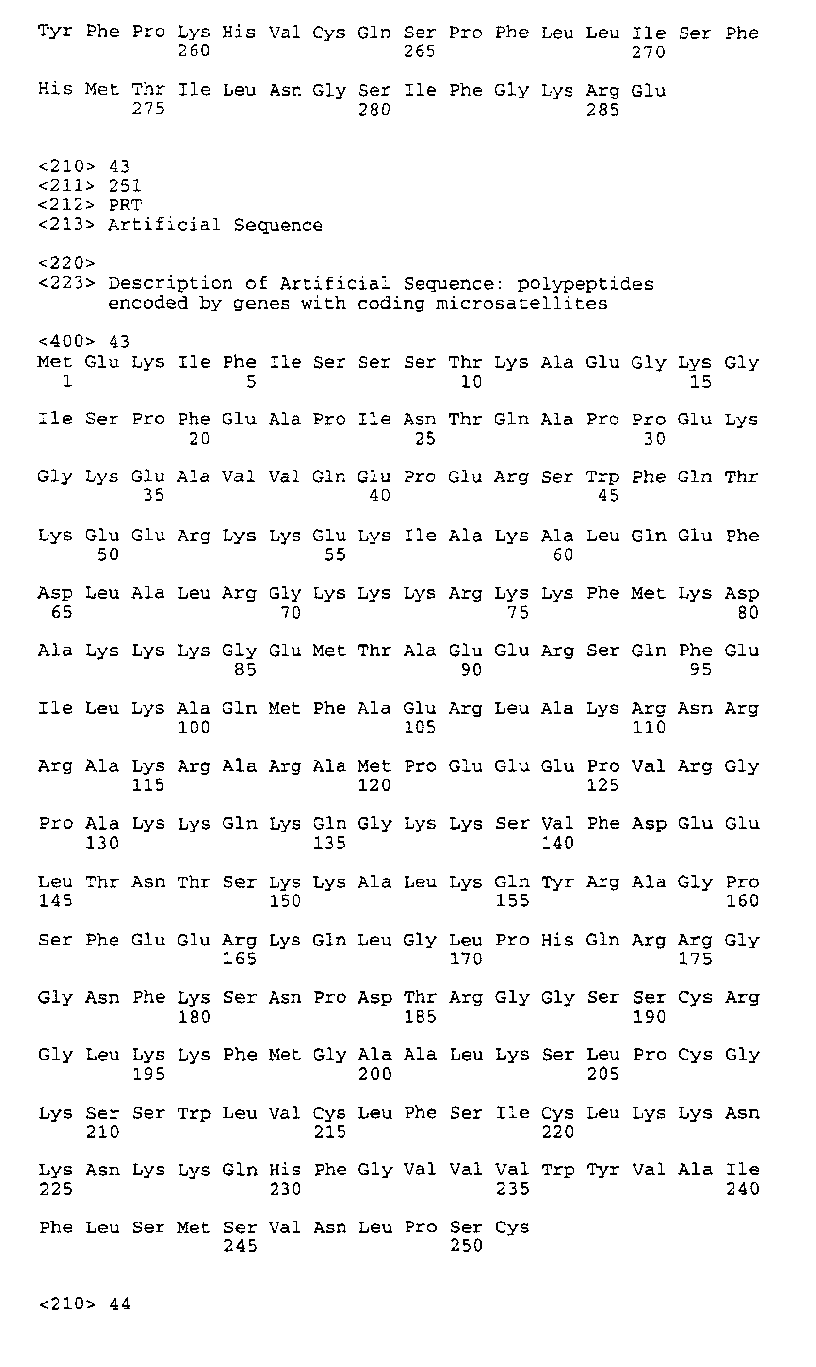

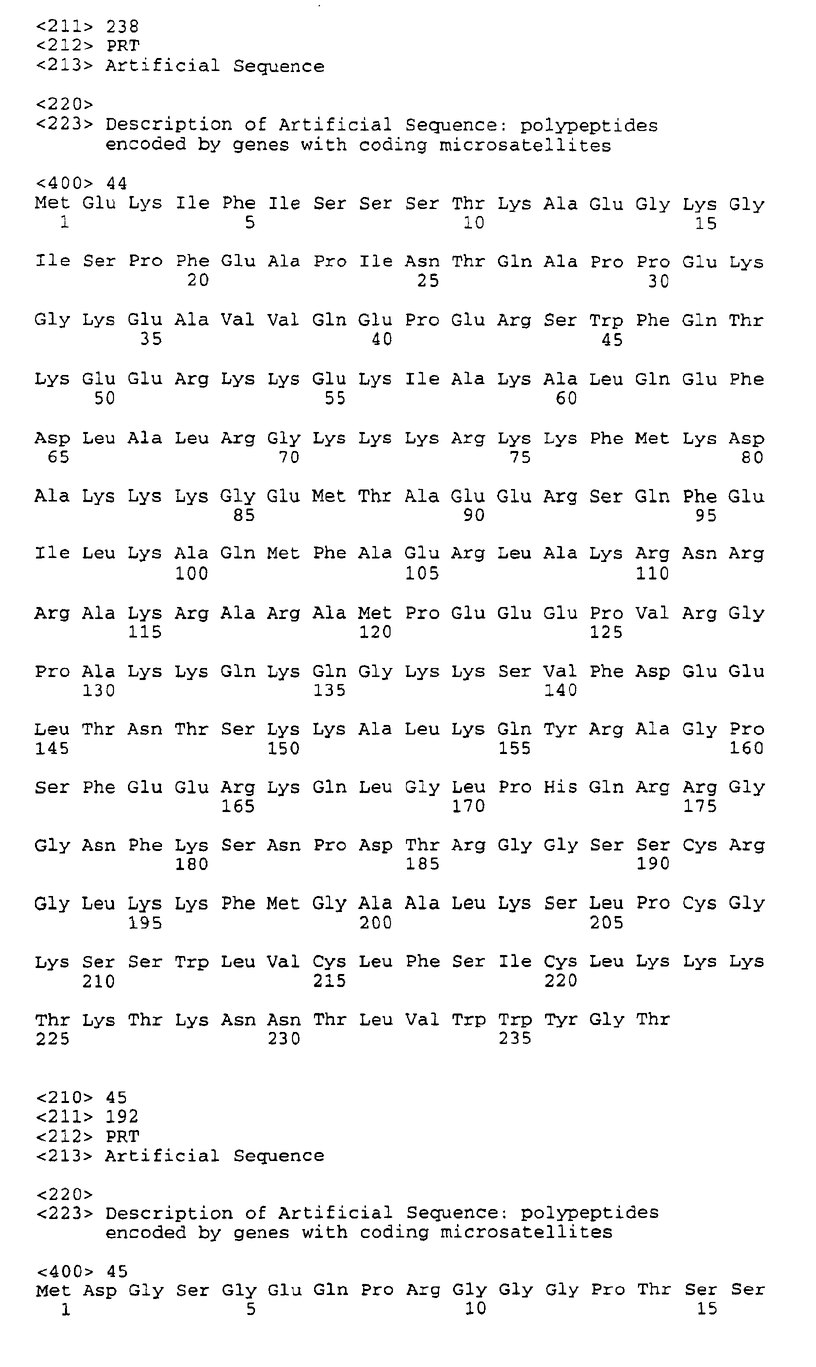

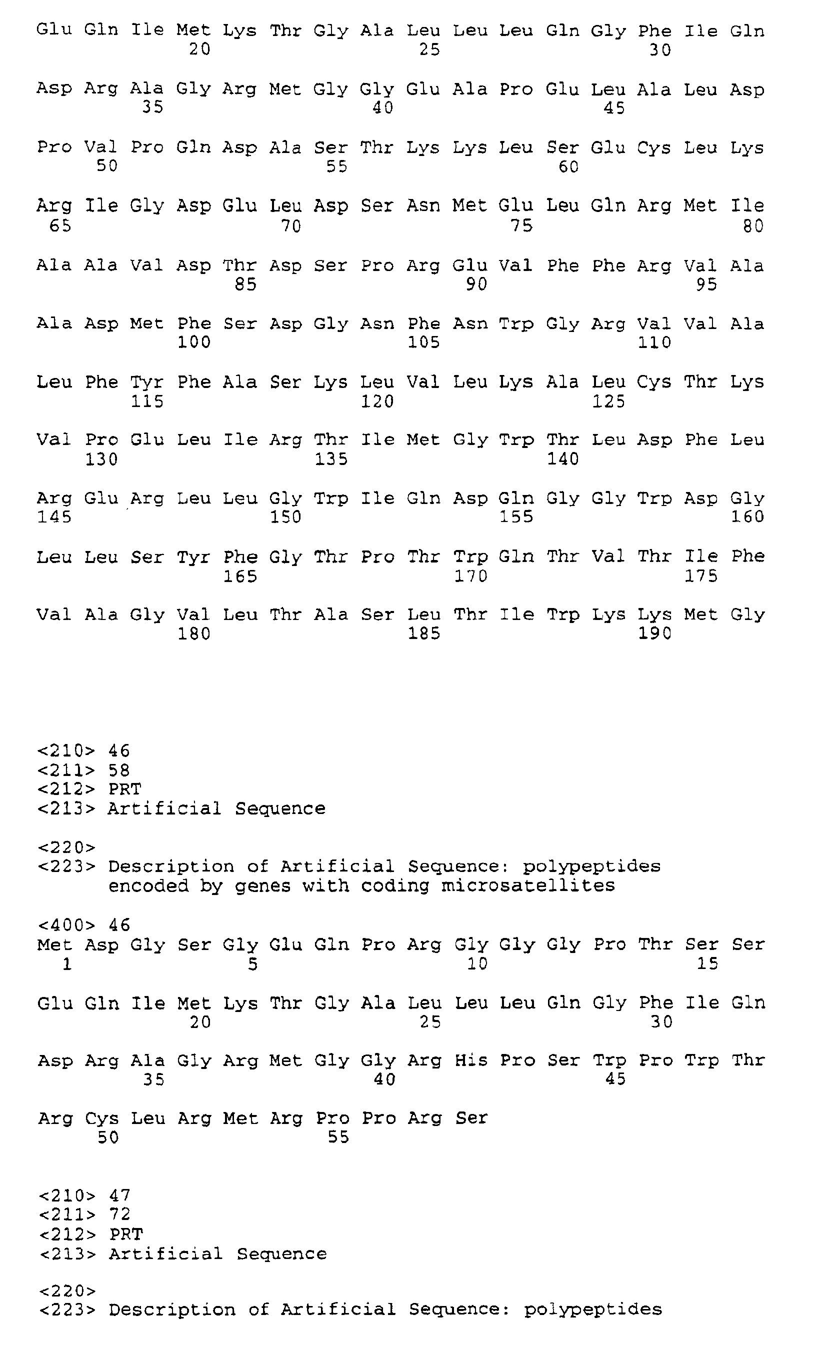

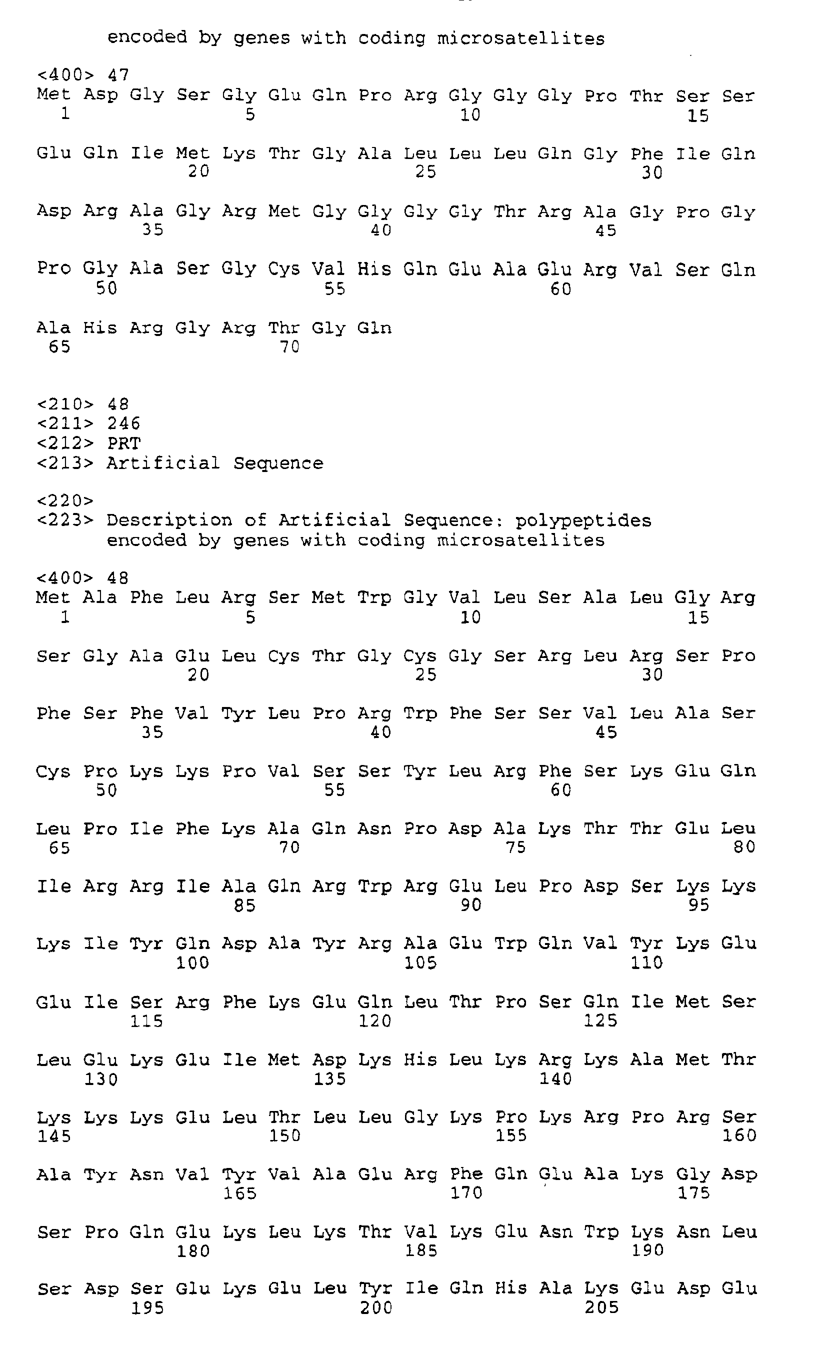

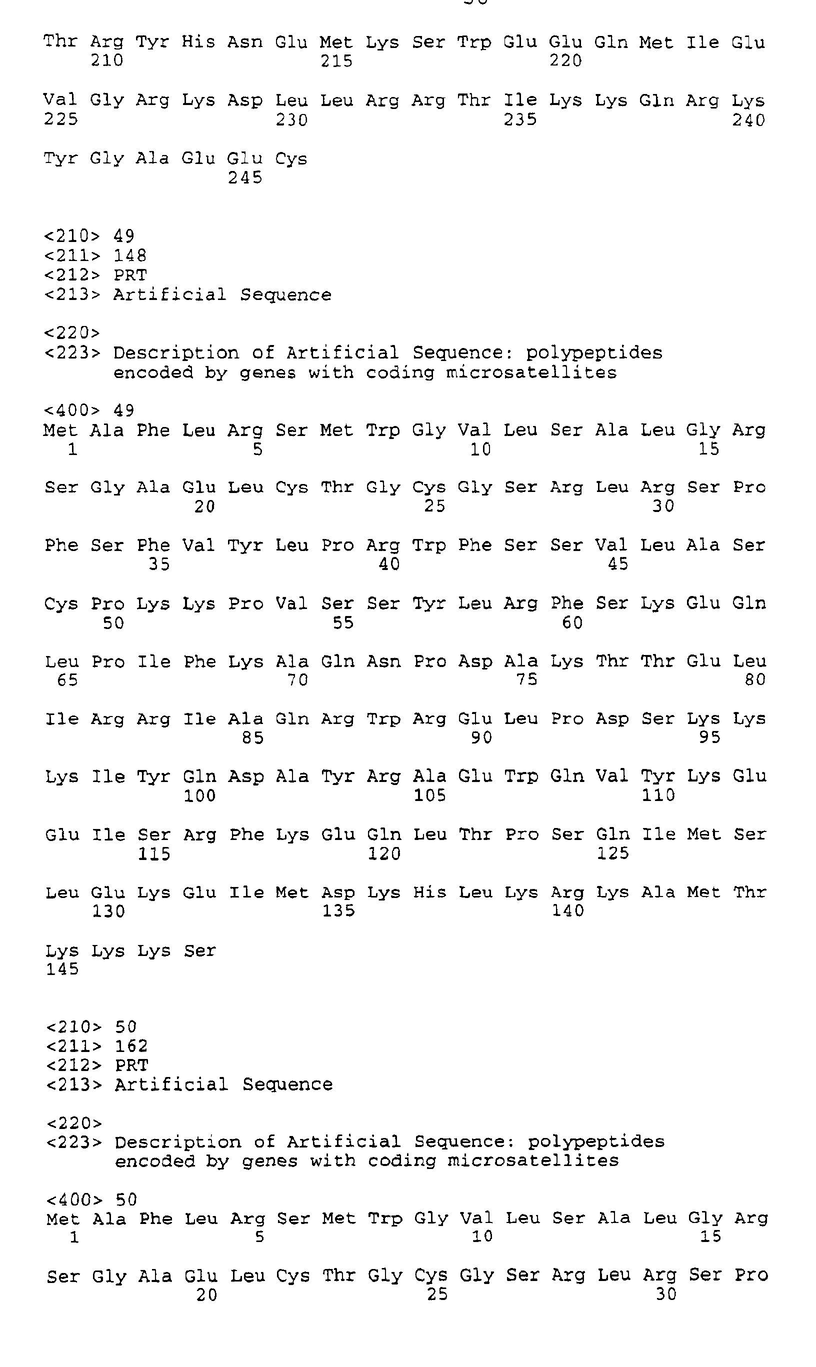

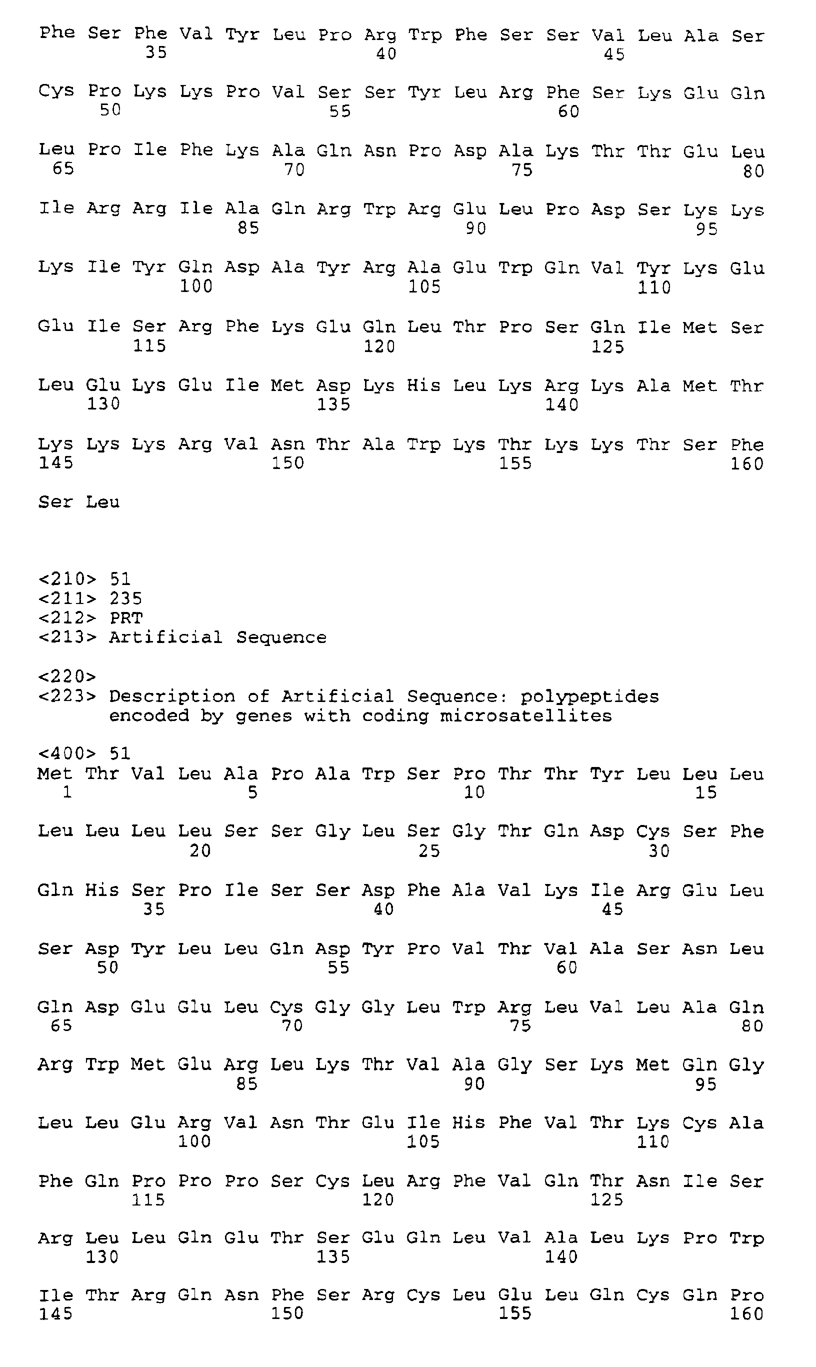

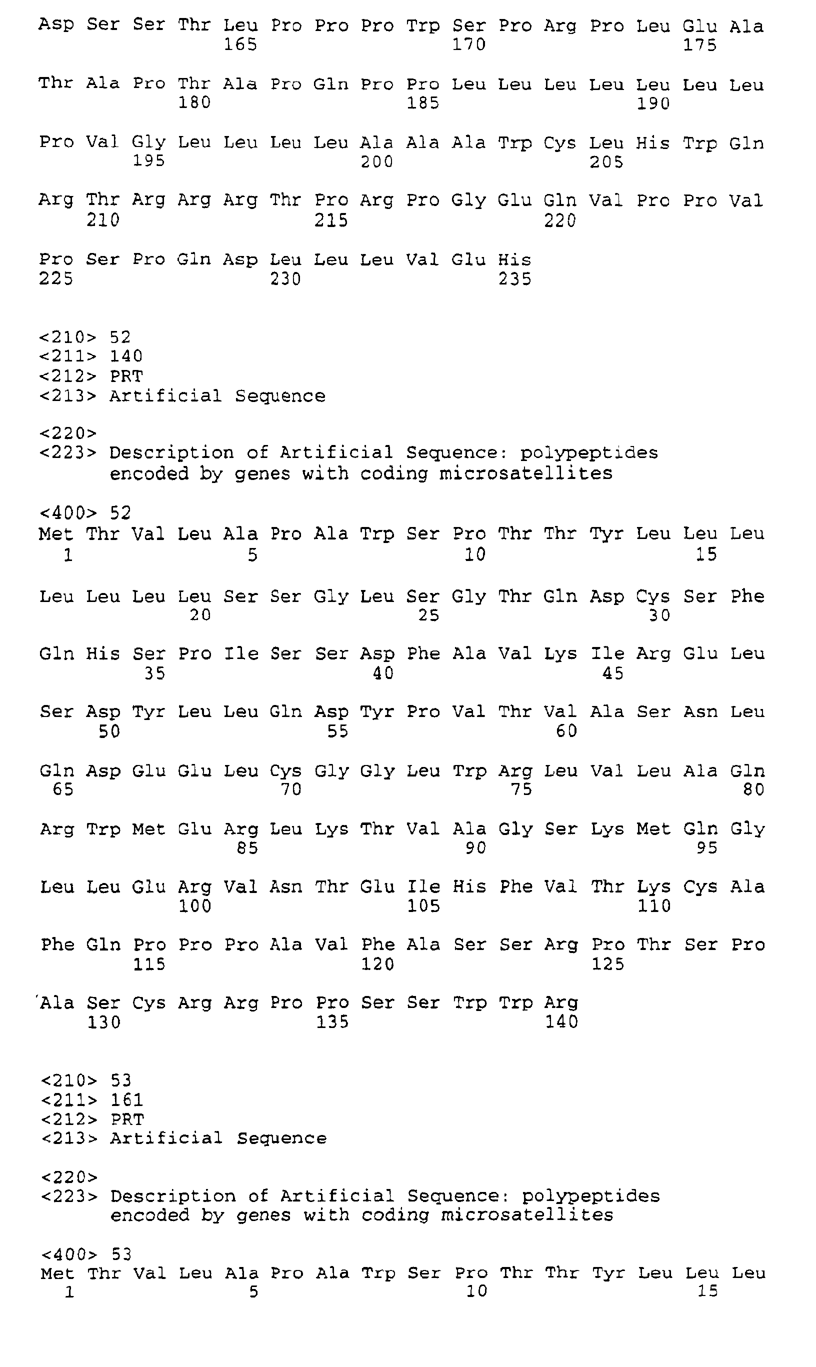

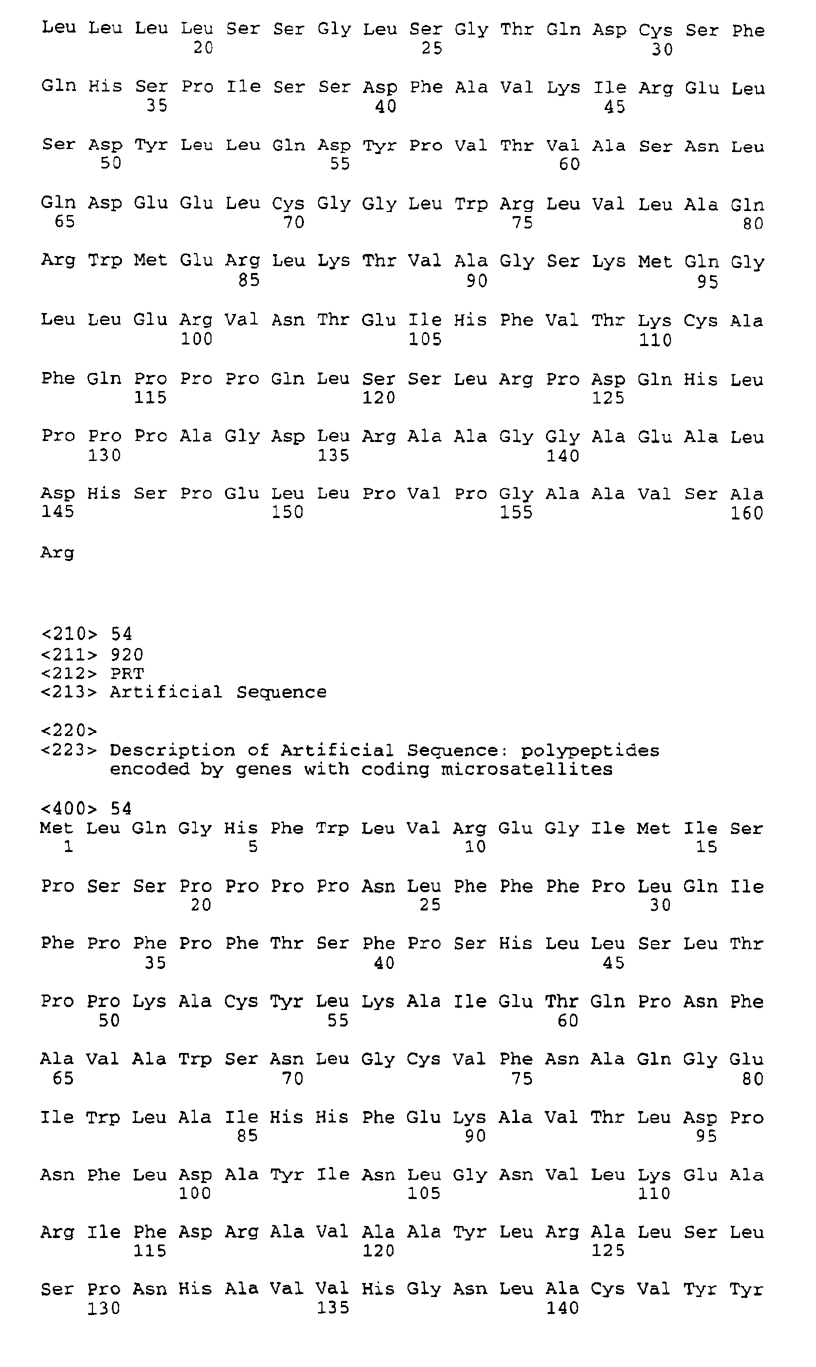

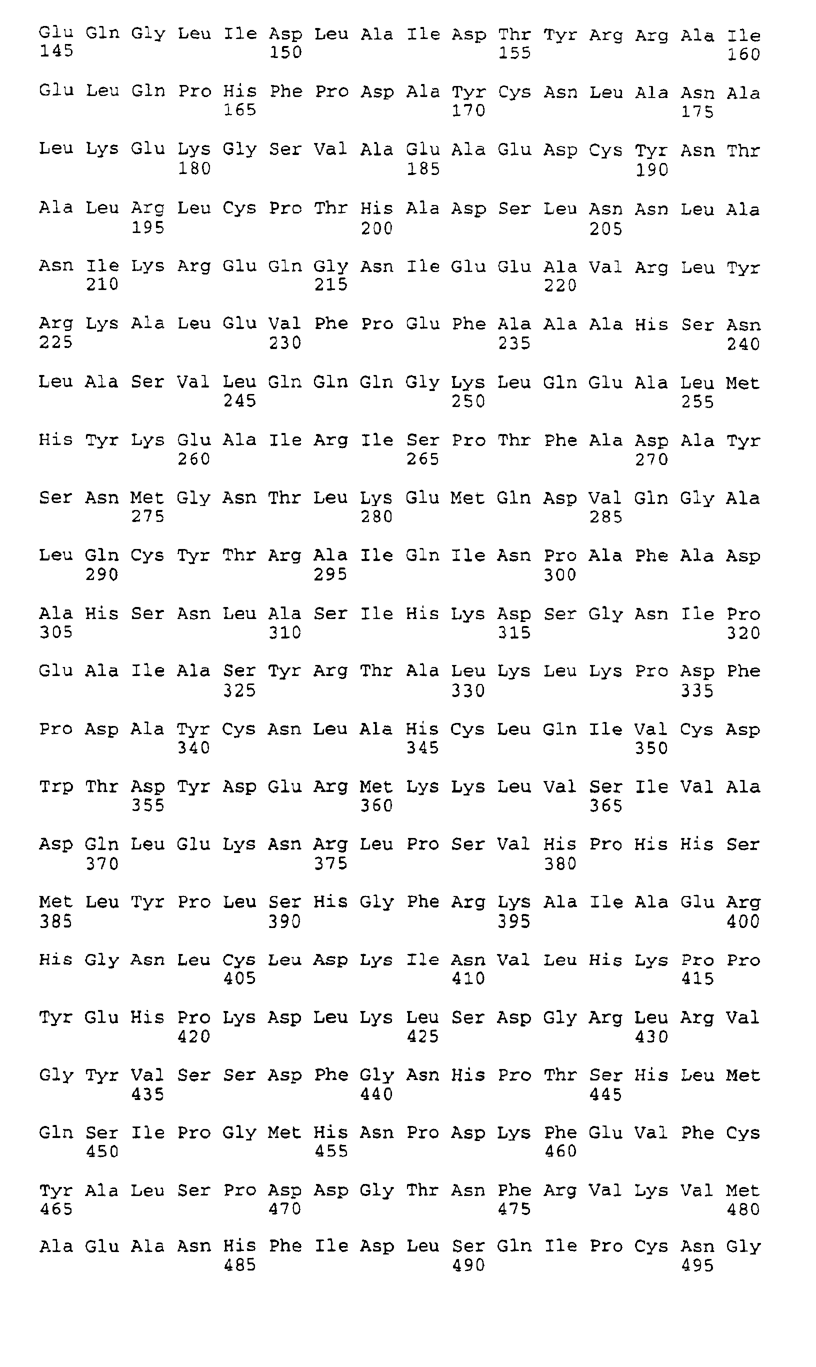

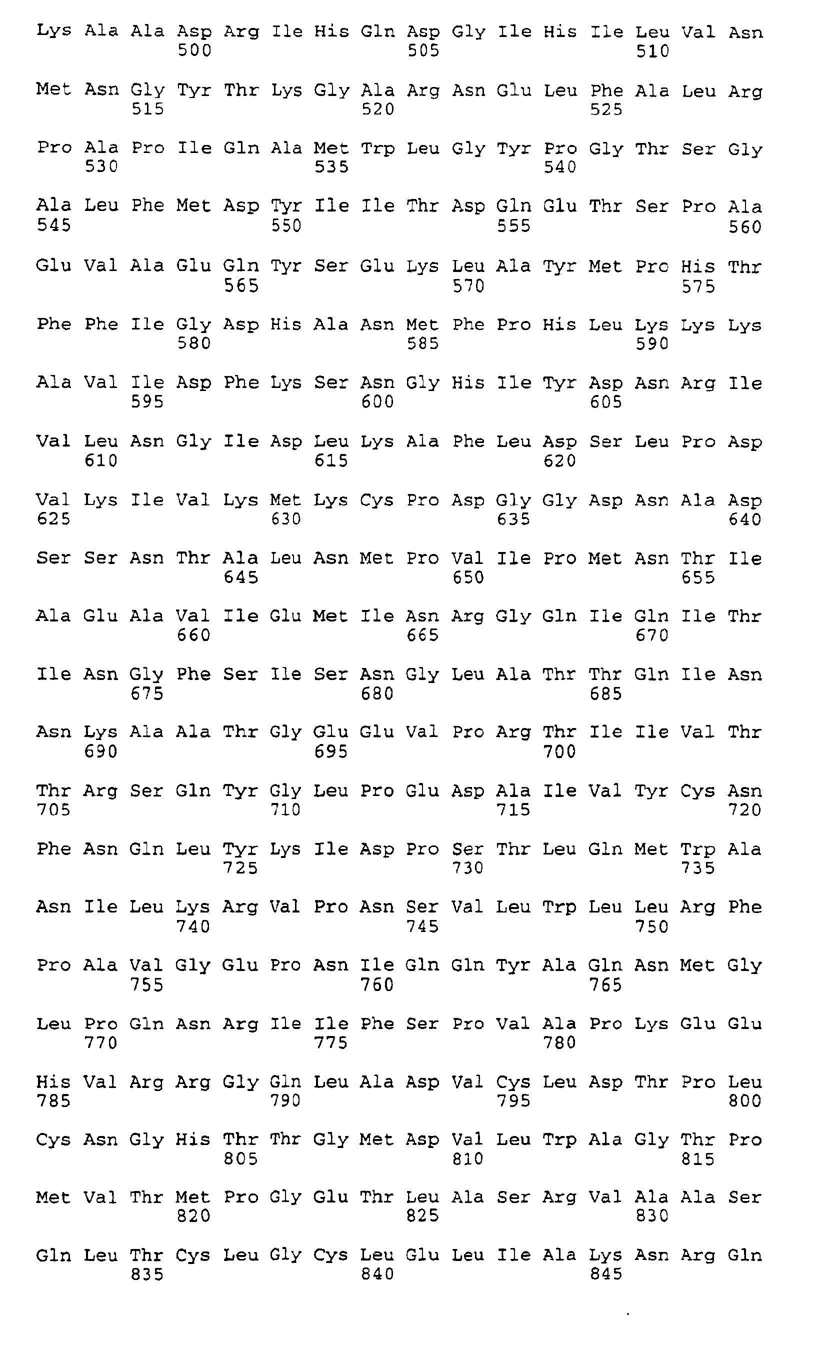

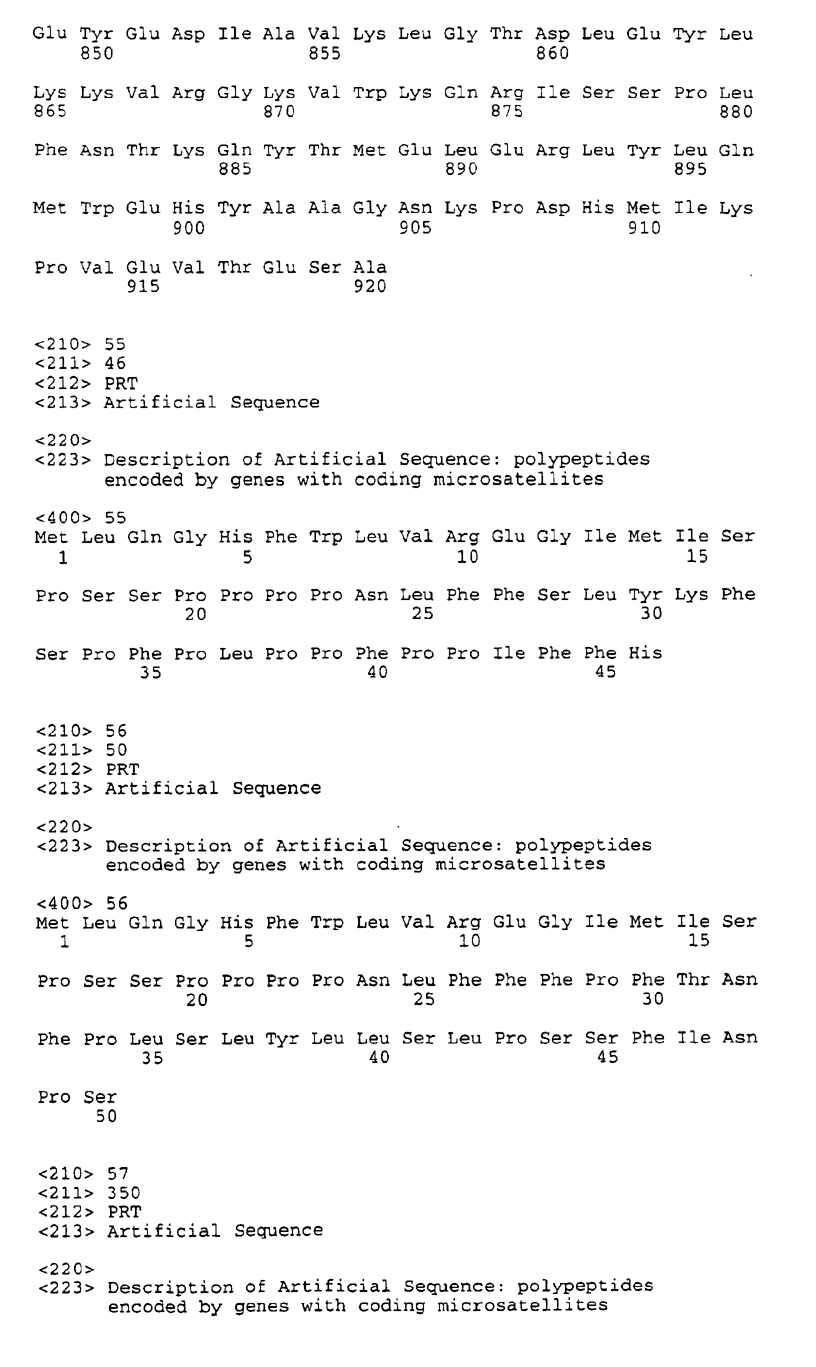

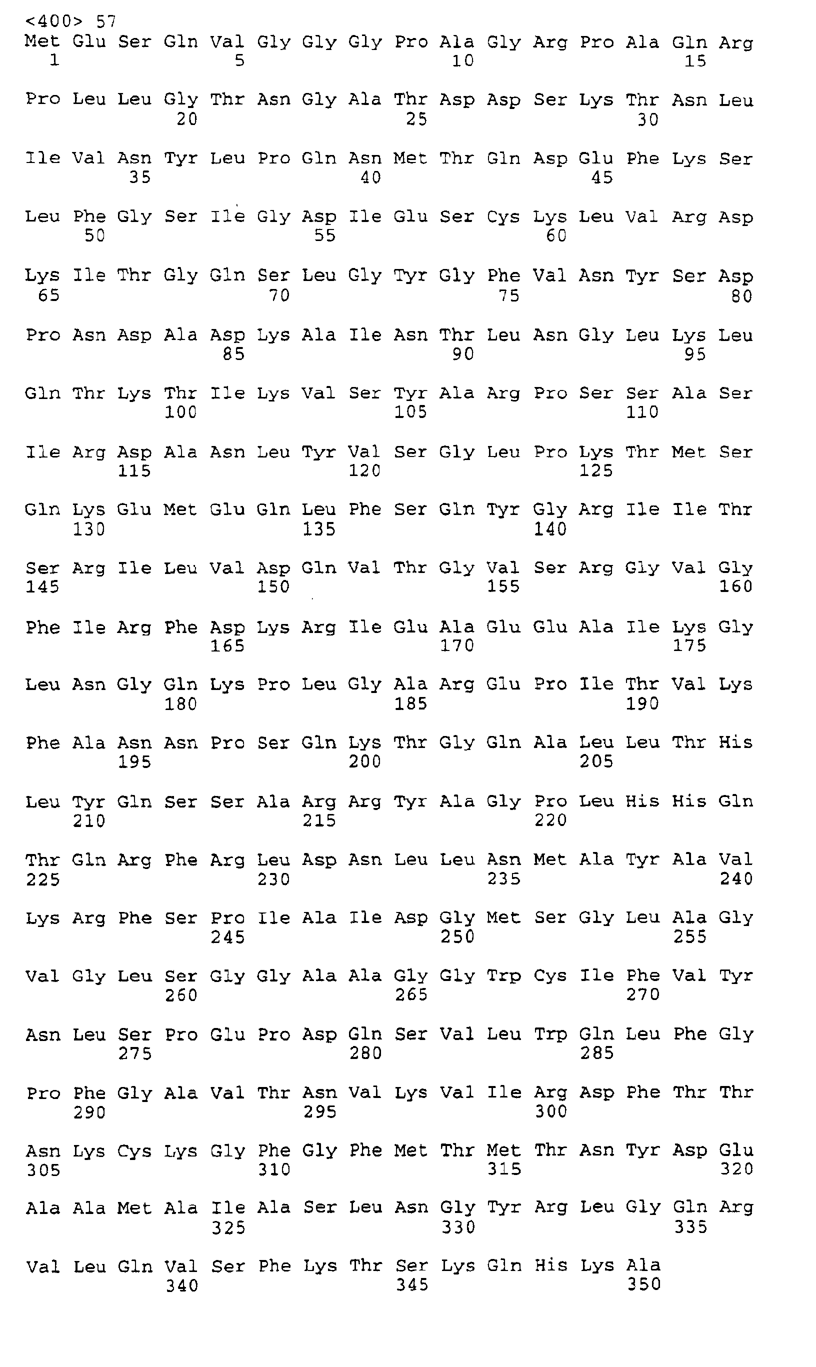

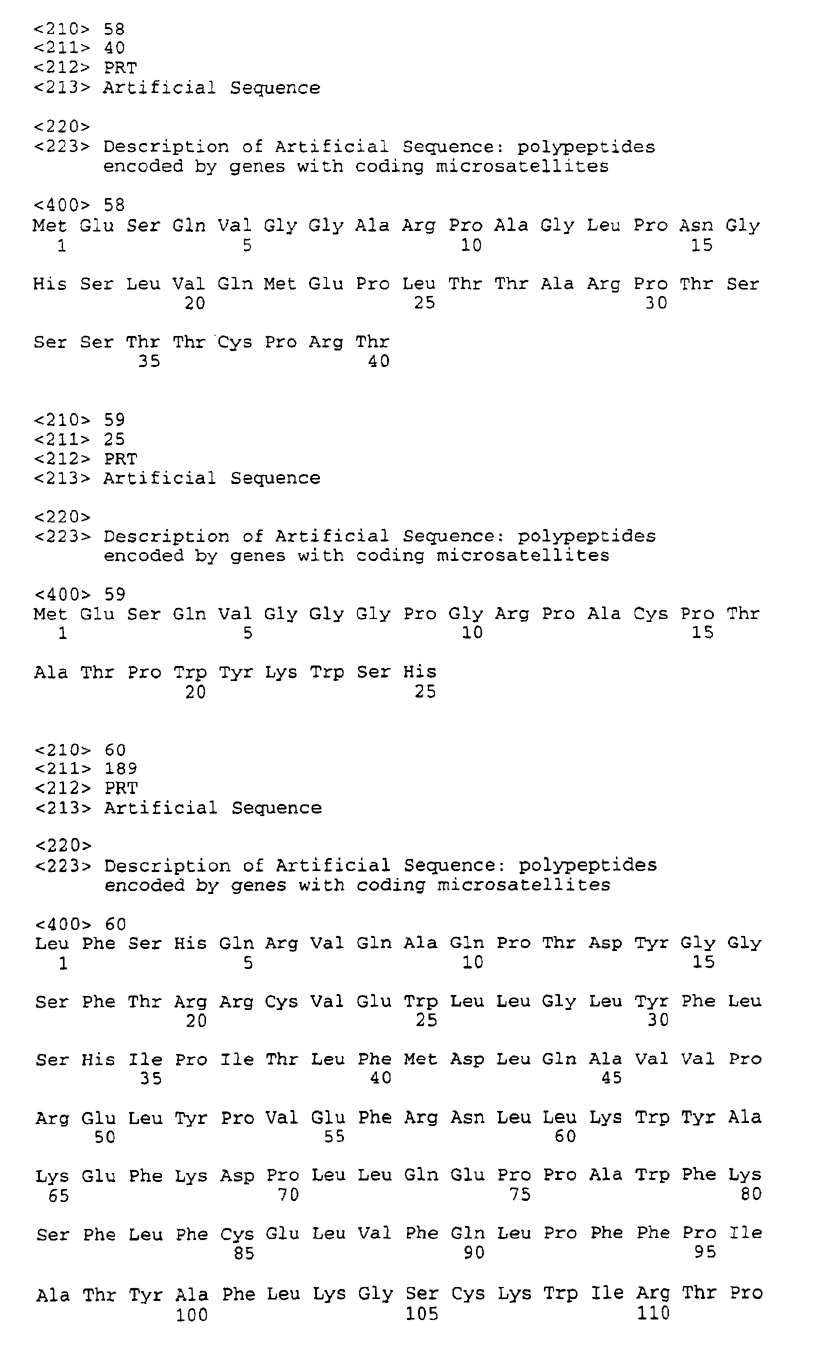

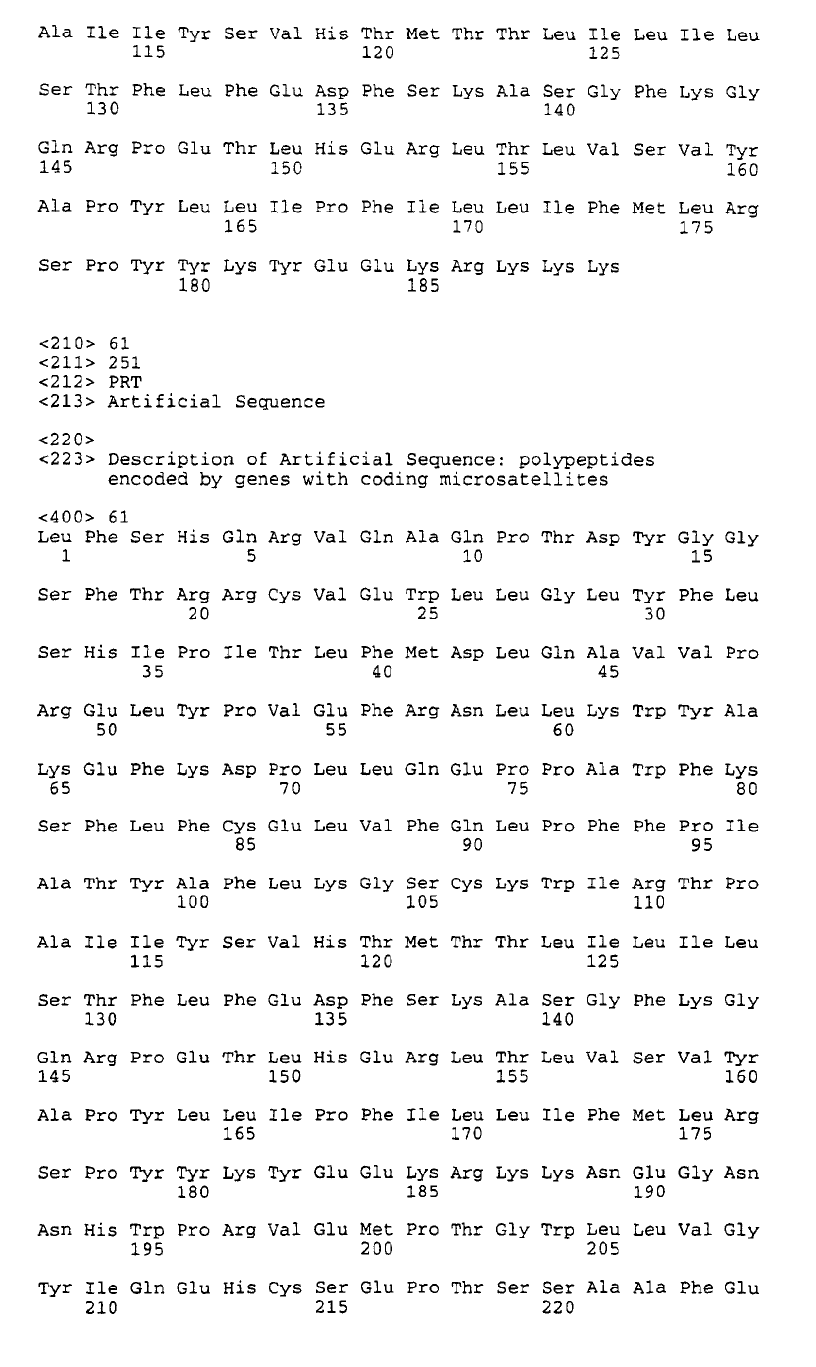

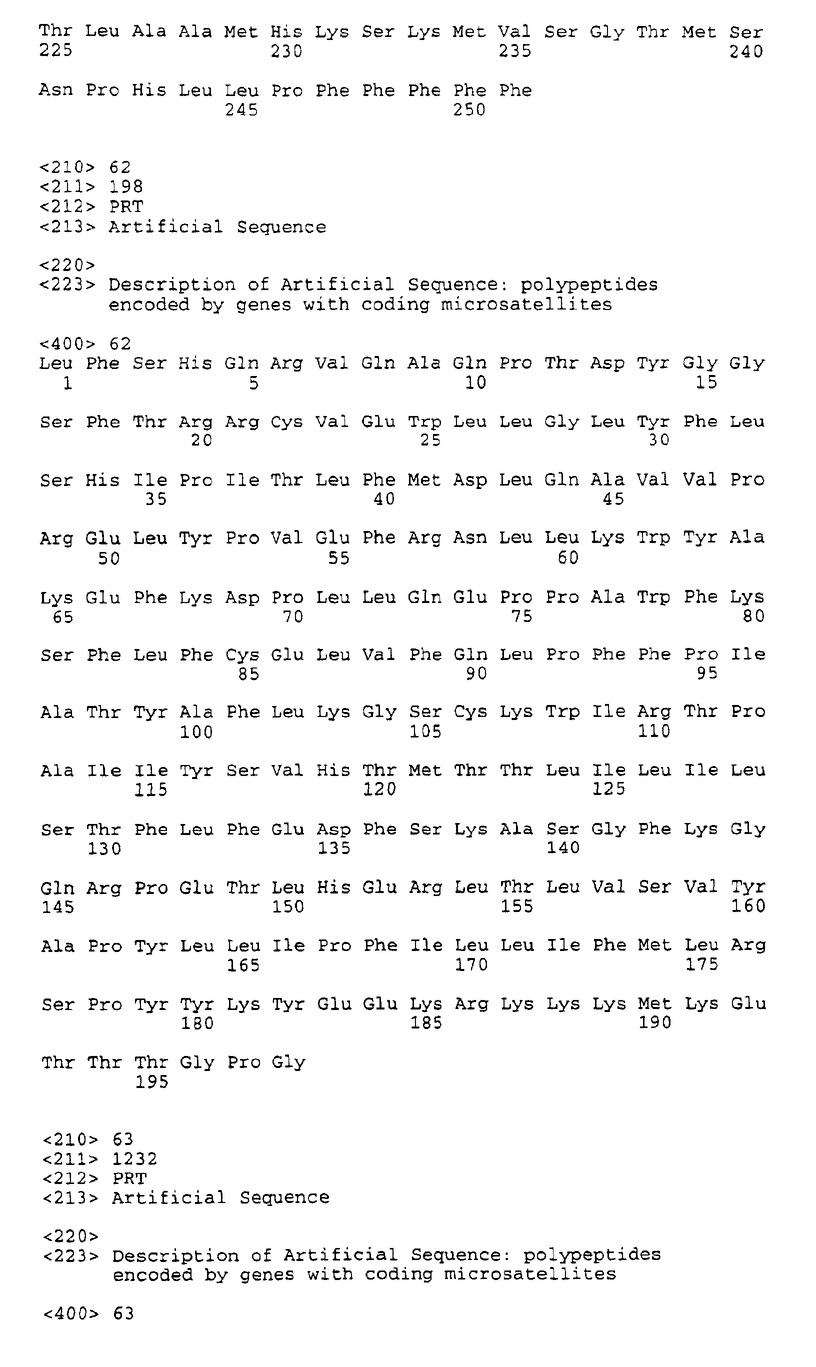

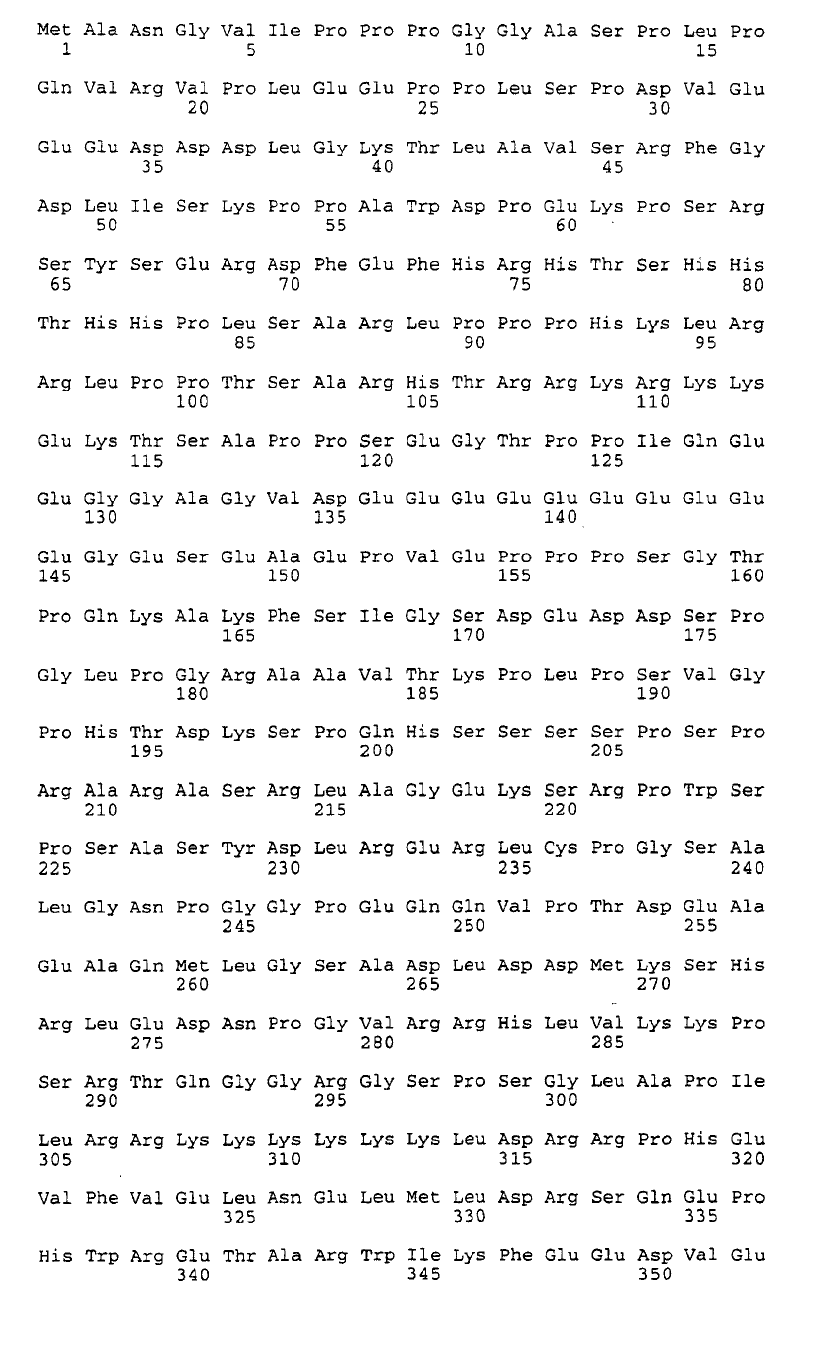

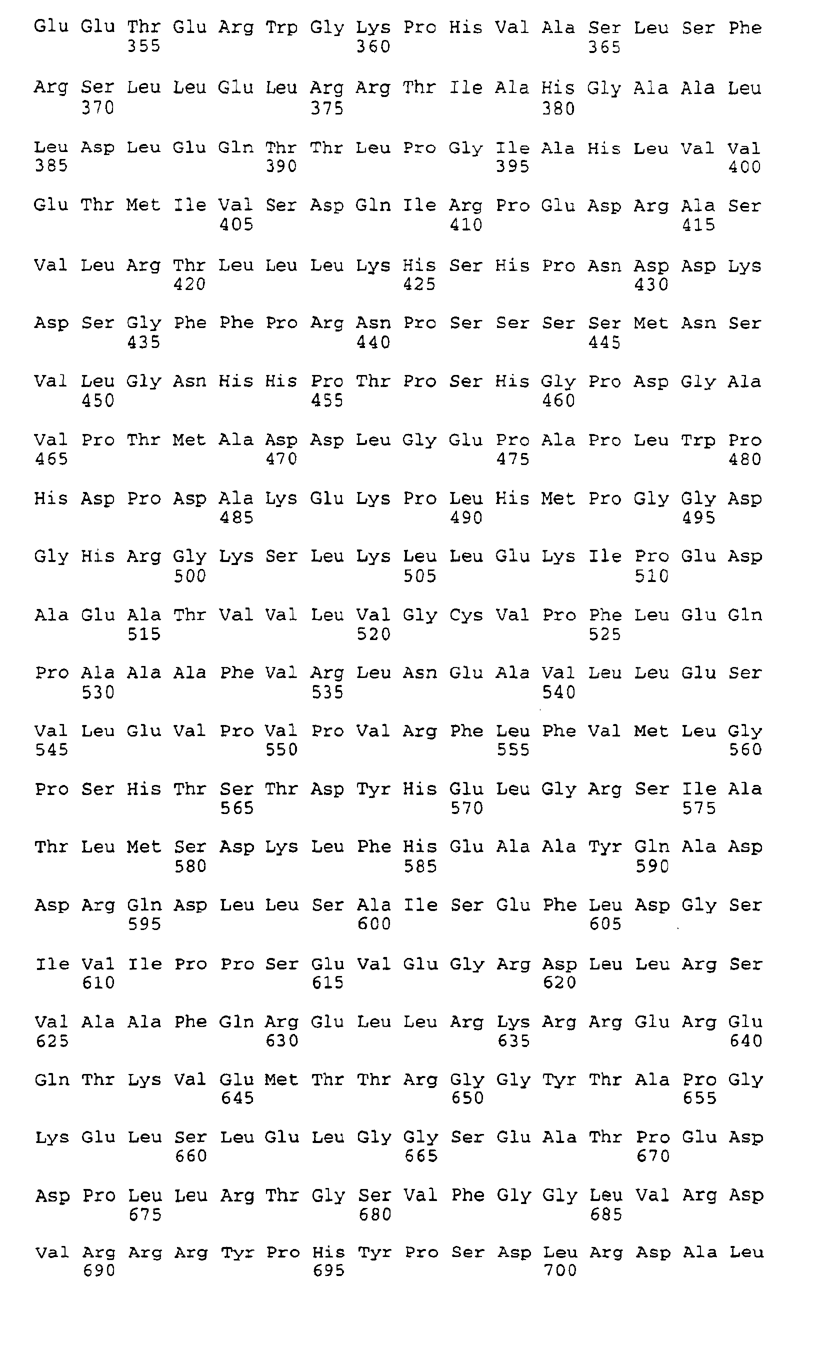

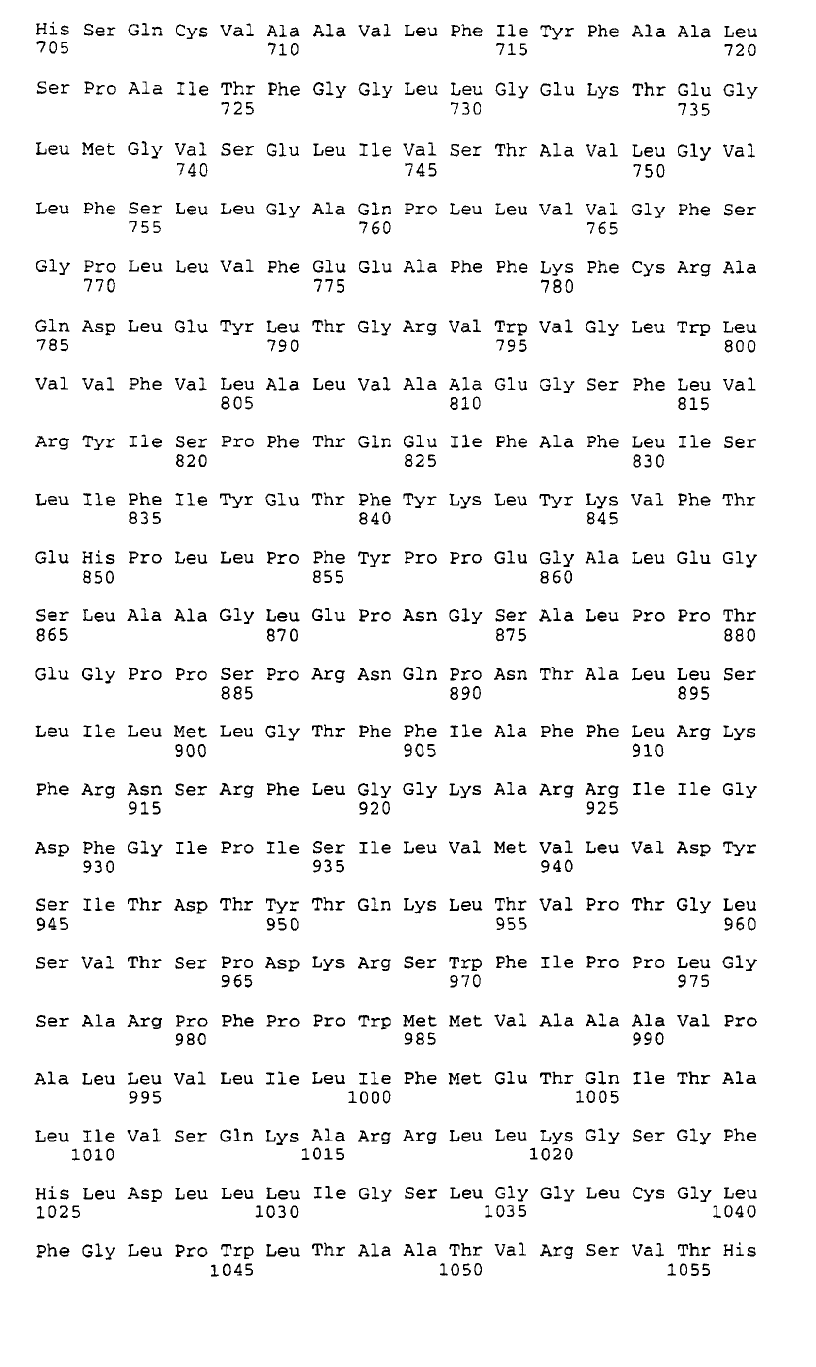

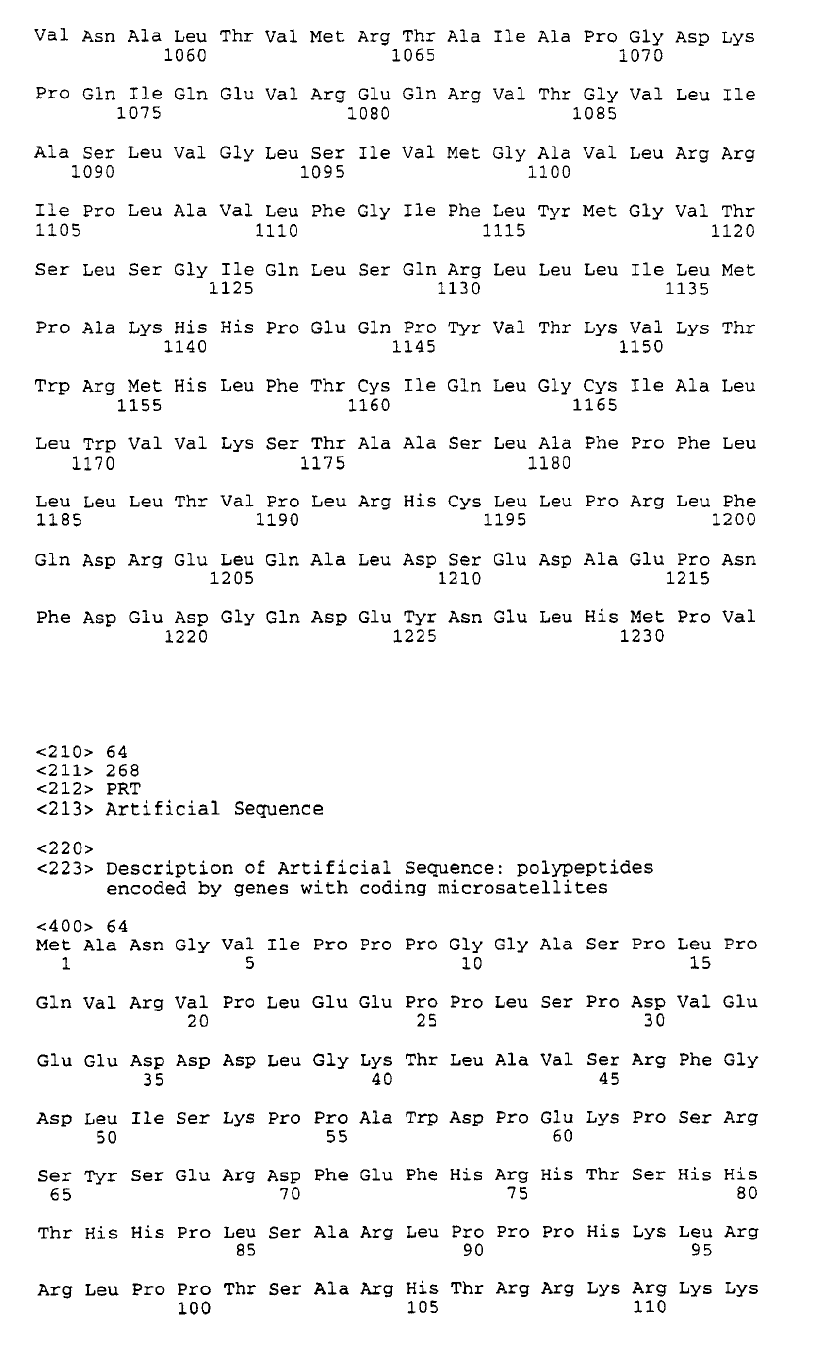

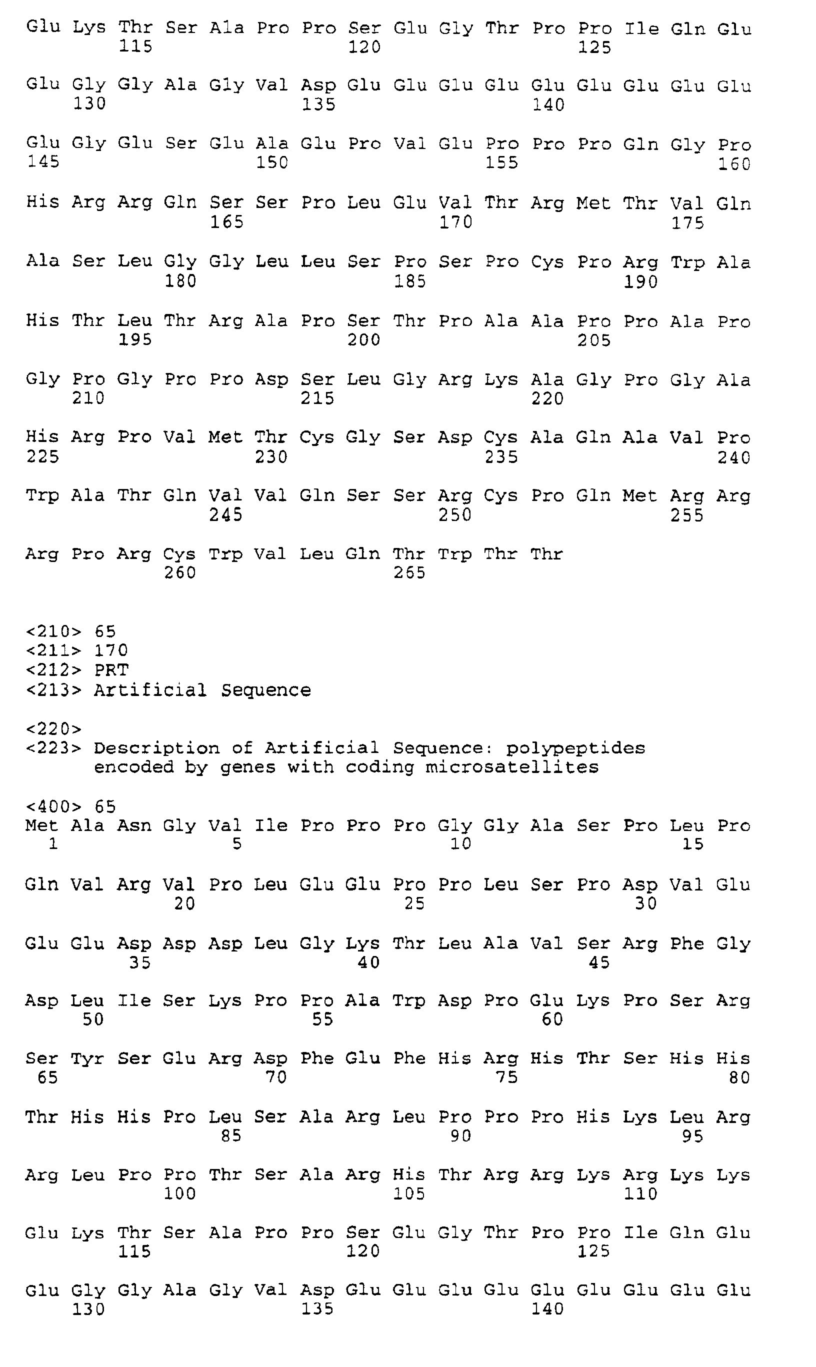

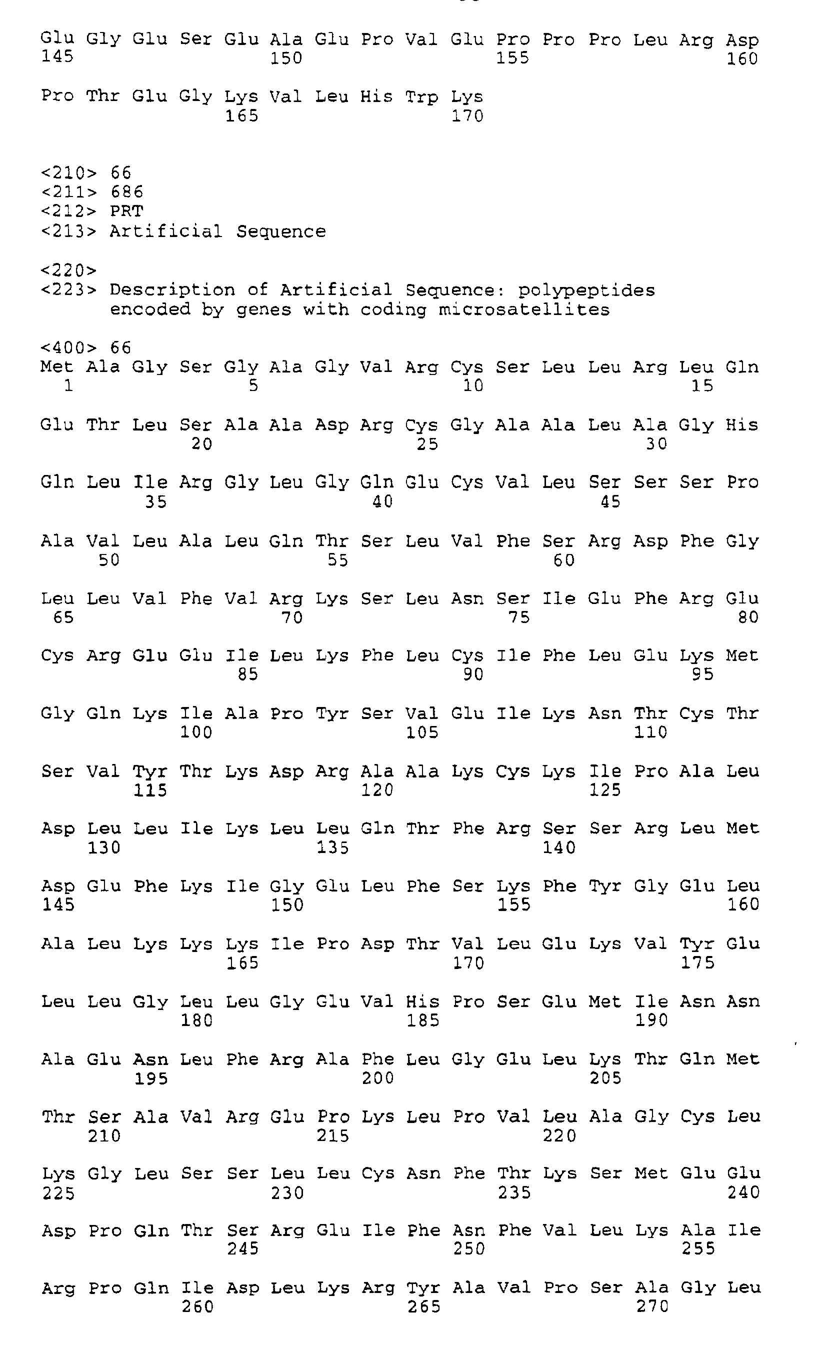

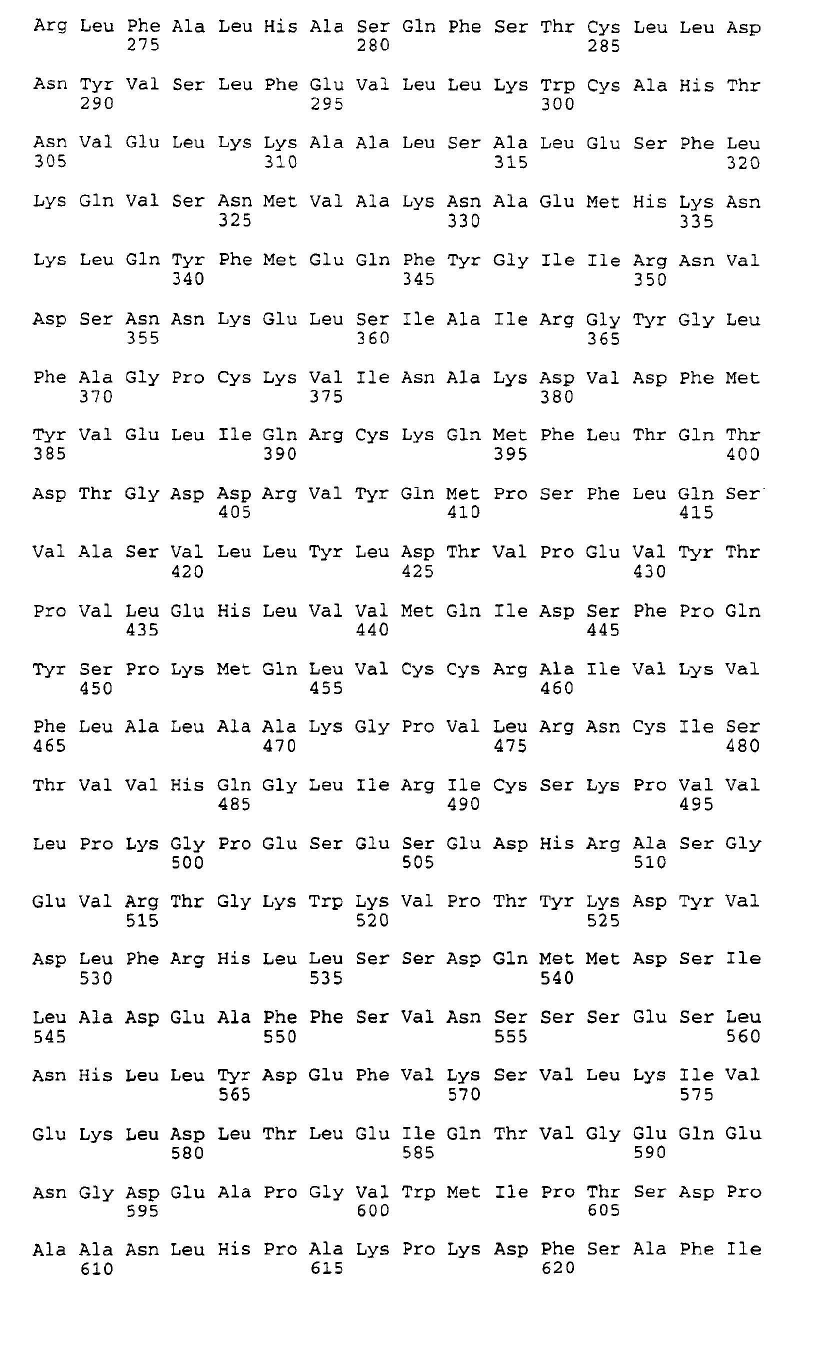

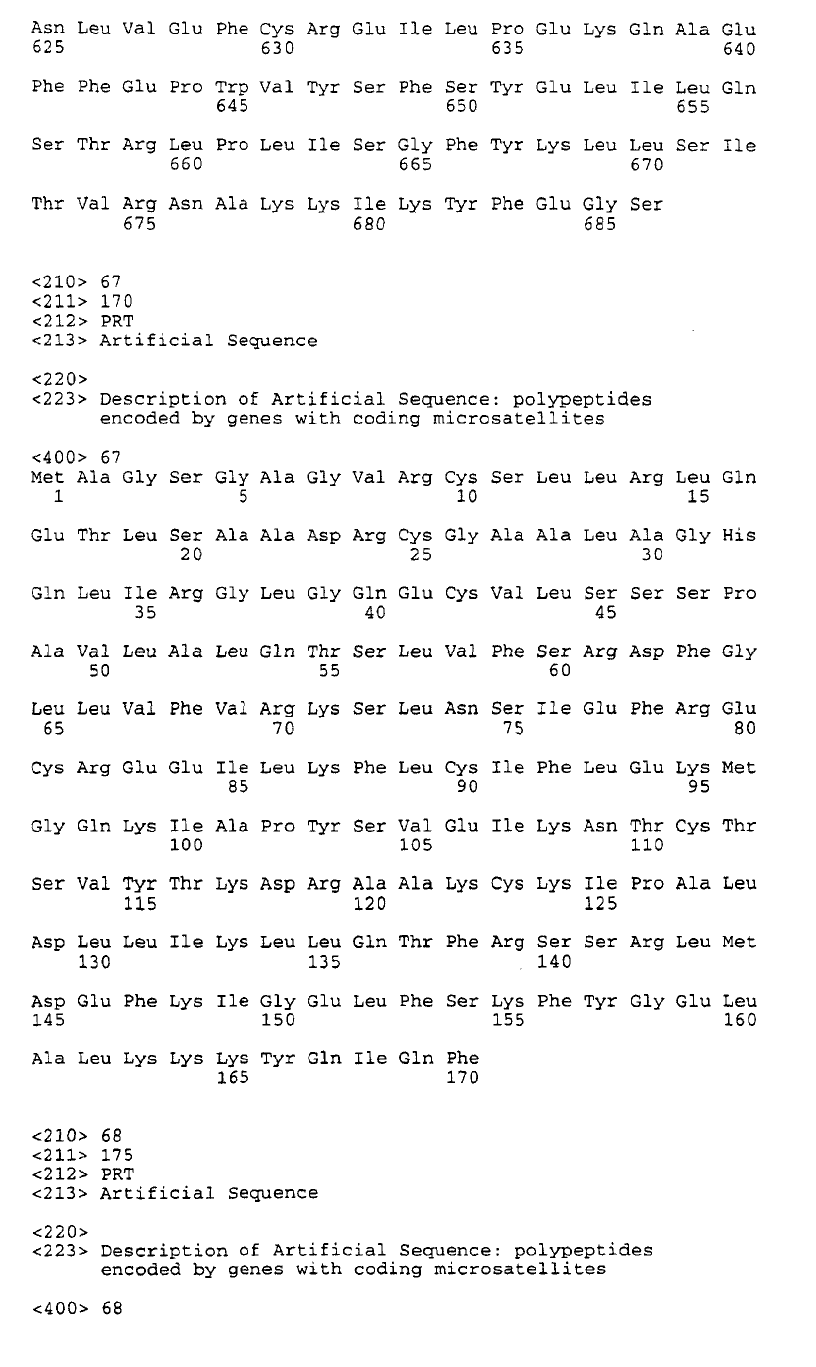

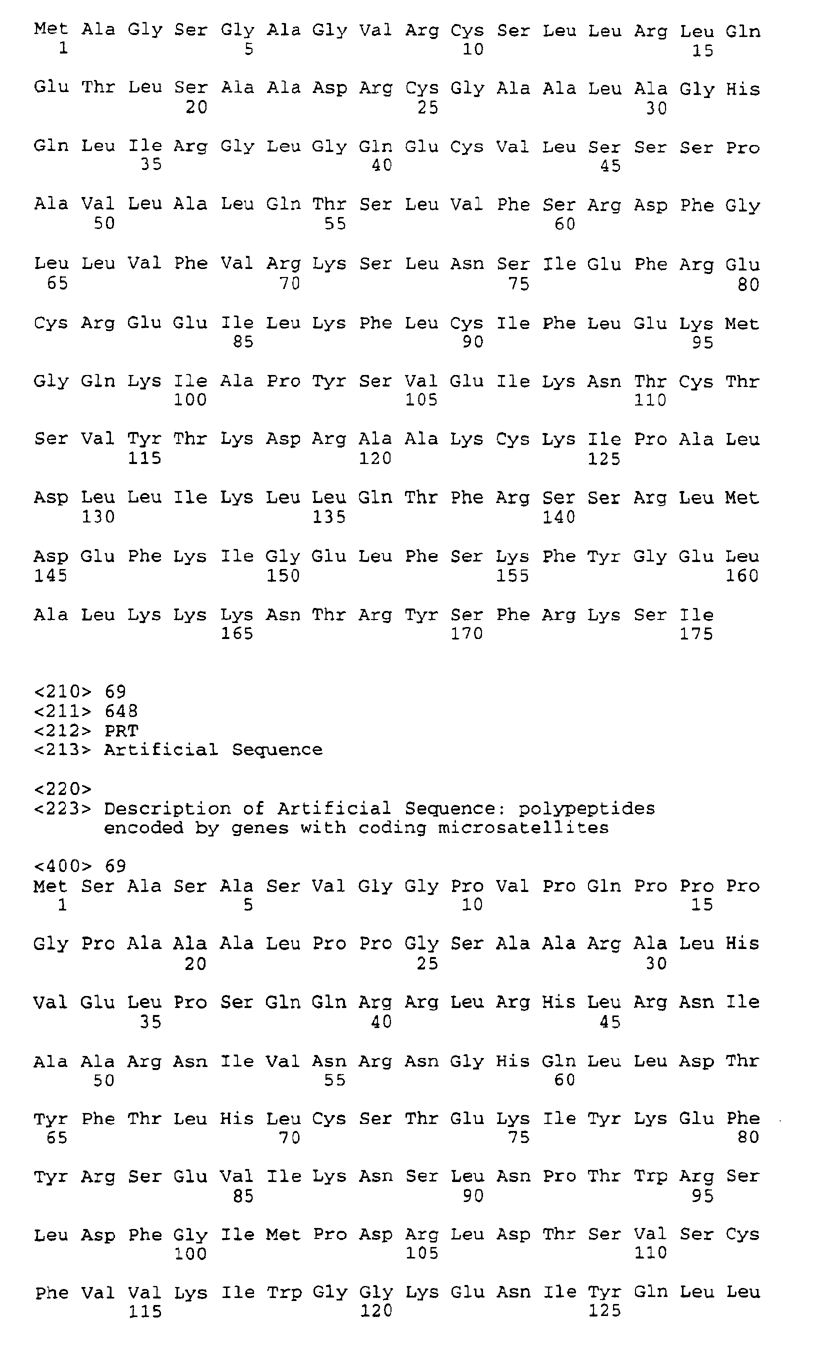

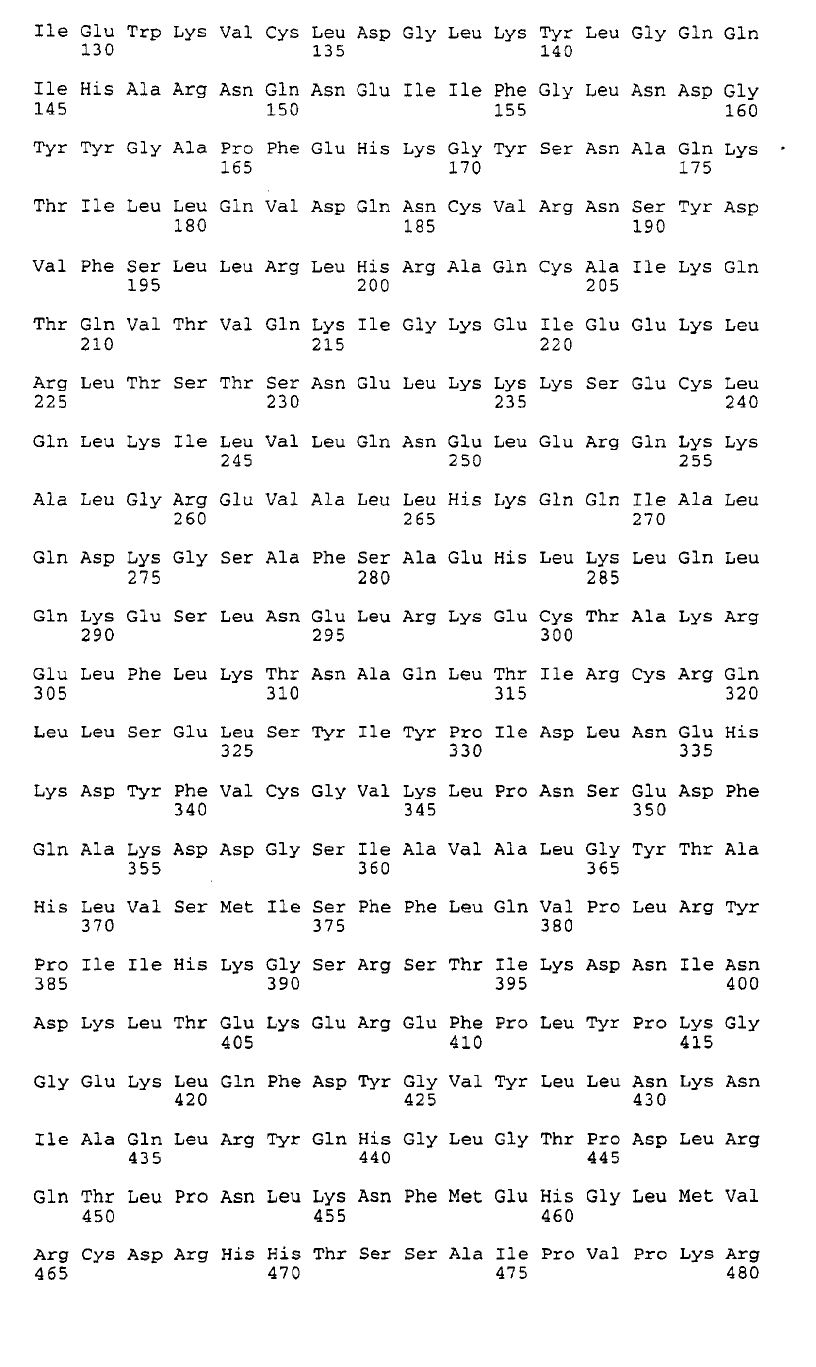

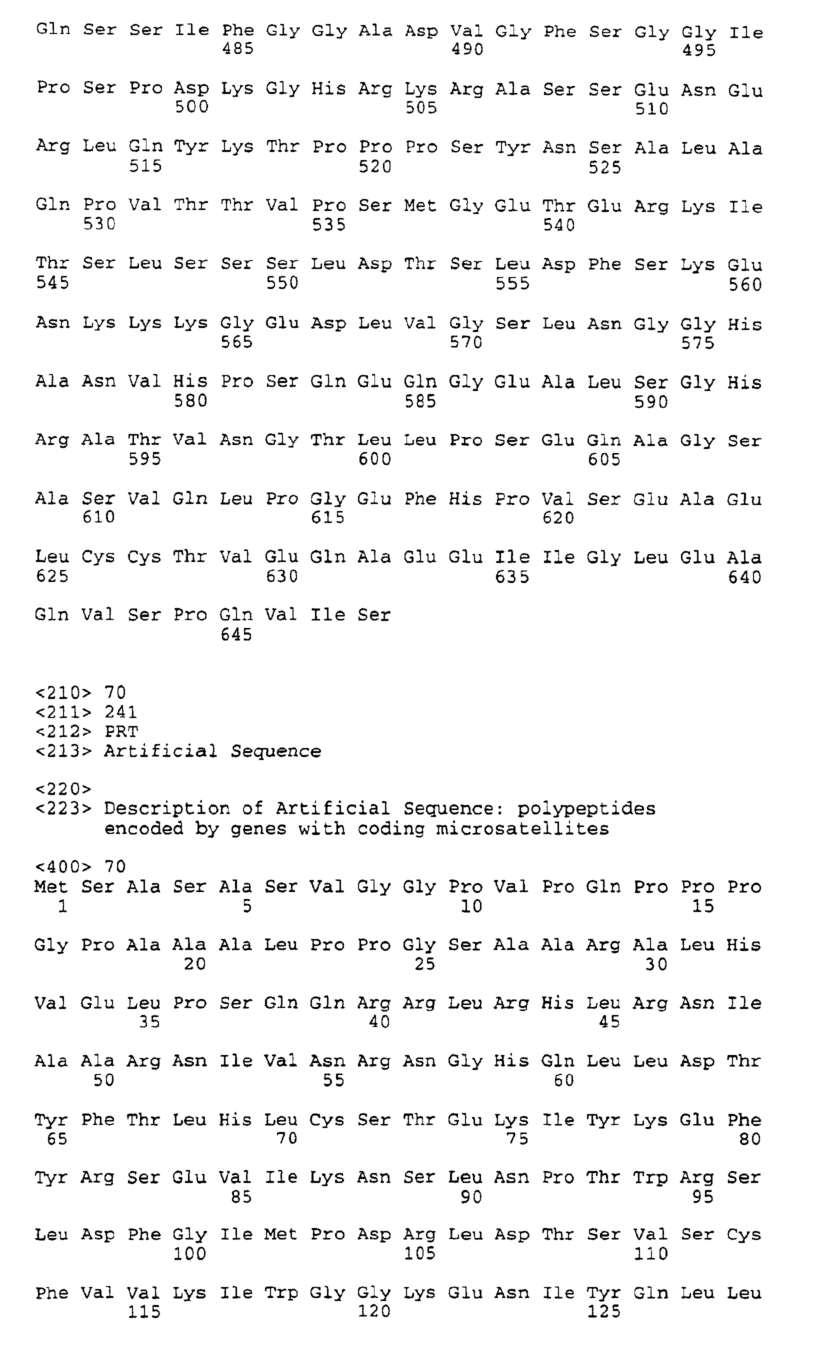

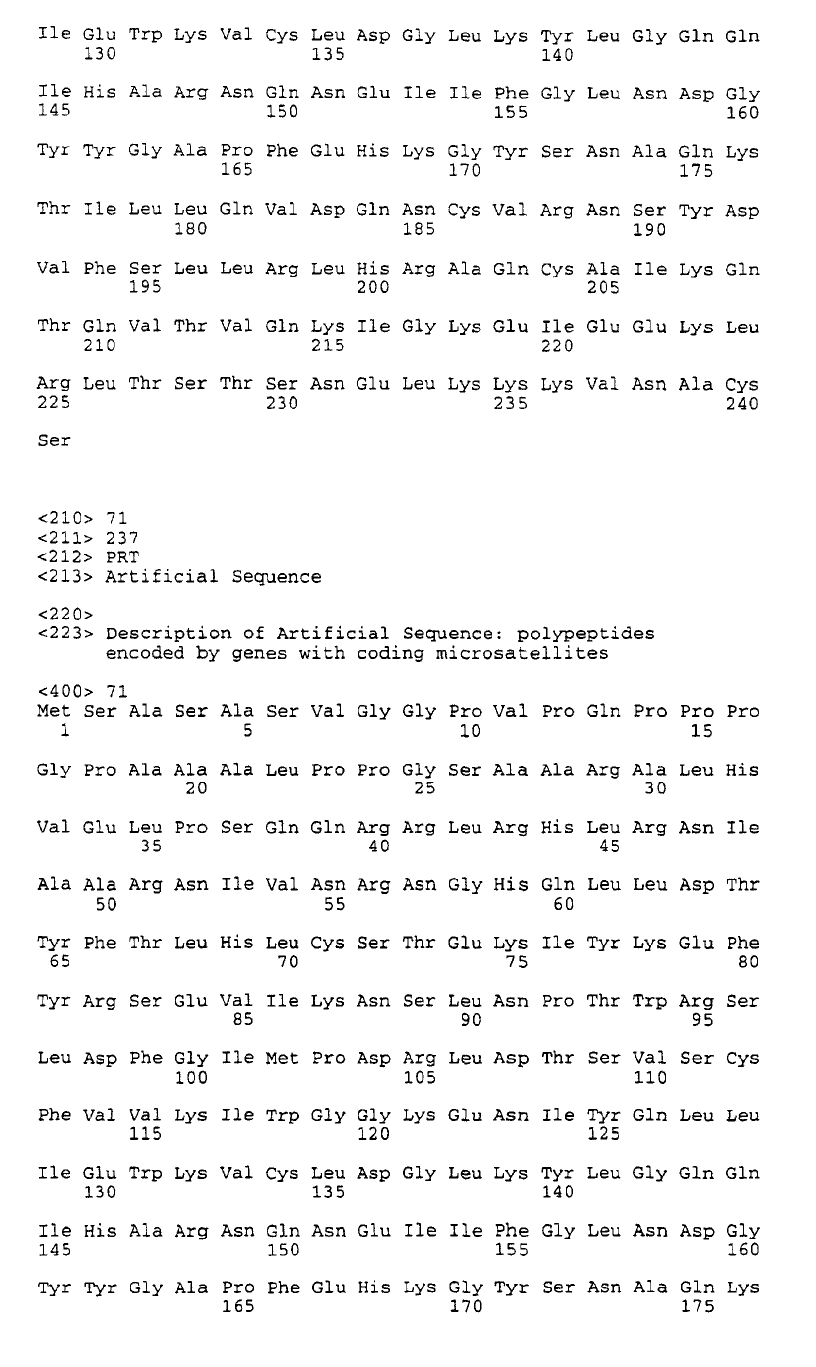

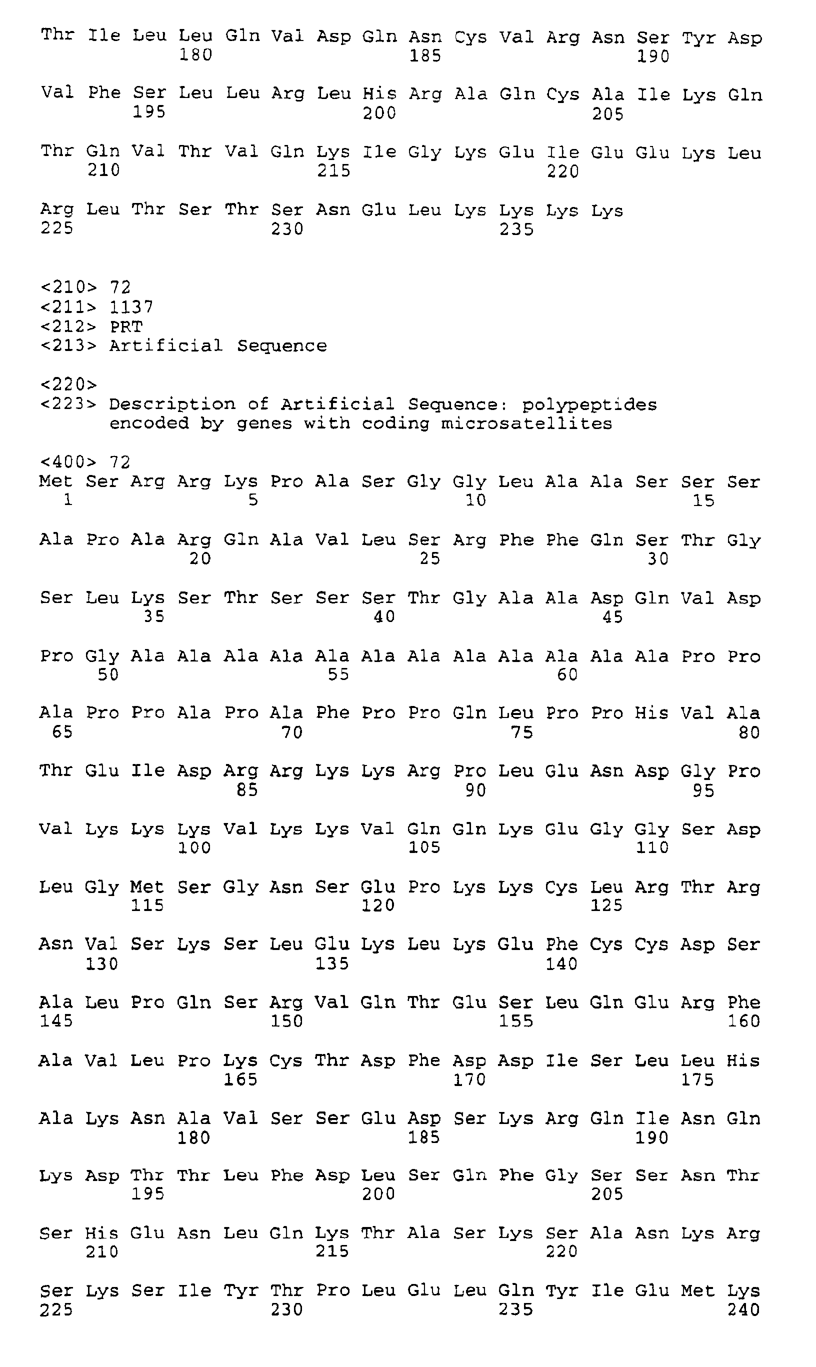

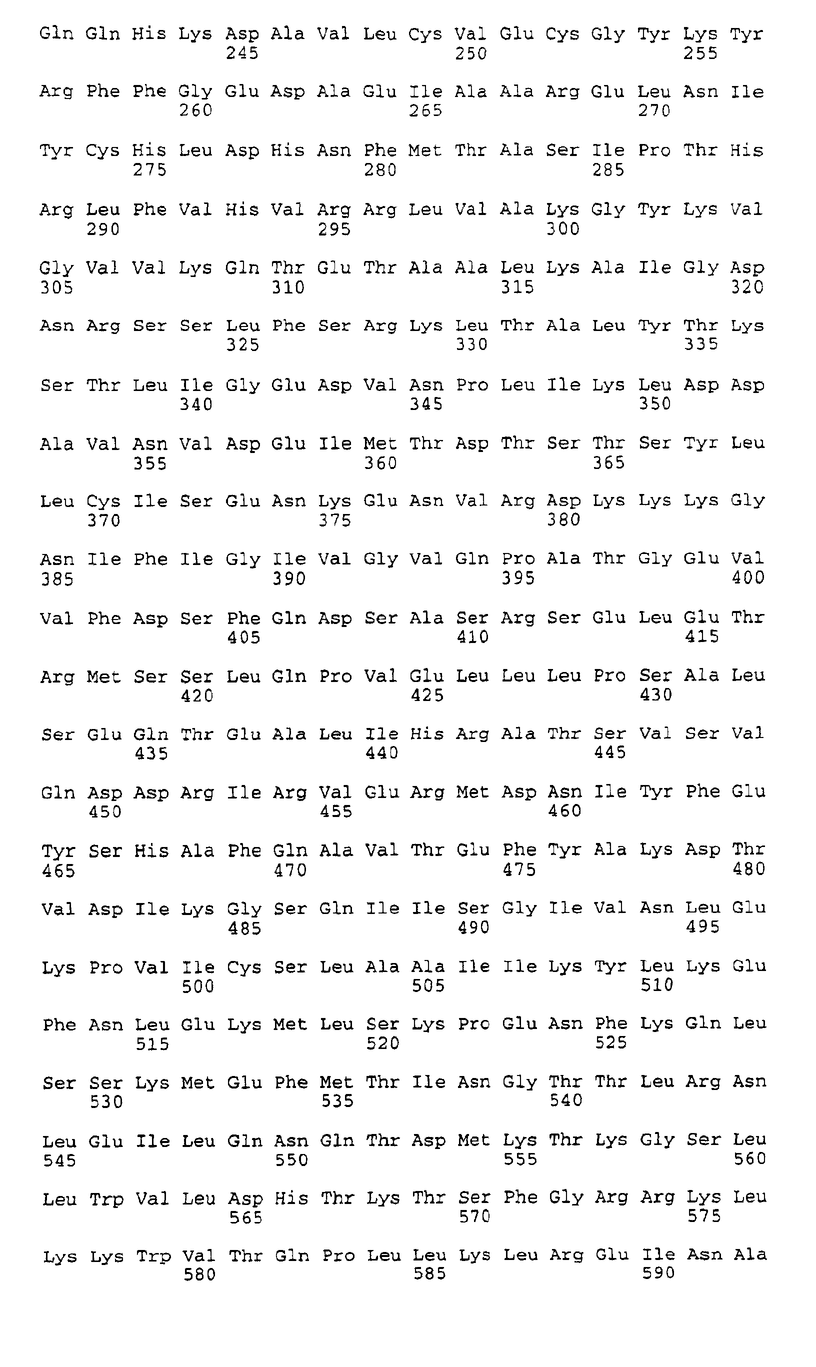

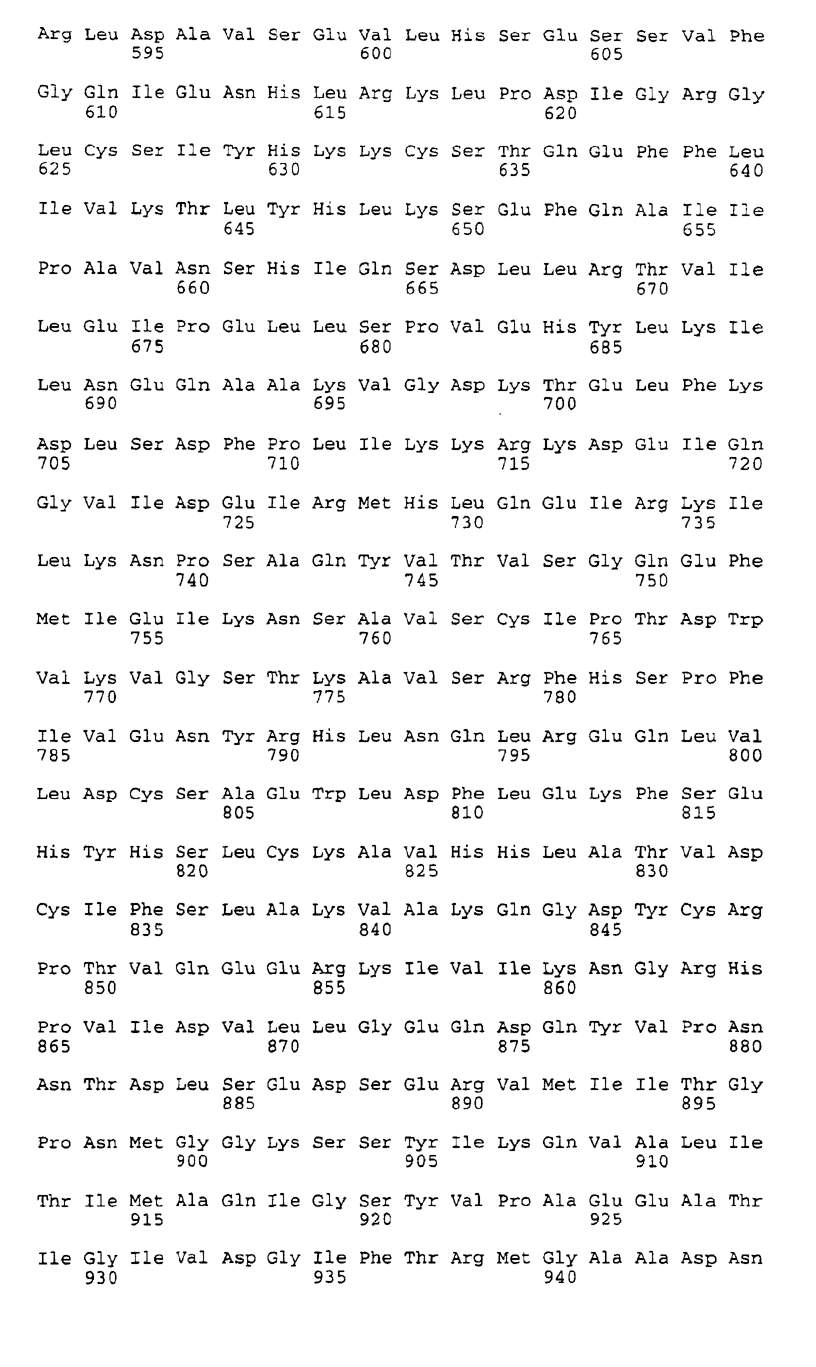

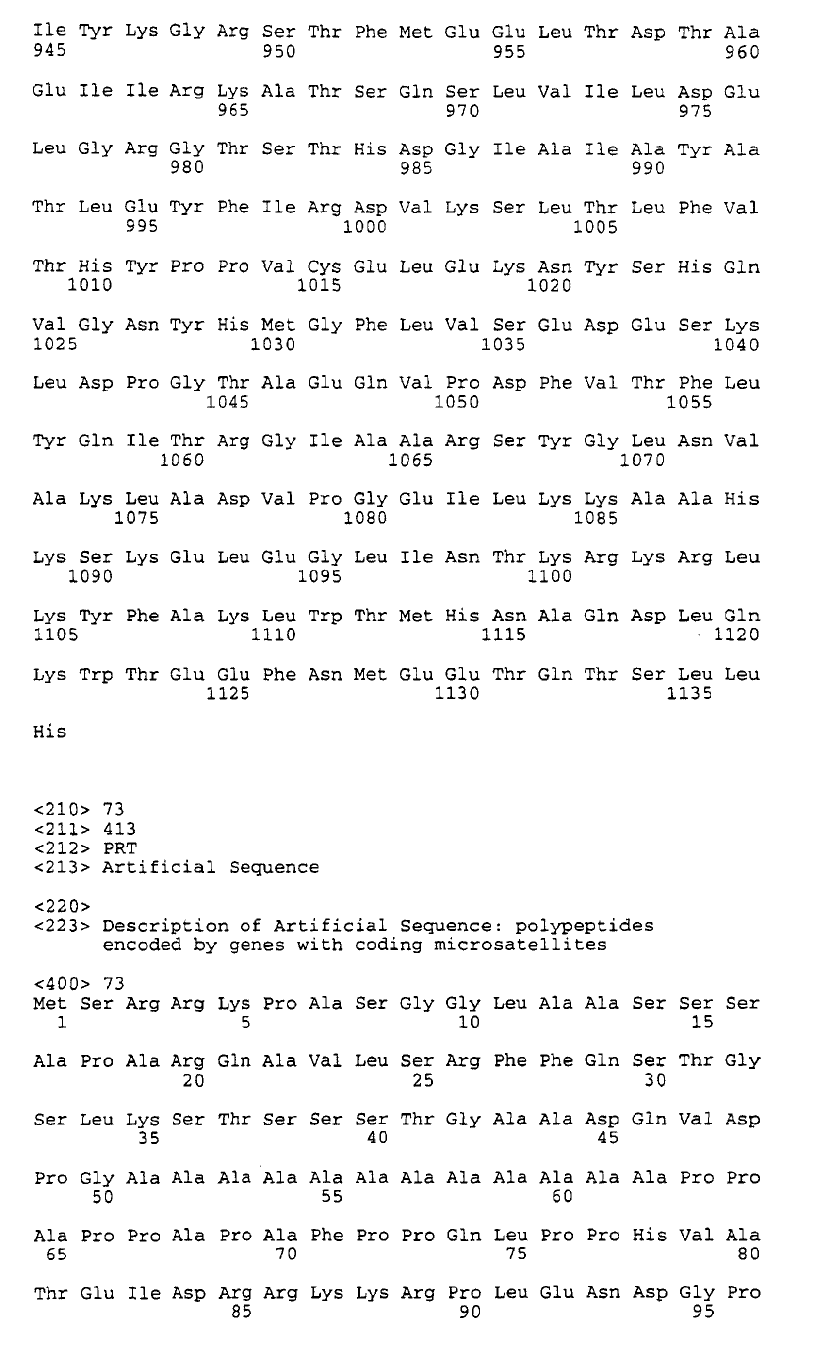

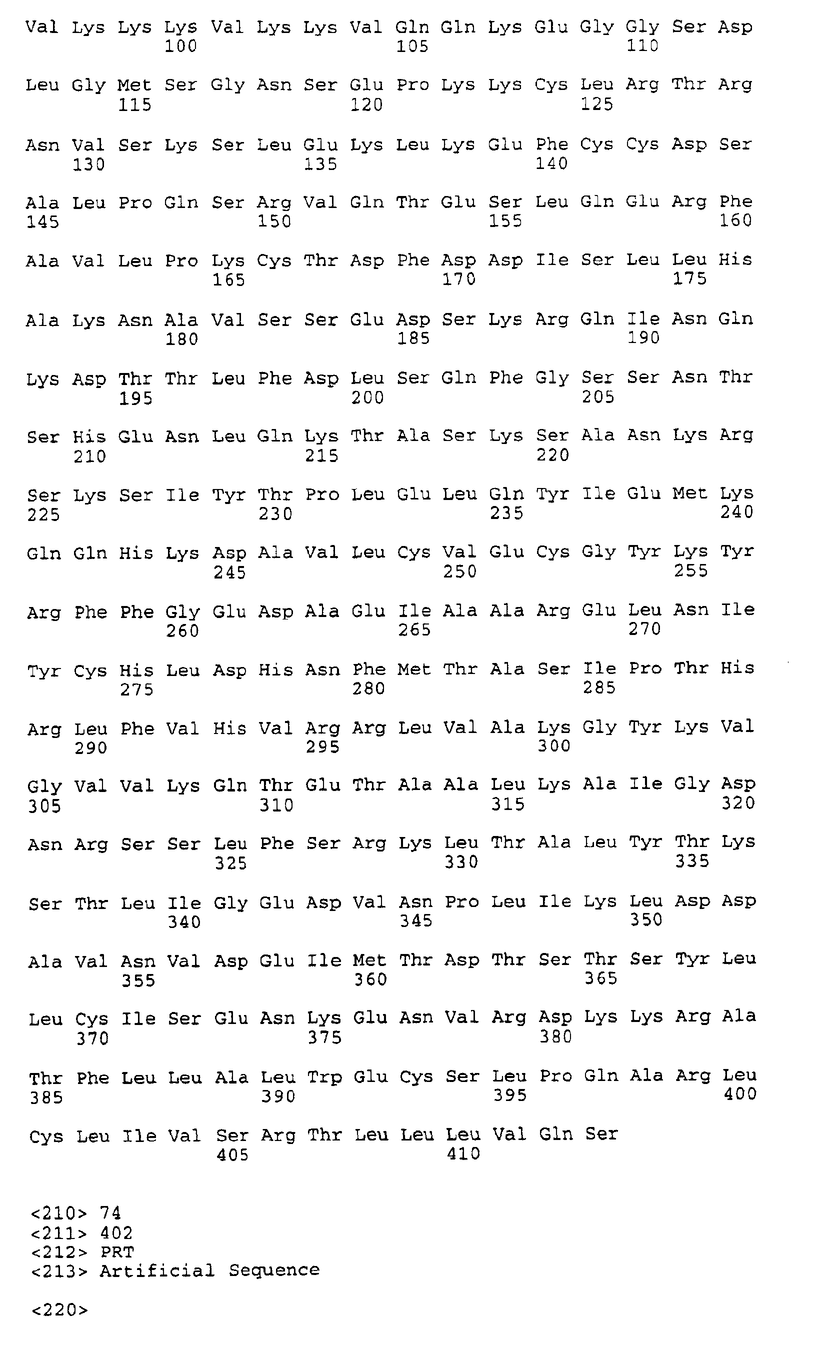

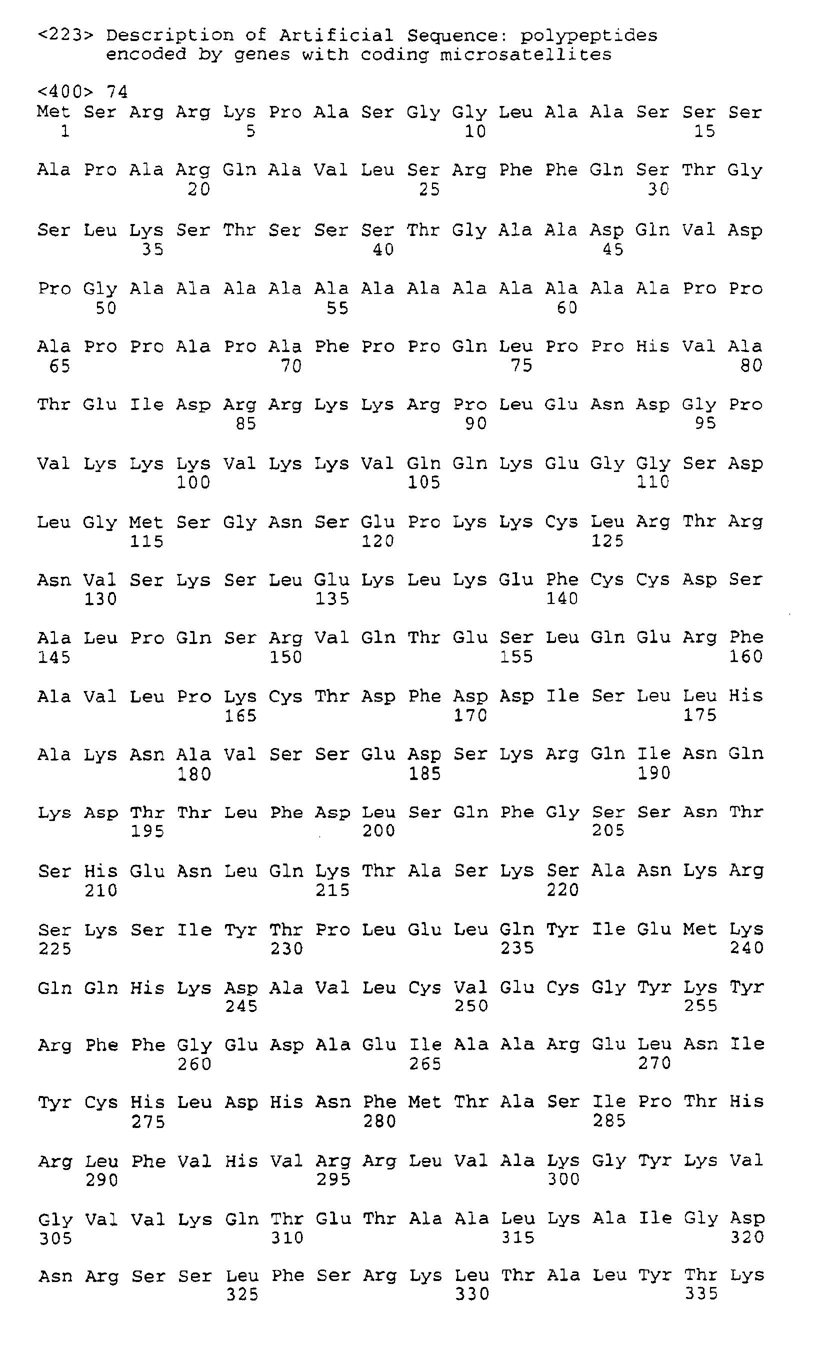

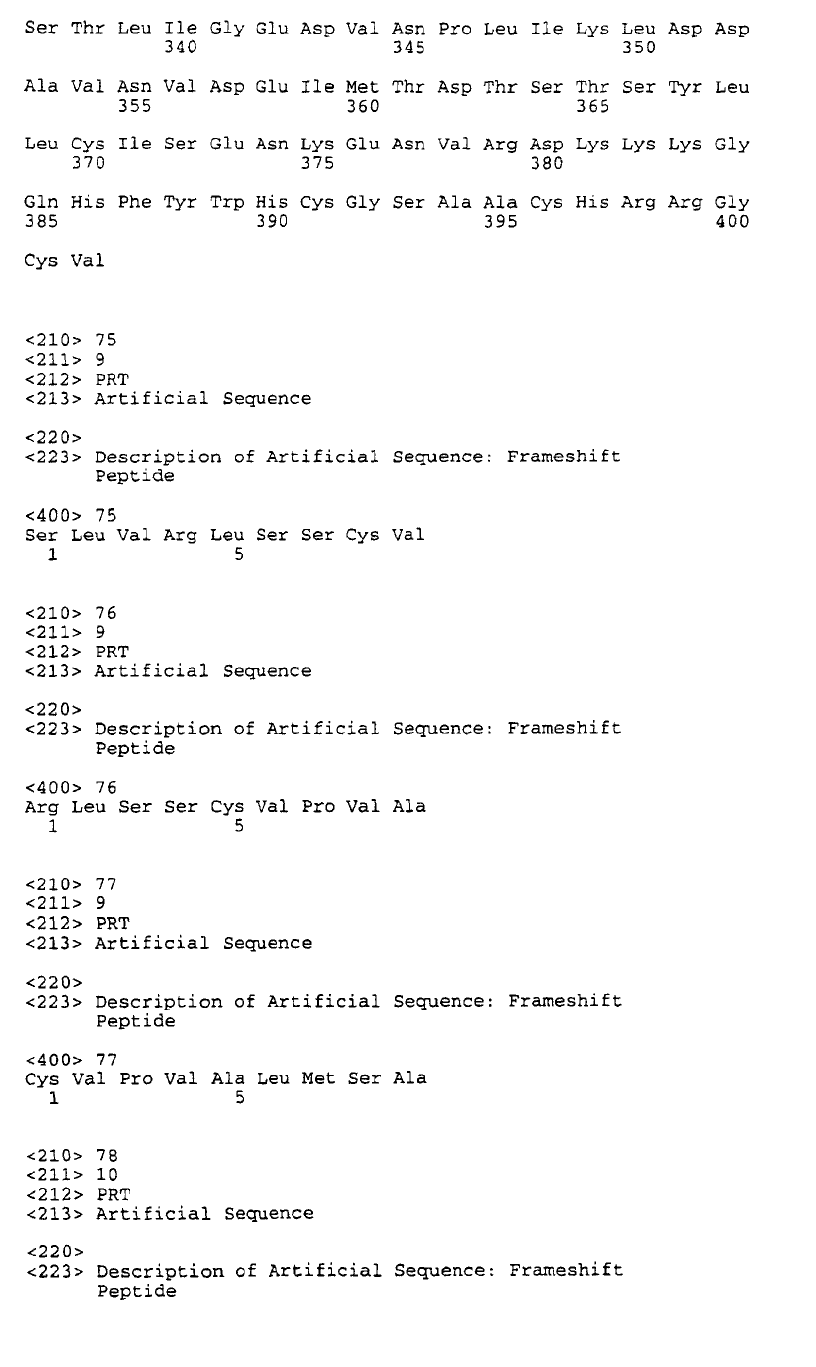

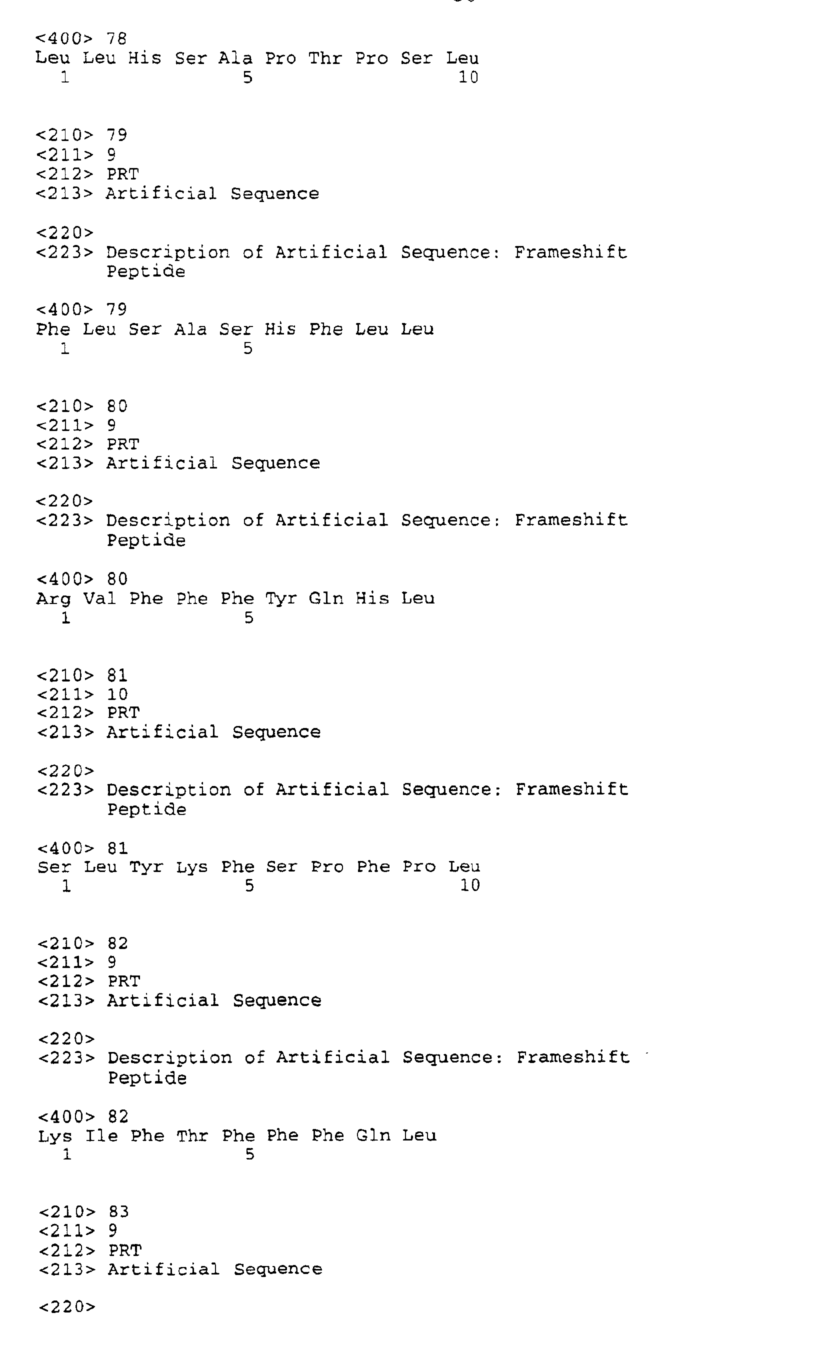

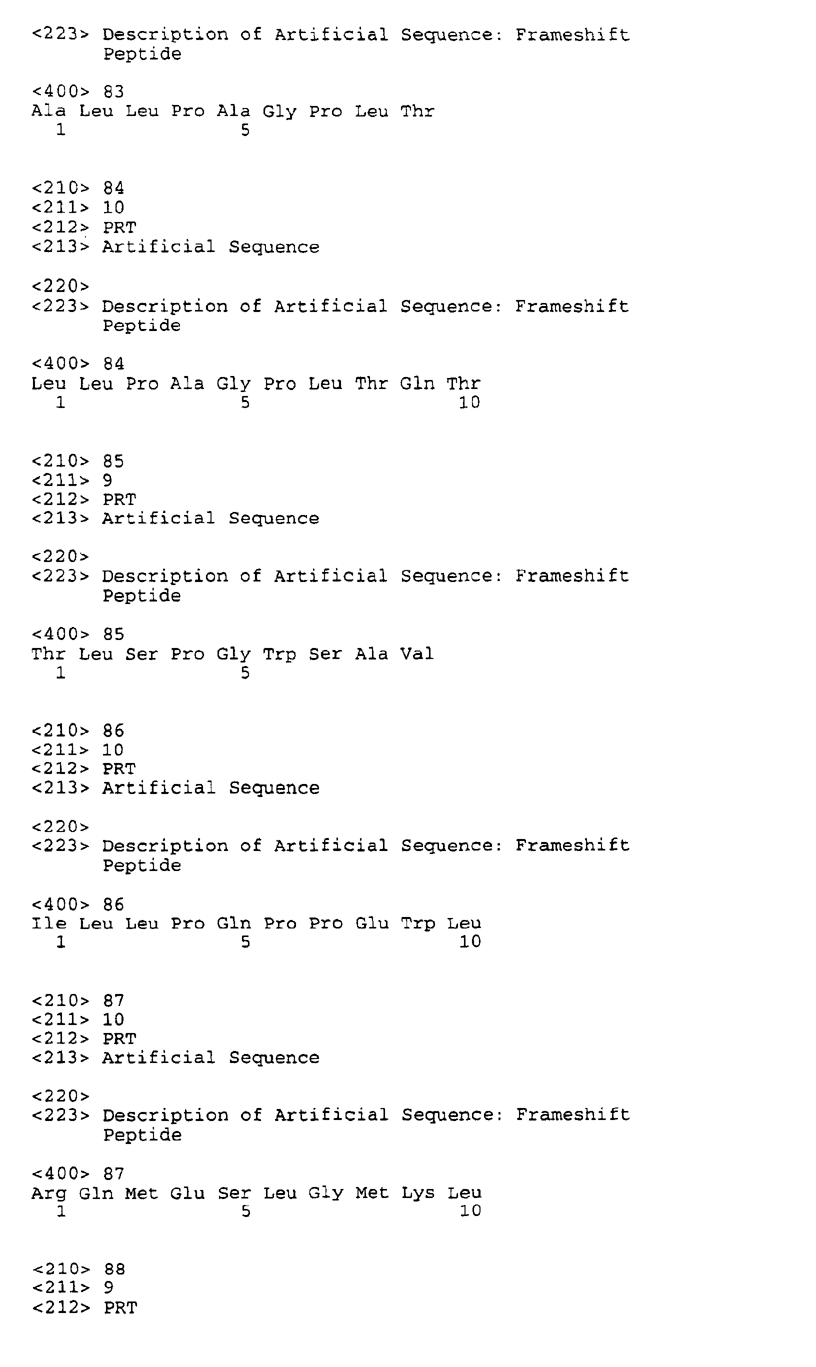

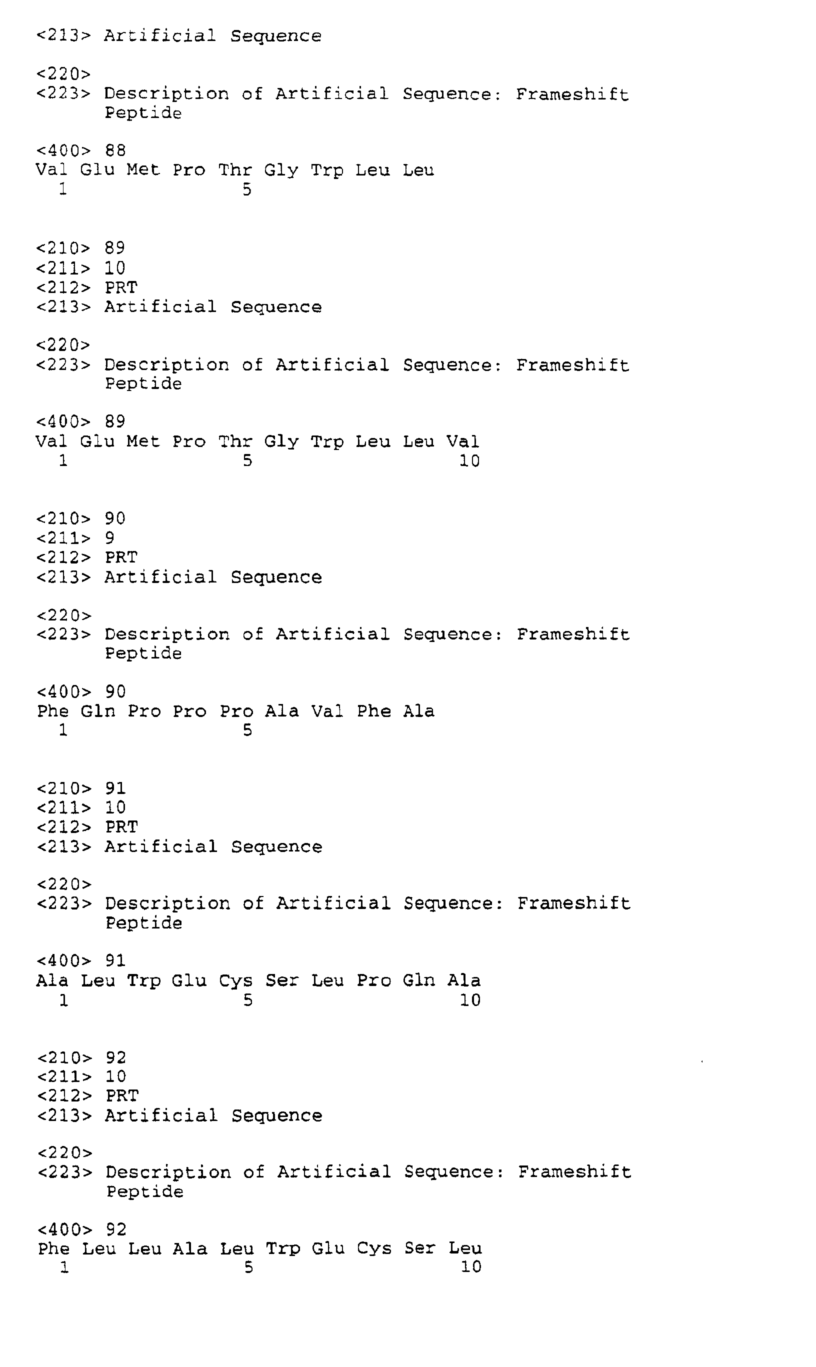

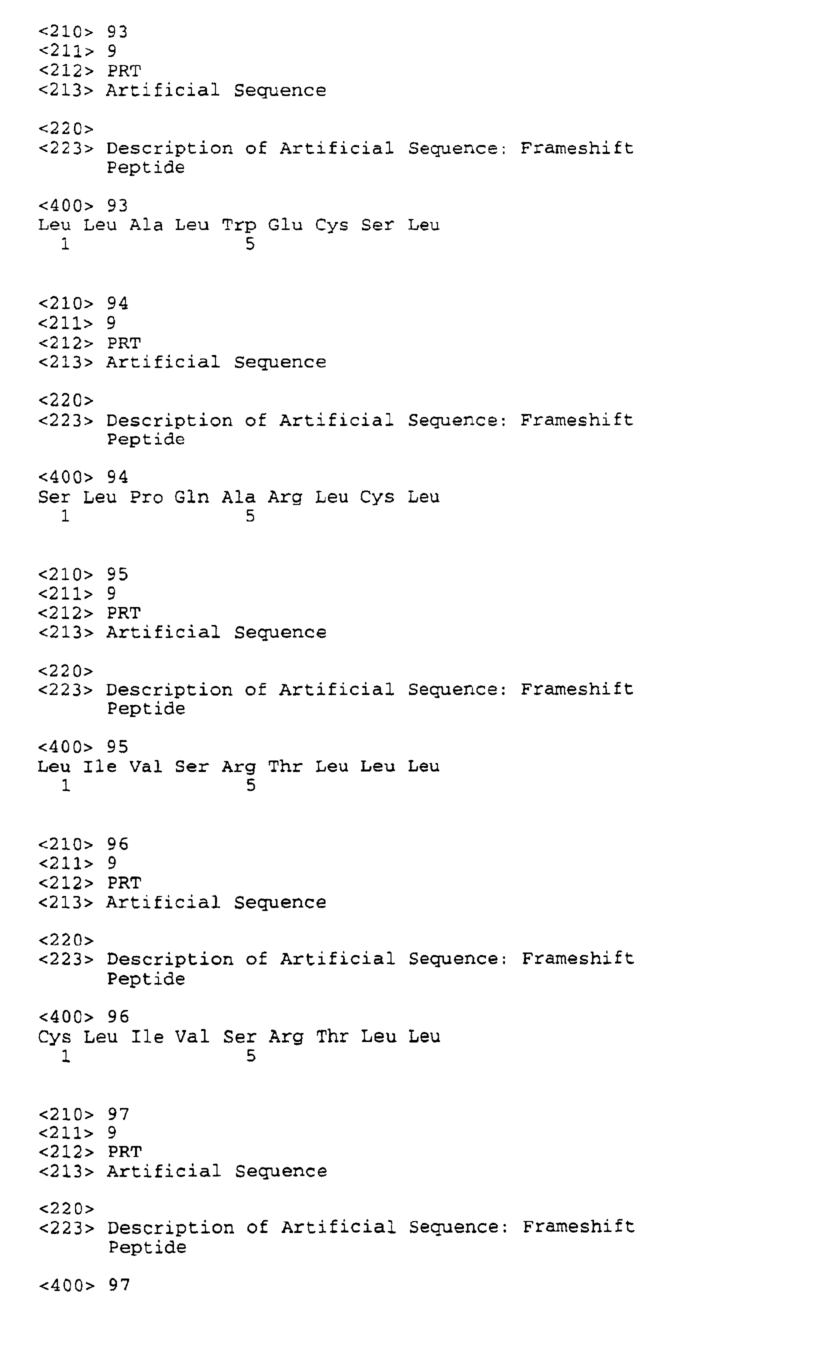

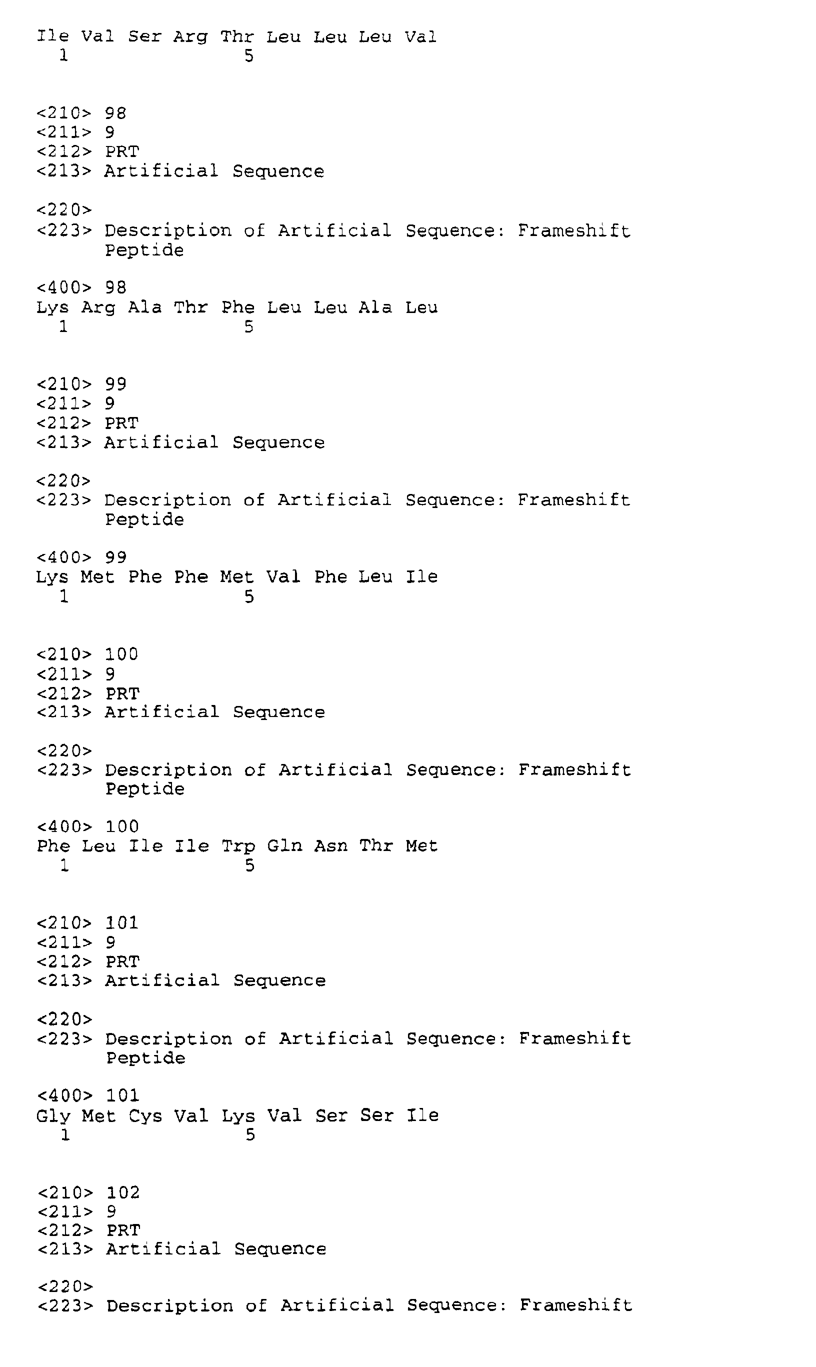

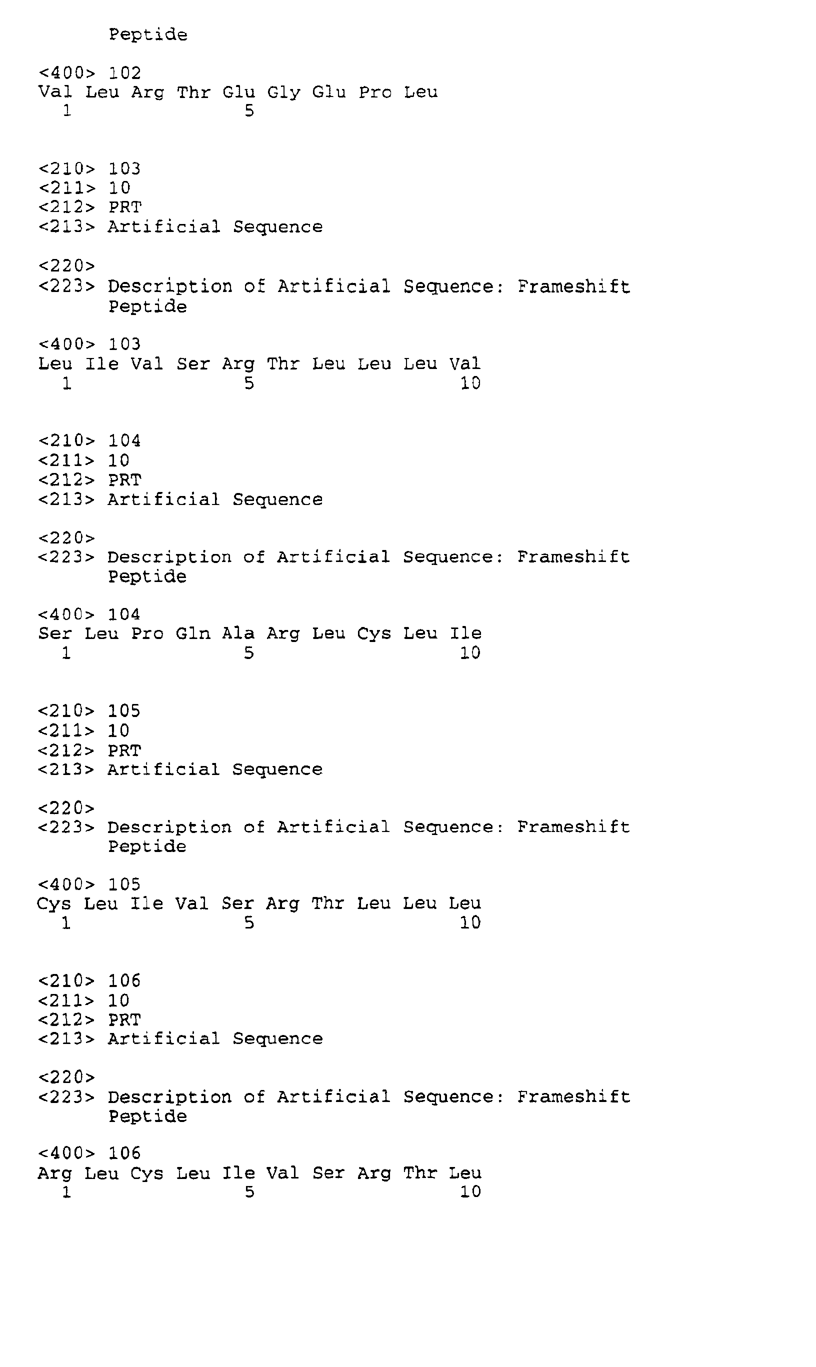

## Claims

1. A method for treatment of disorders associated with peptides arising from frameshift mutations in coding microsatellite regions in individuals, comprising selecting a set of at least 7 different frameshift peptides according to the following requirements:
a. at least 3 of the frameshift peptides occur with a frequency of more than 30% in tissues affected by said disorders;
b. the frameshift peptides comprise each at least one novel amino acid residue compared to the wild-type amino acid sequence; and
c. the frameshift peptides comprise members of at least 3 different biochemical pathways;
and administering said frameshift peptides to an individual either as peptide or in form of a nucleic acid to be expressed in situ in a pharmaceutical acceptable form that enables induction of an immune response in the individual against the peptides.

2. The method of claim 1, wherein at least one coding microsatellite sequence is represented by peptides originating from at least two different reading frames of said single microsatellite region.

3. The method according to any one of the claims 1-2, wherein at least one frameshift peptide is derived from a frameshift mutation in the (A)₁₀ tract of the TGFβRII gene.

4. The method according to any one of the claims 1-3, wherein at least one frameshift peptide is derived from a frameshift mutation in the (G)₈ tract of the BAX gene.

5. The method according to any one of the claims 1-4, comprising at least five frameshift peptides selected from a group consisting of frameshift peptides derived from the (A)₁₁ tract of the MACS gene, the (A)₁₀ tract of the CASP5 gene, the (A)₉ tract of the TCF-4 gene, the (G)₈ tract of the IGFIIR gene, the (T)₁₀ tract of the AC1 gene, the (A)₉ or (A)₁₀ tract of the SEC63 gene, the (A)₁₁ tract of the TAF1B gene, the (A)₁₁ tract of the PTHL3 gene, the (T)₁₄ tract of the U79260 gene, the (A)₁₀ tract of the AIM2 gene, the (A)₁₀ tract of the ABCF1 gene.

6. A method according to claim 1, wherein the set consists of frameshift peptides derived from mutations in the coding regions of the following genes: TGFβRII, U79260, CASP 5, HT001, PTHL3, MACS, TCF4, TAF1B, AC1 and SEC63

7. A method according to claim 1, wherein the set consists of frameshift peptides derived from mutations in the coding regions of the following genes: AIM2, ABCF1, BAX, FLT3L, OGT, ELAVL3, MAC30X, SLC4A3, UVRAG and MSH3.

8. The method according to claims 1 -7, wherein the disorder is cancer or its precursory stages.

9. The method according to claim 8, wherein the cancer is colon cancer.

10. The method according to claims 1-9, which is immuno-therapeutic treatment of disorders.

11. The method according to claims 1-10, which is preventive vaccination against disorders.

12. The method according to any one of the claims 1-11, wherein the nucleic acid administered to the individual is mRNA and/or cDNA.

13. A pharmaceutical composition comprising a set of frameshift polypeptides as used in the methods of any one of the claims 1-7 for the use in immunotherapy according to claim 8 or 9 of disorders associated with frameshift mutations in coding microsatellite regions.

14. The pharmaceutical composition of claim 13, which is used for the treatment of cancer.
